(19) Europäisches Patentamt
European Patent Office
Office européen des brevets

(11) EP 4 452 211 B1

(12) # EUROPEAN PATENT SPECIFICATION

(45) Date of publication and mention of the grant of the patent:
**03.12.2025 Bulletin 2025/49**

(21) Application number: **22843650.7**

(22) Date of filing: **19.12.2022**

(51) International Patent Classification (IPC):
***A61K 8/46** (2006.01)* ***A61Q 5/08** (2006.01)*
***A61Q 1/14** (2006.01)*

(52) Cooperative Patent Classification (CPC):
**A61K 8/463; A61Q 1/14; A61Q 5/08**

(86) International application number:
**PCT/EP2022/086727**

(87) International publication number:
**WO 2023/117954 (29.06.2023 Gazette 2023/26)**

(54) **PROCESS FOR REMOVING MAKEUP FROM KERATIN FIBRES WHICH HAVE BEEN TREATED BEFOREHAND WITH A COMPOSITION COMPRISING A PHOTOCROSSLINKABLE POLYMER**

VERFAHREN ZUR ENTFERNUNG VON MAKEUP VON KERATINFASERN, DIE IM VORAUS BEHANDELT WURDEN, MIT EINER ZUSAMMENSETZUNG MIT EINEM LICHTVERNETZBAREN POLYMER

PROCÉDÉ D'ÉLIMINATION DU MAQUILLAGE DE FIBRES KÉRATINIQUES AYANT ÉTÉ PRÉALABLEMENT TRAITÉES AVEC UNE COMPOSITION COMPRENANT UN POLYMÈRE PHOTORÉTICULABLE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **23.12.2021 FR 2114344**

(43) Date of publication of application:
**30.10.2024 Bulletin 2024/44**

(73) Proprietor: **L'OREAL**
**75008 Paris (FR)**

(72) Inventors:
- **VIC, Gabin**
  **93400 SAINT-OUEN (FR)**
- **PARRIS, Eric**
  **93400 SAINT-OUEN (FR)**
- **BURG, Pauline**
  **94152 CHEVILLY LARUE (FR)**

(74) Representative: **L'Oreal**
**Service D.I.P.I.**
**9, rue Pierre Dreyfus**
**92110 Clichy (FR)**

(56) References cited:
**WO-A1-2017/108767** **WO-A1-2021/104709**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 452 211 B1

## Description

The present invention relates to a process for removing makeup from keratin fibres to remove the colour of keratin fibres which have been treated beforehand with a composition comprising:

- at least one photocrosslinkable polymer including at least one photodimerizable group; and
- at least one colouring agent chosen from pigments, direct dyes and mixtures thereof, this process comprising the application to the keratin fibres of a makeup-removing composition, notably for removing the colour, comprising:
- at least one surfactant, preferably an anionic or nonionic surfactant; and
- at least one acidifying agent;
- and having a pH of less than or equal to 5.5, preferably from 1 to 5.

A subject of the present invention is also the use of the makeup-removing composition for removing colour according to the invention from keratin fibres which have been treated beforehand with a composition, notably a hair dye composition, comprising at least one photocrosslinkable polymer and at least one colouring agent chosen from pigments, direct dyes and mixtures thereof.

## Technical field

Photocrosslinkable polymers such as those described in patent application FR 3090364 are known in the prior art. These polymers form, on keratin fibres, coatings that are notably persistent with respect to shampoo washing. These polymers are proposed in particular in hair products for their styling properties.

WO2021104709 A1 discloses a process for removing colour from keratin fibres and in particular for removing the colour of keratin fibres treated beforehand by means of a composition comprising a polymer and a dye (pigment), the process comprising the application to the keratin fibres of a makeup-removing composition comprising an anionic surfactant and an acidifying agent, said makeup-removing composition having a pH of 1 to 3. The process is aimed at removal of amino-functionalised silicone polymers. WO2017108767 A1 discloses a process for the treatment of keratin fibres, comprising at least one photocrosslinkable polymer comprising at least one photodimerizable group and at least one hydrophobic group.

It appears important to find a process which allows the hair coating formed by the photocrosslinkable polymer coat to be removed at any time, so that the user can regain his/her original hair if he/she so desires, notably if the effect imparted by said polymer is not to his/her liking or if the user wishes to modify this effect. It is important for the process not to degrade the hair and for the hair obtained to have the same cosmetic properties as before the treatment.

The Applicant has just discovered, surprisingly, that it is possible to achieve the objectives mentioned above by applying to the hair a composition comprising at least one surfactant and an acidifying agent.

Moreover, the Applicant has found, surprisingly, that hair treated by means of the process according to the invention has a totally natural feel, and that it is not degraded by the process.

In the field of dyeing keratin fibres, in particular human keratin fibres, it is already known practice to dye hair keratin fibres via various techniques using direct dyes or pigments for non-permanent colouring, or dye precursors for permanent colouring.

There are essentially three types of process for dyeing the hair:

a) "permanent" dyeing, the function of which is to afford a substantial modification to the natural colour and which uses oxidation dyes which penetrate into the hair fibre and forms the dye via an oxidative condensation process;

b) non-permanent, semi-permanent or direct dyeing, which does not use the oxidative condensation process and withstands four or five shampoo washes; it consists in dyeing hair keratin fibres with dye compositions containing direct dyes;

c) temporary dyeing, which gives rise to a modification of the natural colour of the hair that remains from one shampoo wash to the next, and which serves to enhance or correct a shade that has already been obtained. It may also be likened to a "makeup" process.

For this last type of dyeing, it is known practice to use coloured polymers formed by grafting one or more dyes of azo, triphenylmethane, azine, indoamine or anthraquinone nature onto a polymer chain. These coloured polymers are not entirely satisfactory, notably as regards the homogeneity of the colouring obtained and its resistance, not to mention the problems associated with their manufacture and notably with their reproducibility.

Another dyeing method consists in using pigments. Specifically, the use of pigment on the surface of hair keratin fibres generally makes it possible to obtain visible colourings on dark hair, since the surface pigment masks the natural colour of the fibre. This dyeing method can also afford dye compositions which have the advantage of producing a uniform

coloured coating on keratin fibres, notably the hair, while at the same time forming a coat which withstands shampoo washing and the various attacking factors to which the hair may be subjected such as brushing and/or friction, without degradation of the hair.

Coloured coatings that are persistent with respect to shampoo washing can be obtained with compositions comprising photocrosslinkable polymers and dyes chosen from direct dyes, pigments and mixtures thereof.

However, no processes exist using colour-removing compositions that are efficient on hair that has been dyed beforehand with this type of temporary hair dye composition, which is persistent with respect to shampoo washing.

A need thus remains for a process for efficiently removing colour from keratin fibres which have been dyed beforehand using dye compositions that are persistent with respect to shampoo washing.

Thus, the aim of the present invention is to develop a process for treating hair keratin fibres which makes it possible to obtain very good efficiency as regards colour removal.

**Disclosure of the invention**

This aim is achieved with the present invention, one subject of which is a process for removing makeup from keratin fibres to remove colour from keratin fibres which have been treated beforehand with a composition comprising:

- at least one photocrosslinkable polymer including at least one photodimerizable group; and
- at least one colouring agent chosen from pigments, direct dyes and mixtures thereof, this process comprising the application to the keratin fibres of a makeup-removing composition, notably to remove the colour, comprising:
- at least one surfactant, preferably an anionic or nonionic surfactant; and
- at least one acidifying agent,

and said makeup-removing composition having a pH of less than or equal to 5.5, preferably from 1 to 5.

A subject of the invention is also the use of the makeup-removing composition for removing colour from hair keratin fibres that have been treated beforehand with a composition, notably a hair dye composition, comprising
a photocrosslinkable polymer including at least one photodimerizable group; and

- at least one colouring agent chosen from pigments, direct dyes and mixtures thereof.

Other subjects, features, aspects and advantages of the invention will emerge even more clearly on reading the description and the examples that follow.

The term "makeup-removing composition" means a composition which allows the removal of the coloured or uncoloured coating present on keratin fibres.

In the text hereinbelow, unless otherwise indicated, the limits of a range of values are included in that range, notably in the expressions "between" and "ranging from ... to ..".

The term "at least one" used in the present description is equivalent to the term "one or more". The term "at least one" means one, two, three or more.

The invention is not limited to the examples illustrated. The characteristics of the various examples may notably be combined within variants which are not illustrated.

For the purposes of the present invention and unless otherwise indicated:

- an *"alkyl"* radical denotes a linear or branched saturated radical containing, for example, from 1 to 20 carbon atoms;
- an *"aminoalkyl"* radical denotes an alkyl radical as defined previously, said alkyl radical comprising an $NH_2$ group;
- *a "hydroxyalkyl"* radical denotes an alkyl radical as defined previously, said alkyl radical comprising an OH group;
- *a "cycloalkyl"* or *"alicycloalkyl"* radical denotes a cyclic saturated monocyclic or bicyclic, preferably monocyclic, hydrocarbon-based group comprising from 1 to 3 rings, preferably 2 rings, and comprising from 3 to 24 carbon atoms, in particular comprising from 3 to 20 carbon atoms, more particularly from 3 to 13 carbon atoms, even more particularly from 3 to 12 carbon atoms, preferably between 5 and 10 carbon atoms, such as cyclopropyl, cyclopentyl, cyclohexyl, cycloheptyl or norbornyl, in particular cyclopropyl, cyclopentyl or cyclohexyl, it being understood that the cycloalkyl radical may be substituted with one or more $(C_1-C_4)$alkyl groups such as methyl; preferably, the cycloalkyl radical is then an isobornyl group;
- *a "cycloalkylene"* radical denotes a divalent cycloalkyl group with *"cycloalkyl"* as defined above, preferably of $C_3-C_{12}$;
- an *"aryl"* radical is a monocyclic, bicyclic or tricyclic, fused or non-fused, unsaturated and aromatic hydrocarbon-based cyclic radical, comprising from 6 to 14 carbon atoms, preferably between 6 and 12 carbon atoms; preferably, the aryl group comprises 1 ring of 6 carbon atoms such as phenyl, naphthyl, anthryl, phenanthryl and biphenyl, it being understood that the aryl radical may be substituted with one or more $(C_1-C_4)$alkyl groups such as methyl, preferably tolyl, xylyl, or methylnaphthyl; preferably, the aryl group represents phenyl;

- an *"arylene"* radical is a divalent aryl radical with *"aryl"* as defined previously; preferably, arylene represents phenylene;
- an *"aryloxy"* radical denotes an aryl-oxy radical with *"aryl"* as defined previously;
- an *"acyloxy"* radical denotes an ester radical R-C(O)-O- with R being an alkyl group as defined above;
- *a "reactive"* group is a group that is capable of forming a covalent bond with another identical or different group, by chemical reaction.

▪ an *"alkylene chain"* represents an acyclic hydrocarbon-based divalent chain which is of $C_1$-$C_{20}$, particularly $C_1$-$C_6$, more particularly $C_1$-$C_2$ when the chain is linear, optionally substituted with one or more groups, which may be identical or different, chosen from i) hydroxyl, ii) ($C_1$-$C_2$)alkoxy, iii) (poly)hydroxy($C_2$-$C_4$)alkoxy(di)($C_1$-$C_2$)(alkyl) amino, iv) $R^a$-$Z^a$-C($Z^b$)-$Z^c$-, and v) $R^a$-$Z^a$-S$(O)_t$-$Z^c$-with $Z^a$ and $Z^b$, which may be identical or different, representing an oxygen or sulfur atom, or a group $NR^{a'}$, $Z^c$ representing a bond, an oxygen or sulfur atom, or a group $NR^a$; $R^a$ representing an alkali metal, a hydrogen atom, an alkyl group, or alternatively is absent if another part of the molecule is cationic and $R^{a'}$ representing a hydrogen atom or an alkyl group; more particularly, the groups iv) are chosen from carboxylate -C(O)$O^-$ or -C(O)OMetal (Metal = alkali metal), carboxyl -C(O)-OH, guanidino $H_2$H-C($NH_2$)-NH-, amidino $H_2$H-C($NH_2$)-, (thio)ureo $H_2$N-C(O)-NH- and $H_2$N-C(S)-NH-, aminocarbonyl -C(O)-$NRa'_2$ or aminothiocarbonyl -C(S)-$NRa'_2$; carbamoyl Ra'-C(O)-NRa'- or thiocarbamoyl Ra'-C(S)-NRa'- with Ra', which may be identical or different, representing a hydrogen atom or a ($C_1$-$C_4$)alkyl group;

▪ the "aryl" or "heteroaryl" radicals or the aryl or heteroaryl part of a radical may be substituted with at least one substituent borne by a carbon atom, chosen from:

- a $C_1$-$C_{16}$ and preferably $C_1$-$C_8$ alkyl radical optionally substituted with one or more radicals chosen from hydroxyl, $C_1$-$C_2$ alkoxy, $C_2$-$C_4$ (poly)hydroxyalkoxy, acylamino, amino substituted with two $C_1$-$C_4$ alkyl radicals, which may be identical or different, optionally bearing at least one hydroxyl group, or the two radicals possibly forming, with the nitrogen atom to which they are attached, a saturated or unsaturated, optionally substituted 5- to 7-membered and preferably 5- or 6-membered heterocycle optionally comprising another heteroatom identical to or different from nitrogen;
- a halogen atom;
- a hydroxyl group;
- a $C_1$-$C_2$ alkoxy radical;
- a $C_2$-$C_4$ (poly)hydroxyalkoxy radical;
- an amino radical;
- a 5- or 6-membered heterocycloalkyl radical;
- an optionally cationic 5- or 6-membered heteroaryl radical, preferentially imidazolium, optionally substituted with a ($C_1$-$C_4$)alkyl radical, preferentially methyl;
- an amino radical substituted with one or two identical or different $C_1$-$C_6$ alkyl radicals, optionally bearing at least:

  i) a hydroxyl group;
  ii) an amino group optionally substituted with one or two optionally substituted $C_1$-$C_3$ alkyl radicals, said alkyl radicals possibly forming, with the nitrogen atom to which they are attached, a saturated or unsaturated, optionally substituted 5- to 7-membered heterocycle, optionally comprising at least one other nitrogen or non-nitrogen heteroatom;
  iii) a quaternary ammonium group $-N^+R'R''R'''$, $M^-$ for which R', R" and R"', which may be identical or different, represent a hydrogen atom or a $C_1$-$C_4$ alkyl group; and $M^-$ represents the counterion of the organic or mineral acid or of the corresponding halide;
  iv) or an optionally cationic 5- or 6-membered heteroaryl radical, preferentially imidazolium, optionally substituted with a ($C_1$-$C_4$)alkyl radical, preferentially methyl;

- an acylamino radical (-NR-C(O)-R') in which the radical R is a hydrogen atom or a $C_1$-$C_4$ alkyl radical optionally bearing at least one hydroxyl group and the R' radical is a $C_1$-$C_2$ alkyl radical;
- a carbamoyl radical ($(R)_2$N-C(O)-) in which the radicals R, which may be identical or different, represent a hydrogen atom or a $C_1$-$C_4$ alkyl radical optionally bearing at least one hydroxyl group;
- an alkylsulfonylamino radical (R'-$S(O)_2$-N(R)-) in which the radical R represents a hydrogen atom or a $C_1$-$C_4$ alkyl radical optionally bearing at least one hydroxyl group and the radical R' represents a $C_1$-$C_4$ alkyl radical or a phenyl radical; an aminosulfonyl radical ($(R)_2$N-$S(O)_2$-) in which the radicals R, which may be identical or different, represent a hydrogen atom or a $C_1$-$C_4$ alkyl radical optionally bearing at least one hydroxyl group;
- a carboxylic radical in acid form or salified (preferably with an alkali metal or a substituted or unsubstituted ammonium) form;

- a cyano group;
- a nitro or nitroso group;
- a polyhaloalkyl group, preferentially trifluoromethyl;

the cyclic, cycloalkyl or heterocyclic part of a non-aromatic radical may be substituted with at least one substituent chosen from the following groups:

- hydroxyl;
- $C_1$-$C_4$ alkoxy or $C_2$-$C_4$ (poly)hydroxyalkoxy;
- $C_1$-$C_4$ alkyl;
- alkylcarbonylamino (R-C(O)-N(R')-) in which the radical R' is a hydrogen atom or a $C_1$-$C_4$ alkyl radical optionally bearing at least one hydroxyl group, and the radical R is a $C_1$-$C_2$ alkyl radical or an amino radical optionally substituted with two $C_1$-$C_4$ alkyl groups, which may be identical or different, optionally bearing at least one hydroxyl group, said alkyl radicals possibly forming, with the nitrogen atom to which they are attached, a saturated or unsaturated, optionally substituted 5- to 7-membered heterocycle optionally comprising at least one other nitrogen or non-nitrogen heteroatom;
- alkylcarbonyloxy (R-C(O)-O-) in which the radical R is a $C_1$-$C_4$ alkyl radical or an amino group optionally substituted with one or two identical or different $C_1$-$C_4$ alkyl groups themselves optionally bearing at least one hydroxyl group, said alkyl radicals possibly forming, with the nitrogen atom to which they are attached, a saturated or unsaturated, optionally substituted 5- to 7-membered heterocycle, optionally comprising at least one other nitrogen or non-nitrogen heteroatom;
- alkoxycarbonyl (R-G-C(O)-) in which the radical R is a $C_1$-$C_4$ alkoxy radical, G is an oxygen atom or an amino group optionally substituted with a $C_1$-$C_4$ alkyl group optionally bearing at least one hydroxyl group, said alkyl radical possibly forming, with the nitrogen atom to which they are attached, a saturated or unsaturated, optionally substituted 5- to 7-membered heterocycle, optionally comprising at least one other nitrogen or non-nitrogen heteroatom;

▪ a cyclic, cycloalkyl or heterocyclic radical, or a non-aromatic part of an aryl or heteroaryl radical, may also be substituted with one or more oxo groups;

▪ a cycloalkyl radical is a monocyclic or bicyclic, hydrocarbon-based radical comprising from 3 to 10 carbon atoms, preferably from 4 to 7 carbon atoms, such as cyclopentyl or cyclohexyl;

▪ a hydrocarbon-based chain is unsaturated when it includes one or more double bonds and/or one or more triple bonds;

▪ an "aryl" radical represents a monocyclic or fused or non-fused polycyclic carbon-based group comprising from 6 to 22 carbon atoms, and in which at least one ring is aromatic; preferentially, the aryl radical is a phenyl, biphenyl, naphthyl, indenyl, anthracenyl or tetrahydronaphthyl;

▪ a "heteroaryl" radical represents an optionally cationic, 5- to 22-membered, monocyclic or fused or non-fused polycyclic group, comprising from 1 to 6 heteroatoms chosen from nitrogen, oxygen, sulfur and selenium atoms, at least one ring of which is aromatic; preferentially, a heteroaryl radical is chosen from acridinyl, benzimidazolyl, benzobistriazolyl, benzopyrazolyl, benzopyridazinyl, benzoquinolyl, benzothiazolyl, benzotriazolyl, benzoxazolyl, pyridinyl, tetrazolyl, dihydrothiazolyl, imidazopyridyl, imidazolyl, indolyl, isoquinolyl, naphthoimidazolyl, naphthoxazolyl, naphthopyrazolyl, oxadiazolyl, oxazolyl, oxazolopyridyl, phenazinyl, phenoxazolyl, pyrazinyl, pyrazolyl, pyrilyl, pyrazoyltriazyl, pyridyl, pyridinoimidazolyl, pyrrolyl, quinolyl, tetrazolyl, thiadiazolyl, thiazolyl, thiazolopyridinyl, thiazoylimidazolyl, thiopyrylyl, triazolyl, xanthyl and the ammonium salt thereof;

▪ *a "heterocyclic"* radical denotes a saturated or unsaturated, non-aromatic or aromatic, monocyclic or polycyclic hydrocarbon-based radical, comprising one or more heteroatoms, preferably from 1 to 5 atoms chosen from O, S or N, including from 3 to 20 ring members, preferably between 5 and 10 ring members, such as imidazolyl, pyrrolyl and furanyl;

▪ *a "heterocycloalkyl radical"* is a heterocyclic radical comprising at least one saturated ring;

▪ *a "cationic heteroaryl radical"* is a heteroaryl group as defined previously, which includes a quaternized endocyclic or exocyclic cationic group;

o when the cationic charge is endocyclic, it is included in the electron delocalization via the mesomeric effect, for example it is a pyridinium, imidazolium or indolinium group:

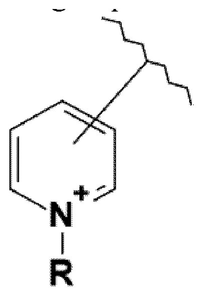
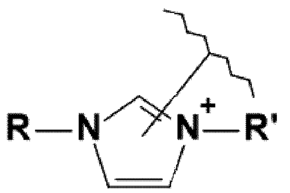
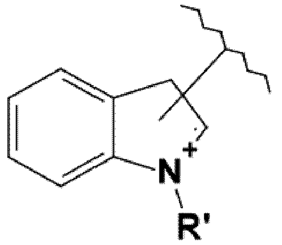

with R and R’ being a heteroaryl substituent as defined previously and particularly a (hydroxy)($C_1$-$C_8$)alkyl group such as methyl;

o when the charge is exocyclic, for example, it is an ammonium or phosphonium substituent $R^+$ such as trimethylammonium, which is exterior to the heteroaryl such as pyridyl, indolyl, imidazolyl or naphthalimidyl in question:

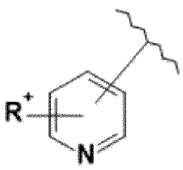
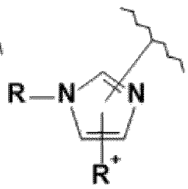
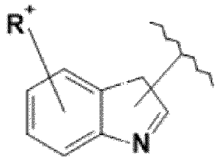
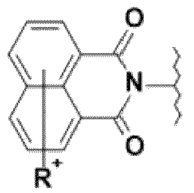
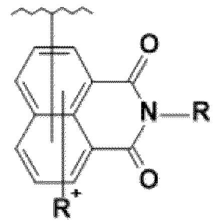

with R a heteroaryl substituent as defined above and $R^+$ an ammonium $R_aR_bR_cN^+$-, phosphonium $R_aR_bR_cP^+$- or ammonium $R_aR_bR_cN^+$-($C_1$-$C_6$)alkylamino group with $R_a$, $R_b$ and $R_c$, which may be identical or different, representing a hydrogen atom or a ($C_1$-Cs)alkyl group such as methyl;

- a "cationic aryl bearing an exocyclic charge" means an aryl ring whose quaternized cationic group is outside said ring: it is notably an ammonium or phosphonium substituent $R^+$ such as trimethylammonium, outside the aryl such as phenyl or naphthyl:

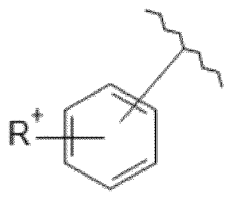
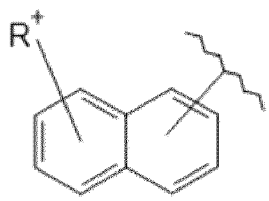

- an "alkyl radical" is a linear or branched $C_1$-$C_{20}$ and preferably $C_1$-$C_8$ hydrocarbon-based radical;
- an *"alkenylene radical"* is an unsaturated divalent hydrocarbon-based radical as defined previously, which may contain from 1 to 4 conjugated or unconjugated double bonds -C=C-; the alkenylene group particularly contains 1 or 2 unsaturations;
- the term "optionally substituted" attributed to the alkyl radical implies that said alkyl radical may be substituted with one or more radicals chosen from the following radicals: i) hydroxyl, ii) $C_1$-$C_4$ alkoxy, iii) acylamino, iv) amino optionally substituted with one or two identical or different $C_1$-$C_4$ alkyl radicals, said alkyl radicals possibly forming with the nitrogen atom that bears them a 5- to 7-membered heterocycle optionally comprising another nitrogen or non-nitrogen heteroatom; v) or a quaternary ammonium group $-N^+R'R''R'''$, $M^-$ for which R’, R" and R"’, which may be identical or different, represent a hydrogen atom or a $C_1$-$C_4$ alkyl group, or alternatively $-N^+R'R''R'''$ forms a heteroaryl such as imidazolium optionally substituted with a $C_1$-$C_4$ alkyl group, and $M^-$ represents the counterion of the organic or mineral acid or of the corresponding halide;
- an "alkoxy radical" is an alkyl-oxy radical for which the alkyl radical is a linear or branched $C_1$-$C_{16}$ and preferentially $C_1$-$C_8$ hydrocarbon-based radical; when the alkoxy group is optionally substituted, this implies that the alkyl group is optionally substituted as defined previously;
- the term *"organic or mineral acid salt"* more particularly means salts chosen from a salt derived from i) hydrochloric acid HCl, ii) hydrobromic acid HBr, iii) sulfuric acid $H_2SO_4$, iv) alkylsulfonic acids: Alk-$S(O)_2OH$ such as methylsulfonic

acid and ethylsulfonic acid; v) arylsulfonic acids: Ar-S(O)$_2$OH, such as benzenesulfonic acid and toluenesulfonic acid; vi) citric acid; vii) succinic acid; viii) tartaric acid; ix) lactic acid; x) alkoxysulfinic acids: Alk-O-S(O)OH such as methoxysulfinic acid and ethoxysulfinic acid; xi) aryloxysulfinic acids such as tolueneoxysulfinic acid and phenoxysulfinic acid; xii) phosphoric acid $H_3PO_4$; xiii) acetic acid $CH_3C(O)OH$; xiv) triflic acid $CF_3SO_3H$; and xv) tetrafluoroboric acid $HBF_4$;

- the term *"anionic counterion"* means an anion or an anionic group derived from an organic or mineral acid salt which counterbalances the cationic charge of the dye; more particularly, the anionic counterion is chosen from: i) halides such as chloride or bromide; ii) nitrates; iii) sulfonates, including $C_1$-$C_6$ alkylsulfonates: Alk-S(O)$_2$O$^-$ such as methanesulfonate or mesylate and ethanesulfonate; iv) arylsulfonates: Ar-S(O)$_2$O$^-$ such as benzenesulfonate and toluenesulfonate or tosylate; v) citrate; vi) succinate; vii) tartrate; viii) lactate; ix) alkyl sulfates: Alk-O-S(O)O$^-$ such as methyl sulfate and ethyl sulfate; x) aryl sulfates: Alk-O-S(O)O$^-$ such as benzene sulfate and toluene sulfate; xi) alkoxy sulfates: Alk-O-S(O)$_2$O$^-$ such as methoxy sulfate and ethoxy sulfate; xii) aryloxy sulfates: Ar-O-S(O)$_2$O$^-$, xiii) phosphates O=P(OH)$_2$-O$^-$, O=P(O$^-$)$_2$-OH O=P(O$^-$)$_3$, HO-[P(O)(O$^-$)]$_w$-P(O)(O$^-$)$_2$ with w being an integer; xiv) acetate; xv) triflate; and xvi) borates such as tetrafluoroborate, xvii) disulfate (O=)$_2$S(O$^-$)$_2$ or $SO_4^{2-}$ and monosulfate $HSO_4^-$; the anionic counterion, derived from the organic or mineral acid salt, ensures the electrical neutrality of the molecule: thus, it is understood that when the anion comprises several anionic charges, then the same anion may serve for the electrical neutrality of several cationic groups in the same molecule or else may serve for the electrical neutrality of several molecules; for example, polymers which comprise two cationic species may contain either two "singly charged" anionic counterions or a "doubly charged" anionic counterion such as (O=)$_2$S(O$^-$)$_2$ or O=P(O$^-$)$_2$-OH;
- moreover, the addition salts that may be used in the context of the invention are notably chosen from addition salts with a cosmetically acceptable base such as the basifying agents as defined below, for instance alkali metal hydroxides such as sodium hydroxide or potassium hydroxide, aqueous ammonia, amines or alkanolamines; and
- the term "inclusive" for a range of concentrations means that the limits of the range are included in the defined interval.

The term "hair keratin fibres" particularly means human hair keratin fibres such as the hair, the eyelashes and the eyebrows, and preferentially the hair. In the rest of the description, the terms "keratin fibres" and "hair keratin fibres" have the same meaning.

## Photocrosslinkable polymer

As indicated previously, the treatment composition, notably the hair dye composition, used in the context of the process according to the invention comprises at least one photocrosslinkable polymer. The term "photocrosslinkable polymer" means a polymer including one or more photodimerizable groups. The two terms will be used indiscriminately.
For the purposes of the present invention, the term "photodimerizable group" means a chemical group that leads to photodimerization reactions under irradiation. In the context of the invention, photodimerization is a chemical reaction between two double bonds (of 2+2 type) or two pairs of double bonds (of 4+4 type), and more particularly between two double bonds (of 2+2 type).
The case of a reaction between two double bonds may be represented schematically in the following manner:

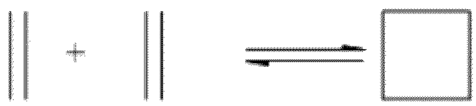

These photodimerization reactions are defined in the book Advanced Organic Chemistry, J. March, 4th edition, Wiley Interscience, NY 1992, page 855.
Thus, the double bond, when it is photo-stimulated, generally when it is subjected to UV radiation, proves to be capable of reacting with another double bond by cyclization.
According to the present invention, the double bond is said to be activated, i.e. it is spontaneously photodimerizable, without requiring the presence of a photoinitiator or a chemical initiator.
This double bond is generally activated by the presence of an electron-withdrawing substituent in the alpha position of this photodimerizable double bond. As electron-withdrawing substituents, mention may be made of aromatic nuclei such as the phenyl group optionally substituted with one or more halogen atoms, or electron-withdrawing groups such as $NO_2$, CN, R'-Y-C(Y')-, R'-C(Y')-Y-, R'-Y-C(Y')-Y-, R'-Y-S(O)$_2$- or -S(O)$_2$-Y-R', where R' represents a hydrogen atom or a ($C_1$-$C_4$)alkyl group optionally substituted with one or more halogen atoms, where Y and Y', which may be identical or different, represent an oxygen or sulfur atom or NR" where R" represents a hydrogen atom or a ($C_1$-$C_6$)alkyl group.
The process according to the invention comprises a step a) of applying a composition comprising a polymer including at least one photodimerizable group, preferably a pendent group.

Preferably, the photodimerizable groups that may be used according to the invention are chosen from the monovalent radicals of formulae (I) and (II) below:

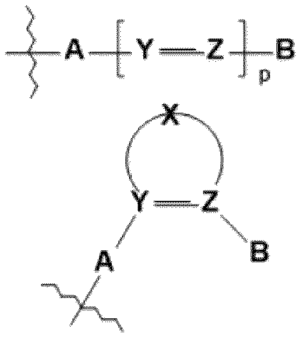

and also the geometrical isomers thereof,
in which formulae (I) and (II):

- Y and Z denote, independently of each other, a nitrogen atom or a C(R) group with R representing a hydrogen atom or a $(C_1-C_4)$alkyl group such as methyl;
- A represents a bond or a divalent group chosen from $(C_1-C_8)$alkylene, arylene, heteroarylene, cycloalkylene, heterocycloalkylene, (thio)carbonyl, $(C_2-C_8)$alkenylene radicals and combinations thereof;
- B represents a monovalent group chosen from $(C_1-C_8)$alkyl radicals, aryl, optionally cationic heteroaryl, cycloalkyl, optionally cationic heterocycloalkyl, (thio)carbonyl, $(C_2-C_8)$alkenyl radicals and combinations thereof;
- X represents a divalent group chosen from $(C_2-C_8)$alkylene, arylene, heteroarylene, cycloalkylene, heterocycloalkylene, (thio)carbonyl, $(C_2-C_8)$alkenylene radicals and combinations thereof;
- p represents an integer between 1 and 5 inclusive, more particularly between 1 and 3, preferably p is 1;

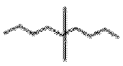

represents the bond that connects the part of the monovalent radical to the rest of the molecule;
and
each of the groups mentioned may optionally be substituted with one or more halogen atoms or groups chosen from the following: $(C_1-C_6)$alkyl, hydroxyl, amino, (di)$(C_1-C_6)$alkylamino, phenyl, carboxyl, $(C_1-C_6)$alkoxy, $(C_1-C_6)$alkoxy(thio)carbonyl, hydrogeno(thio)carbonyl, sulfonato $R-O-S(O)_2-$ or $R-S(O)_2-O-$, amide RR'N-C(O)- or R-C(O)-N(R')- or acyl R-C(O)-, ammonium $RR'R''N^+-$ with R, R' and R", which may be identical or different, representing a hydrogen atom or a $(C_1-C_4)$alkyl group.

The dimerizable pendent groups according to the invention are notably those mentioned in patents US-2 811 510, EP 0 313 220, EP 0 313 221, EP 092 901, GB 2 030 575 and GB 2 076 826, and also in the articles "Chemical Review Vol. 83, 5, 1983, page 507" "Polym, Paint Colour Journal 1988, 178, page 209" and "Current Trends in Polymer Photochemistry, Ellis Morwood edition, NY, 1995".

By way of example, mention may be made more particularly of the photodimerizable pendent groups chosen from the monovalent radicals of the following compounds:

- stilbene,
- styrylpyridinium (stilbazolium) having the formula below, and the geometrical isomers thereof:

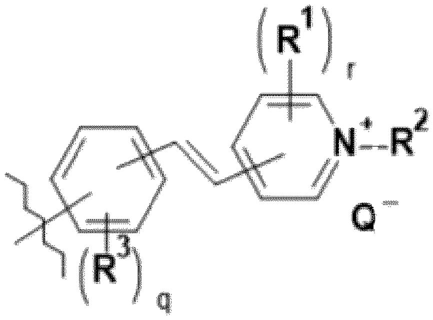

(A1)

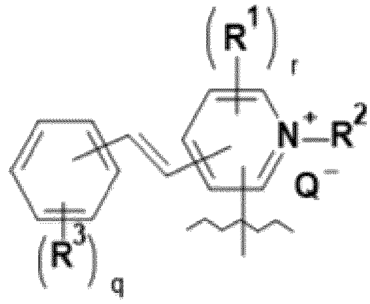

(A2)

where:

- **$R^1$** and **$R^3$**, which may be identical or different, represent a halogen atom or a $(C_1-C_6)$alkyl group; or else two contiguous $R^1$ or $R^3$ groups form, together with the carbon atoms bearing them, a benzo group;
- $R^2$ represents a hydrogen atom, a $(C_1-C_6)$alkyl group optionally substituted with one or more halogen atoms such as chlorine or hydroxyl; preferably, $R^2$ represents a $(C_1-C_6)$alkyl group such as methyl, ethyl or propyl;
- q and r represent an integer between 0 and 4 inclusive; and
- $Q^-$ represents an anionic counter ion preferably chosen from halide ions such as chlorides, bromides and iodides, perchlorates, tetrafluoroborates, methyl sulfate, phosphates, sulfates, methanesulfonates, p-toluenesulfonate;
-

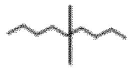

represents the bond which connects the part of the monovalent radical to the rest of the molecule, it being understood that the pendent group $A_2$ can be connected to the rest of the molecule *via* $R^2$; preferably, the

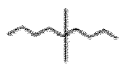

bond is located on the phenyl para to the styryl group on $A_1$ or connected to the rest of the molecule *via* $R^2$ on $A_2$; preferentially, the styryl group of $A_1$ and $A_2$ is located in the position para to the pyridinium group;

- styrylazolium of the formula below, and the geometrical isomers thereof:

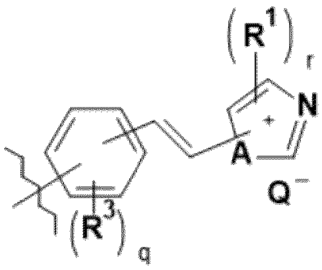

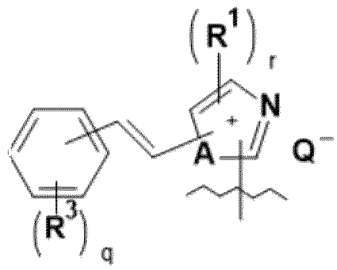

where:

- A represents a sulfur atom, an oxygen atom, or a group $NR^2$ or $C(R^2)_2$; and
-

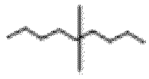

, $Q^-$, r, q, $R^1$, $R^2$ and $R^3$ being as defined previously, preferably, the

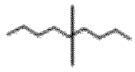

bond is located on the phenyl in the position para to the stryryl group,

- styrylpyrazine,
- chalcone,
- (thio)cinnamate and (thio)cinnamamide,
- maleimide,
- (thio)coumarin,
- thymine,
- uracil,
- butadiene,
- anthracene,
- pyridone,
- pyrrolizinone,
- acridizinium salts,
- furanone,
- phenylbenzoxazole, and
- derivatives thereof.

According to a particular embodiment, the photodimerizable pendent group(s) of the invention are chosen from:

a) photodimerizable group(s) bearing a stilbazolium function of formula (Ia) or (Ib) and the geometrical isomers thereof:

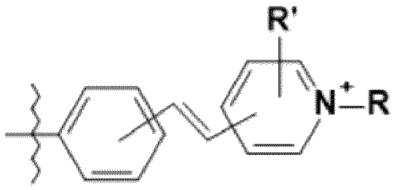

$X^-$ (Ia)

in which:

- R represents a hydrogen atom or a $C_1$-$C_4$ alkyl or $C_1$-$C_4$hydroxyalkyl group;
- R' represents a hydrogen atom or a $C_1$-$C_4$ alkyl group, and
- $X^-$ denotes an anionic counterion chosen from halide ions such as chlorides, bromides, iodides, perchlorates, tetrafluoroborates, methyl sulfate, phosphates, sulfate, methanesulfonate,

p-toluenesulfonate; preferably, the styryl group is located in the para position of the pyridinium group and/or para to the

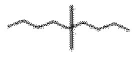

bond;

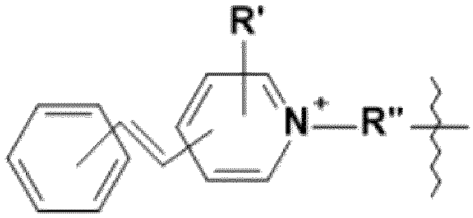

$X^-$ (Ib)

in which:

- R" denotes a divalent alkylene radical containing from 2 to 8 carbon atoms,
- R' represents a hydrogen atom or a $C_1$-$C_4$ alkyl group, and
- $X^-$ has the same meaning as that described for the preceding formula (Ia);
- where

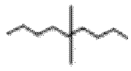

has the same meaning as previously;

preferably, the styryl group is located in the para position of the pyridinium group;
or
b) photodimerizable groups bearing a styrylazolium function of formula (IIa):

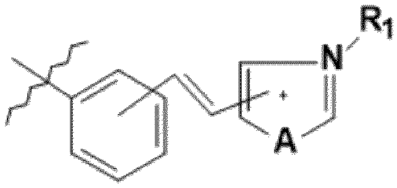

$X^-$(IIa)

in which:

- $R_1$ denotes a hydrogen atom or a $C_1$-$C_4$ alkyl or $C_1$-$C_4$hydroxyalkyl group;
- A denotes a sulfur atom, an oxygen atom or a group NR' or $C(R')_2$, R'; with R' representing a hydrogen atom or a $C_1$-$C_4$ alkyl group, R' preferably representing a hydrogen atom; and
- $X^-$ has the same meaning as that described for the preceding formula (Ia);
- where

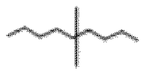

has the same meaning as previously;

preferably, the styryl group is located on the phenyl para to the

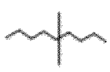

bond.

Such photodimerizable pendent groups bear activated double bonds, and as such the photodimerization of these double bonds starts spontaneously in the UVA range, without requiring a photoinitiator.

For the purposes of the present invention, the term "photoinitiator" means a compound which initiates the photodimerization reaction and releases a radical under irradiation, notably in the UV range.

According to a particular embodiment, the composition of the invention is free of photoinitiators.

According to a particular embodiment of the invention, the polymer may include one or more pendent hydrophobic groups.

As pendent hydrophobic group, mention may be made of:

- saturated or unsaturated ($C_1$-$C_{30}$)alkyl groups, optionally substituted and/or interrupted with one or more heteroatoms,
- alkenyl groups,
- aryl groups such as phenyl, pyridyl, furyl, indolyl, benzofuryl, thiophenyl, imizadolyl, oxazolyl, thiazolyl, pyrazinyl or pyrimidinyl;
- fluorinated groups such as fluorocarbon groups such as $-CF_3$, $-CHF_2$, $-OCF_3$, - $SCF_3$, $CF_3C(O)$-,
- silicone groups such as $-SiR_aR_bR_c$ such as $-Si(CH_3)_3$, polydimethylsiloxane PDMS, $-Si(OR)_3$, PDMS $\alpha,\omega$-diaminopropyl, PDMS $\alpha,\omega$-dihydroxyalkyl, PDMS $\alpha,\omega$-dicarboxyalkyl, with $R_a$, $R_b$ and $R_c$, which may be identical or different, representing a ($C_1$-$C_8$)alkyl group which is optionally interrupted and/or terminated with one or more non-contiguous heteroatoms such as O or S; and R representing a ($C_1$-$C_6$)alkyl group.

Preferably, the preferably pendent hydrophobic group(s) are chosen from ($C_2$-$C_{22}$)alkyl groups, more preferentially ($C_3$-$C_{16}$)alkyl groups, better still ($C_3$-$C_{12}$) groups.

The polymer backbone may be of varied nature. This polymer backbone may be natural or synthetic. As natural polymer backbones, mention may be made of polysaccharides.

As polysaccharides, mention may be made of xanthan, carrageenan, chitosan, cellulose and its derivatives, alginate, starch, dextran, pullulan, galactomannan and the biologically acceptable salts thereof, and derivatives thereof.

As synthetic backbones, mention may be made of poly(vinyl) polymers and polydiorganosiloxanes.

Among the poly(vinyl) polymers, mention may be made of partially or totally hydrolysed poly(vinyl acetate)s, and polyvinyl alcohol (PVA).

As regards the polymers containing pendent photodimerizable groups bearing a stilbazolium function, they are obtained by reacting the polymer under consideration with a chemical species including a group of formula (Ia) or (Ib).

Preferably, the chemical species including a group (Ia) bears a reactive group W of aldehyde or acetal type.

As chemical species that may be used for grafting styrylpyridinium type groups, mention may notably be made of quaternary salts of 2-(4-formylstyryl)pyridinium, 4-(4-formylstyryl)pyridinium, 2-(3-formylstyryl)pyridinium, N-methyl-2-(4-formylstyryl)pyridinium, N-methyl-3-(4-formylstyryl)pyridinium, N-methyl-2-(3-formylstyryl)pyridinium, N-methyl-2-(2-formylstyryl)pyridinium, N-ethyl-2-(4-formylstyryl)pyridinium, N-(2-hydroxyethyl)-2-(4-formylstyryl)pyridinium, N-(2-hydroxyethyl)-4-(4-formylstyryl)pyridinium, N-methyl-4-(4-formylstyryl)pyridinium or N-methyl-4-(3-formylstyryl)pyridinium.

The pyridinium quaternary salts may be chloride, bromide, iodide, perchlorate, tetrafluoroborate, methosulfate, phosphate, sulfate, methanesulfonate or p-toluenesulfonate salts. Such chemical species are described in GB-A-2030575.

Examples of species that may be mentioned include 4-(4-formylphenylethenyl)-1-methylpyridinium methosulfate, 1-(3-ethoxycarbonylmethyl)-4-[2-(4-formylphenyl)-ethenyl]pyridinium bromide and 1-(methoxycarbonylpropyl)-4-[2-(4-formylphenyl)ethenyl]pyridinium bromide. Such species are described in
US 2007/0 112 094.

Use is preferably made of n-methyl-4-(4-formylstyryl)pyridinium methyl sulfate (RN= 74401-04-0), notably sold by the company Wako.

The synthesis, described below, of these polymers functionalized with photodimerizable groups such as those comprising a styryl group and hydrophobic groups may be performed on the basis of the protocol of T. Uhlich et al. (Reactive & Functional Polymers, 28, 55-40 (1995)).

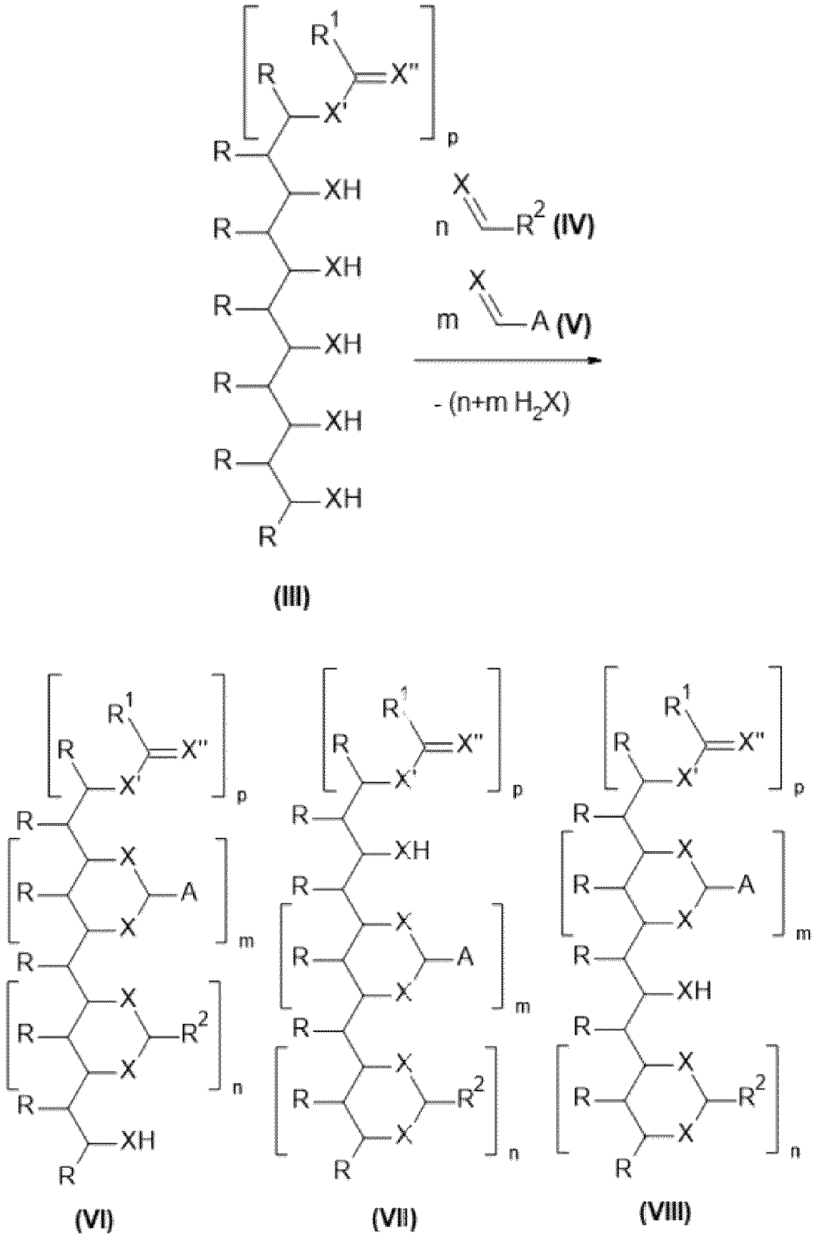


(III)

(VI) (VII) (VIII)

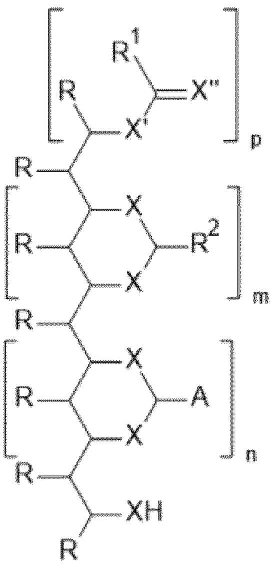
(VI')

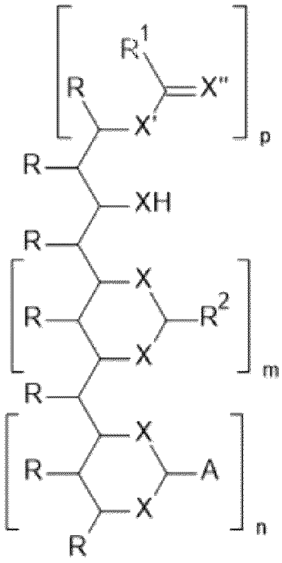
(VII')

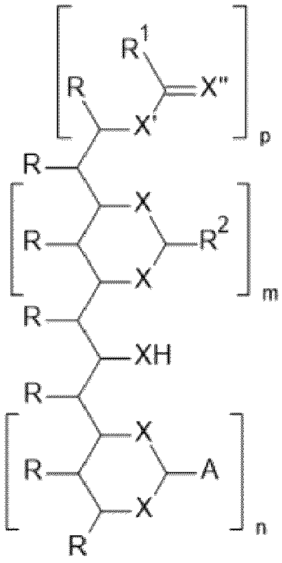
(VIII')

in which compounds of formulae **(III) to (VIII')**

**R,** which may be identical or different, represents a hydrogen atom or a ($C_1$-$C_{10}$)alkyl group, optionally substituted and/or interrupted with one or more heteroatoms; preferably, R represents a hydrogen atom or a ($C_1$-$C_4$)alkyl group such as methyl, ethyl or propyl; more preferentially, R represents a hydrogen atom;

**$R^1$** represents a hydrogen atom or a ($C_1$-$C_{10}$)alkyl group, optionally substituted and/or interrupted with one or more heteroatoms; preferably, $R^1$ represents a hydrogen atom or a ($C_1$-$C_4$)alkyl group;

**$R^2$** represents a saturated or unsaturated ($C_1$-$C_{30}$)alkyl group, optionally substituted and/or interrupted with one or more heteroatoms, an alkenyl group, an aryl group such as phenyl, pyridyl, furyl, indoyl, benzofuryl, thiophenyl, imizadoyl, oxazoyl, thiazoyl, pyrazinyl or pyrimidinyl; a fluoro group such as a fluorocarbon group such as $-CF_3$, $-CHF_2$, $-OCF_3$, $-SCF_3$ or $CF_3C(O)-$; a silicone group such as $-SiR_aR_bR_c$ such as $-Si(CH_3)_3$, polydimethylsiloxane -PDMS, $-Si(OR)_3$, PDMS $\alpha,\omega$-diaminopropyl, PDMS $\alpha,\omega$-dihydroxyalkyl, PDMS $\alpha,\omega$-dicarboxyalkyl, with $R_a$, $R_b$ and $R_c$, which may be identical or different, representing a ($C_1$-$C_8$)alkyl group optionally interrupted and/or terminated with one or more non-contiguous heteroatoms such as O, or S; and R representing a ($C_1$-$C_6$)alkyl group; preferably, $R^2$ represents a ($C_2$-$C_{22}$)alkyl group, more preferentially ($C_3$-$C_{16}$)alkyl;

**A** represents a group obtained from a photodimerizable compound, preferably a styrylpyridinium such as (I), (II), ($A_1$), ($A_2$), (Ia), (Ib) or (IIa) as defined previously, more particularly chosen from ($A_1$) or (Ia) as defined previously;

**X** represents an oxygen or sulfur atom, preferably oxygen;

**X'** and **X"** represent an oxygen or sulfur atom or a group $N(R^3)$ with **$R^3$** representing a hydrogen atom or a ($C_1$-$C_4$)alkyl group; preferably, X' and X" represent an oxygen atom.

The products obtained are predominantly of formula **(VI).**

Advantageously, these chemical species react with a polymer of polyvinyl alcohol or polyvinyl acetal type as described in the documents mentioned previously and also such as the polymer **(III)** described in the above scheme for which X, X' and X" represent an oxygen atom, R and $R^1$ being as described previously.

For example, a grafted polyvinyl alcohol polymer including the following structure units is thus obtained, where A represents a group (I), ($A_1$) or (Ia):

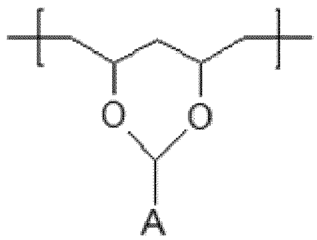

Polyvinyl alcohol polymers grafted with a styrylpyridinium group are notably described in the publication Ichimura K. et al., Preparation and characteristics of photocrosslinkable poly(vinyl alcohol), Journal of Polymer Science, Polymer Chemistry Edition, Vol. 20, 1419-1432 (1982).

The polymers may be obtained by reacting polyvinyl alcohol or partially hydrolysed polyvinyl acetate with styrylpyridinium salts bearing a formyl or acetal group as described in GB-A-2 030 575, WO 96/29312, US 5 061 603, GB-A-2 076 826 and EP-A-092 901.

Cellulose polymers grafted with styrylpyridinium groups are notably described in US 2007/0 112 094.

Preferably, the chemical species including a group ($A_1$) or (Ia) bears a reactive group which is a halogen atom such as chlorine.

In this variant, the chemical species has, for example, the formula:

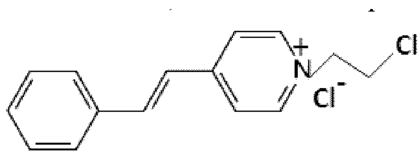

Advantageously, the photocrosslinkable polymer comprising the groups (Ib) is obtained, for example, by reaction of the above species with the polysaccharide chosen from those defined previously.

As regards the polymers containing photodimerizable groups bearing a styrylazolium function, they are obtained by reacting the polymer with a chemical species including a group of formula (IIa).

Preferably, the chemical species including a group (IIa) bears a reactive group W of aldehyde or acetal type.

As chemical species that may be used for grafting groups of styrylazolium type, mention may be made of those described in EP-A-313 220.

Advantageously, these chemical species react with a polymer of polyvinyl alcohol or polyvinyl acetate type as described in the documents mentioned previously.

A polyvinyl alcohol grafted polymer including units having the following structure is thus obtained:

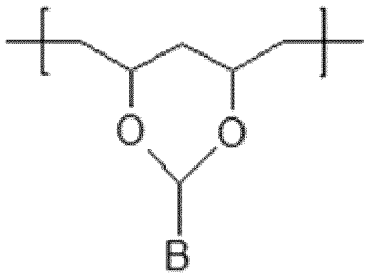

with B corresponding to the group

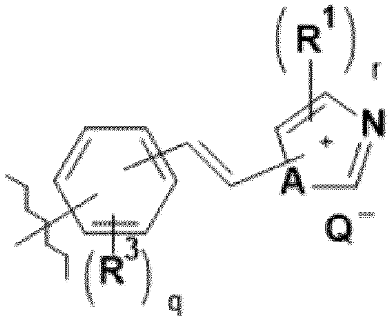

or (IIa) as defined previously.

Polyvinyl alcohol polymers grafted with styrylazolium groups are notably described in EP-A-313 220. In said document, these polymers may be obtained by reaction of polyvinyl alcohol or partially hydrolysed polyvinyl acetate with styrylazolium salts bearing an aldehyde or acetal group.

According to one embodiment, the polymer bearing photodimerizable group(s) is in the form of particles, in particular of dispersed particles. Thus, in the latter case, the polymer particles are more particularly polyvinyl alcohol particles.

According to a preferred embodiment, the polymer bearing photodimerizable group(s) of the invention is soluble in the cosmetic medium.

Thus, according to one embodiment variant, the polymer is a polyvinyl acetate (PVA) type polymer partly functionalized with one or more hydroxyl functions and one or more functions of formula (IX):

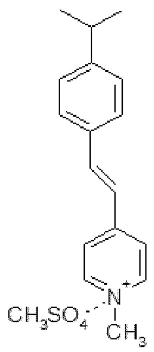

(IX)

The degree of polymerization of PVA may be between 100 and 5000 and the degree of substitution, as a percentage of functions of formula (I) as defined above, may be between 0.1 and 25.

The following diagram represents a variant in which the polymer is the polymer (III) as defined previously bearing functions grafted with stilbazolium species such as those of formula ($A_1$) as defined previously, which is capable of crosslinking under the effect of light, as illustrated below.

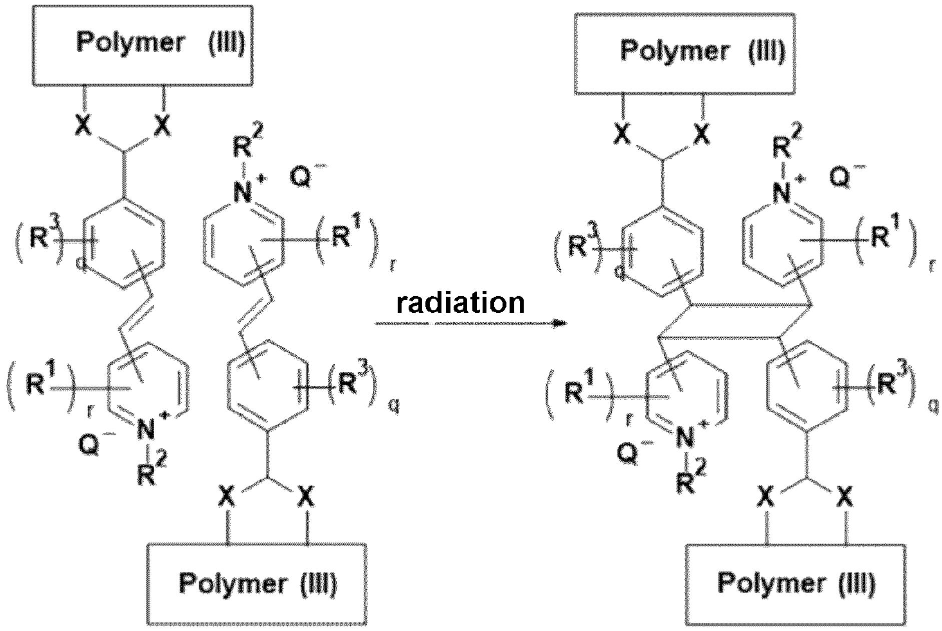


These materials react to radiation that may include both a UV light and visible light component, particularly a low dose of UV.

Preferentially, the following scheme represents the polymer which is PVA-SbQ (PVAcetate type polymer bearing a few hydrolysed functions and a few functions grafted with stilbazolium species), which is capable of crosslinking under the effect of light, as illustrated below.

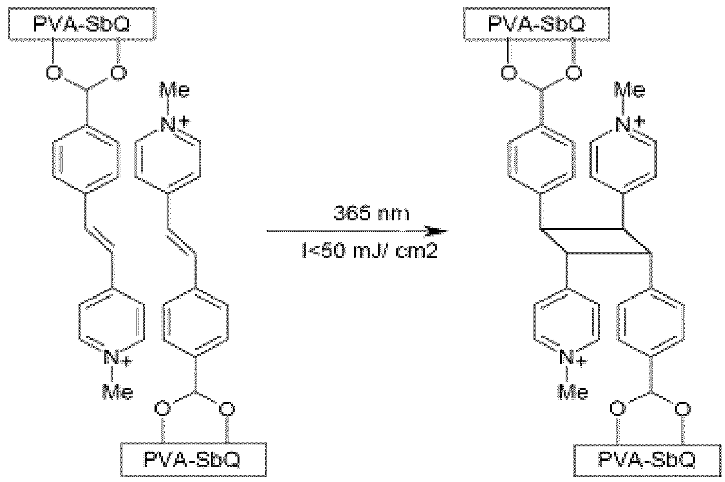


These materials are particularly appreciated since they do not require a photoinitiator and react with radiation that may include both UV light and visible light, in particular a low dose of UV.

Pendent groups which have reactivity both in UV light and in visible light are favoured.

According to another embodiment variant, the photocrosslinkable polymer is featured by a natural polymer which is functionalized with photodimerizable groups.

It may notably be a polysaccharide that may notably be chosen from chondroitin sulfate, keratan, keratan sulfate, heparin, heparin sulfate, xanthan, carrageenan, hyaluronic acid, chitosan, cellulose and derivatives thereof, alginate, starch, dextran, pullulan, galactomannan and biologically acceptable salts thereof.

Needless to say, the degree of functionalization is adjusted to be able to provide the degree of crosslinking required during activation.

According to the invention, the degree of functionalization with photodimerizable units is at least 0.1%, or even at least 0.5%, or even at least 2%.

Preferably, in a composition according to the invention, the photodimerizable groups are borne by a polymer of polyvinyl acetate or polysaccharide type.

The crosslinkable polymer may be conveyed in an aqueous medium.

The composition may contain a single polymer bearing photodimerizable pendent groups that may or may not be of different nature.

Use may also be made of a blend of polymers having different functions.

Consequently, the reactions may take place between two photodimerizable groups that may or may not be of the same chemical nature.

The activated double bonds may react with another double bond of the same chemical nature or may react with another double bond of different chemical nature.

As an example of a polymer that is useful in the invention, mention may be made of the partially or totally hydrolysed polyvinyl acetate polymer comprising the units below, in particular the polymer known as PVA-SbQ-propyl, in which the amount of SbQ unit is between, limits inclusive, 0.5 and 6 mol%, preferably between 1.5 and 5 mol%, the amount of propyl group is between, limits inclusive, 2 and 20 mol%, preferably between 5 and 15 mol%, better still from 8% to 12%, the amount of hydroxyl group is between, limits inclusive, 50 and 97.5 mol%, preferably between 60 and 97.5 mol%, better still from 80 to 90 mol%:

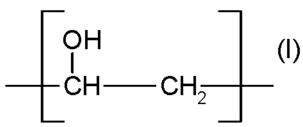 (I)

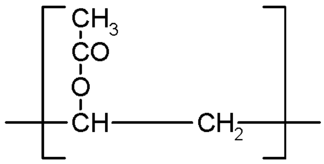 (II)

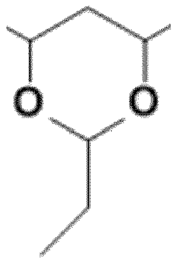

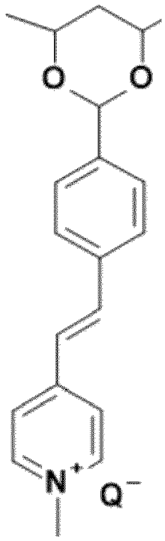

with $Q^-$ representing an anionic counterion preferably chosen from halide ions such as chlorides, bromides, iodides, perchlorates, tetrafluoroborates, methylsulfate, phosphates, sulfates, methanesulfonates and p-toluenesulfonate, preferably mesylate $CH_3OSO_3^-$.

According to a particular embodiment, the molecular weight Mw of the PVA is between 10 000 and 100 000 g/mol, preferably between 15 000 and 80 000 g/mol, more particularly from 20 000 to 50 000, even better still from 25 000 to 30 000 g/mol.

The total amount of the polymer(s) including at least one photodimerizable group and at least one hydrophobic group, present in the composition according to the invention, preferably ranges from 0.01% to 40% by weight, more preferentially from 0.1% to 30% by weight, better still from 0.5% to 25% by weight, even better still from 1% to 10% by weight relative to the total weight of the composition (C).

The composition according to the invention may also comprise an effective amount of at least one photosensitizer.

For the purposes of the present invention, the term "photosensitizer" means an ingredient which modifies the irradiation wavelength, thus triggering the photodimerization reaction.

For example, the photodimerization of dimethylmaleimide groups is triggered by irradiation centred on the wavelength range from 270 to 300 nm. In the presence of a photosensitizer such as thioxanthone, photodimerization becomes effective with irradiation centred on the wavelength range from 360 to 430 nm.

Among the photosensitizers that may be used according to the invention, mention may notably be made of

thioxanthone, rose Bengal, phloxine, eosin, erythrosine, fluorescein, acriflavine, thionine, riboflavin, proflavine, chlorophylls, haematoporphyrin, methylene blue and mixtures thereof.

In practice, the photosensitizer that may be used according to the invention represents 0.00001% to 5% by total weight of the composition.

The total amount of photodimerizable polymer(s) (including at least one photodimerizable group), present in the hair dye composition, preferably ranges from 0.01% to 30% by weight, more preferentially from 0.1% to 25% by weight, better still from 0.2% to 20% by weight and even better still from 1% to 15% by weight relative to the total weight of the hair dye composition.

## Colouring agent

As indicated previously, the hair dye composition used in the context of the process according to the invention comprises at least one colouring agent chosen from pigments, direct dyes and mixtures thereof.

### Pigments

Preferably, the hair dye composition comprises one or more pigments.

The term "pigment" refers to any pigment that gives colour to keratin fibres. Their solubility in water at 25°C and at atmospheric pressure (760 mmHg) is less than 0.05% by weight, and preferably less than 0.01%.

The pigments that may be used are notably chosen from the organic and/or mineral pigments known in the art, notably those described in Kirk-Othmer's *Encyclopedia of Chemical Technology* and in Ullmann's *Encyclopedia of Industrial Chemistry.*

They may be natural, of natural origin, or non-natural.

These pigments may be in pigment powder or paste form. They may be coated or uncoated.

The pigments may be chosen, for example, from mineral pigments, organic pigments, lakes, pigments with special effects such as nacres or glitter flakes, and mixtures thereof.

The pigment may be a mineral pigment. The term "mineral pigment" refers to any pigment that satisfies the definition in Ullmann's encyclopedia in the chapter on inorganic pigments. Among the mineral pigments that are useful in the present invention, mention may be made of iron oxides, chromium oxides, manganese violet, ultramarine blue, chromium hydrate, ferric blue and titanium oxide.

The pigment may be an organic pigment. The term "organic pigment" refers to any pigment that satisfies the definition in Ullmann's Encyclopedia in the chapter on organic pigments.

The organic pigment may notably be chosen from nitroso, nitro, azo, xanthene, pyrene, quinoline, anthraquinone, triphenylmethane, fluorane, phthalocyanine, metal-complex, isoindolinone, isoindoline, quinacridone, perinone, perylene, diketopyrrolopyrrole, indigo, thioindigo, dioxazine, triphenylmethane and quinophthalone compounds.

In particular, the white or coloured organic pigments may be chosen from carmine, carbon black, aniline black, azo yellow, quinacridone, phthalocyanine blue, the blue pigments codified in the Color Index under the references CI 42090, 69800, 69825, 74100, 74160, the yellow pigments codified in the Color Index under the references CI 11680, 11710, 19140, 20040, 21100, 21108, 47000, 47005, the green pigments codified in the Color Index under the references CI 61565, 61570, 74260, the orange pigments codified in the Color Index under the references CI 11725, 45370, 71105, the red pigments codified in the Color Index under the references CI 12085, 12120, 12370, 12420, 12490, 14700, 15525, 15580, 15620, 15630, 15800, 15850, 15865, 15880, 26100, 45380, 45410, 58000, 73360, 73915, 75470, the pigments obtained by oxidative polymerization of indole or phenol derivatives as described in patent FR 2 679 771.

Examples that may also be mentioned include pigment pastes of organic pigments, such as the products sold by the company Hoechst under the names:

- Cosmenyl Yellow 10G: Yellow 3 pigment (CI 11710);
- Cosmenyl Yellow G: Yellow 1 pigment (CI 11680);
- Cosmenyl Orange GR: Orange 43 pigment (CI 71105);
- Cosmenyl Red R: Red 4 pigment (CI 12085);
- Cosmenyl Carmine FB: Red 5 pigment (CI 12490);
- Cosmenyl Violet RL: Violet 23 pigment (CI 51319);
- Cosmenyl Blue A2R: Blue 15.1 pigment (CI 74160);
- Cosmenyl Green GG: Green 7 pigment (CI 74260);
- Cosmenyl Black R: Black 7 pigment (CI 77266).

The pigments in accordance with the invention may also be in the form of composite pigments, as described in patent EP 1 184 426. These composite pigments may notably be composed of particles including a mineral core, at least

one binder for attaching the organic pigments to the core, and at least one organic pigment which at least partially covers the core.

The organic pigment may also be a lake. The term "lake" refers to dyes adsorbed onto insoluble particles, the assembly thus obtained remaining insoluble during use.

The mineral substrates onto which the dyes are adsorbed are, for example, alumina, silica, calcium sodium borosilicate or calcium aluminium borosilicate and aluminium.

Among the dyes, mention may be made of carminic acid. Mention may also be made of the dyes known under the following names: D&C Red 21 (CI 45 380), D&C Orange 5 (CI 45 370), D&C Red 27 (CI 45 410), D&C Orange 10 (CI 45 425), D&C Red 3 (CI 45 430), D&C Red 4 (CI 15 510), D&C Red 33 (CI 17 200), D&C Yellow 5 (CI 19 140), D&C Yellow 6 (CI 15 985), D&C Green 5 (CI 61 570), D&C Yellow 10 (CI 77 002), D&C Green 3 (CI 42 053), D&C Blue 1 (CI 42 090).

An example of a lake that may be mentioned is the product known under the following name: D&C Red 7 (CI 15 850:1).

The pigment may also be a pigment with special effects. The term "pigments with special effects" means pigments that generally create a coloured appearance (characterized by a certain shade, a certain vivacity and a certain level of luminance) that is non-uniform and that changes as a function of the conditions of observation (light, temperature, angles of observation, etc.). They thereby differ from coloured pigments, which afford a standard uniform opaque, semi-transparent or transparent shade.

Several types of pigments with special effects exist: those with a low refractive index, such as fluorescent or photochromic pigments, and those with a higher refractive index, such as nacres, interference pigments or glitter flakes.

Examples of pigments with special effects that may be mentioned include nacreous pigments such as mica coated with titanium or with bismuth oxychloride, coloured nacreous pigments such as mica covered with titanium and with iron oxides, mica covered with iron oxide, mica covered with titanium and notably with ferric blue or with chromium oxide, mica covered with titanium and with an organic pigment as defined previously, and also nacreous pigments based on bismuth oxychloride. Nacreous pigments that may be mentioned include the nacres Cellini sold by BASF (mica-$TiO_2$-lake), Prestige sold by Eckart (mica-$TiO_2$), Prestige Bronze sold by Eckart (mica-$Fe_2O_3$) and Colorona sold by Merck (mica-$TiO_2$-$Fe_2O_3$).

Mention may also be made of the gold-coloured nacres sold notably by the company BASF under the name Brilliant gold 212G (Timica), Gold 222C (Cloisonne), Sparkle gold (Timica), Gold 4504 (Chromalite) and Monarch gold 233X (Cloisonne); the bronze nacres sold notably by the company Merck under the name Bronze fine (17384) (Colorona) and Bronze (17353) (Colorona) and by the company BASF under the name Super bronze (Cloisonne); the orange nacres sold notably by the company BASF under the name Orange 363C (Cloisonne) and Orange MCR 101 (Cosmica) and by the company Merck under the name Passion orange (Colorona) and Matte orange (17449) (Microna); the brown nacres sold notably by the company BASF under the name Nu-antique copper 340XB (Cloisonne) and Brown CL4509 (Chromalite); the nacres with a copper tint sold notably by the company BASF under the name Copper 340A (Timica); the nacres with a red tint sold notably by the company Merck under the name Sienna fine (17386) (Colorona); the nacres with a yellow tint sold notably by the company BASF under the name Yellow (4502) (Chromalite); the red nacres with a gold tint sold notably by the company BASF under the name Sunstone G012 (Gemtone); the pink nacres sold notably by the company BASF under the name Tan opale G005 (Gemtone); the black nacres with a gold tint sold notably by the company BASF under the name Nu antique bronze 240 AB (Timica), the blue nacres sold notably by the company Merck under the name Matte blue (17433) (Microna), the white nacres with a silvery tint sold notably by the company Merck under the name Xirona Silver, and the golden-green pink-orange nacres sold notably by the company Merck under the name Indian summer (Xirona), and mixtures thereof.

Still as examples of nacres, mention may also be made of particles including a borosilicate substrate coated with titanium oxide.

Particles comprising a glass substrate coated with titanium oxide are notably sold under the name Metashine MC1080RY by the company Toyal.

Finally, examples of nacres that may also be mentioned include polyethylene terephthalate glitter flakes, notably those sold by the company Meadowbrook Inventions under the name Silver 1P 0.004X0.004 (silver glitter flakes). It is also possible to envisage multilayer pigments based on synthetic substrates, such as alumina, silica, calcium sodium borosilicate, calcium aluminium borosilicate and aluminium.

The pigments with special effects may also be chosen from reflective particles, i.e. notably from particles whose size, structure, notably the thickness of the layer(s) of which they are made and their physical and chemical nature, and surface state, allow them to reflect incident light. This reflection may, where appropriate, have an intensity sufficient to create at the surface of the composition or of the mixture, when it is applied to the support to be made up, highlight points that are visible to the naked eye, i.e. more luminous points that contrast with their environment making them appear to sparkle.

The reflective particles may be selected so as not to significantly alter the colouring effect generated by the colouring agents with which they are combined, and more particularly so as to optimize this effect in terms of colour

rendition. They may more particularly have a yellow, pink, red, bronze, orange, brown, gold and/or coppery colour or tint.

These particles may have varied forms and may notably be in platelet or globular form, in particular in spherical form.

The reflective particles, whatever their form, may or may not have a multilayer structure and, in the case of a multilayer structure, may have, for example, at least one layer of uniform thickness, notably of a reflective material.

When the reflective particles do not have a multilayer structure, they may be composed, for example, of metal oxides, notably titanium or iron oxides obtained synthetically.

When the reflective particles have a multilayer structure, they may include, for example, a natural or synthetic substrate, notably a synthetic substrate at least partially coated with at least one layer of a reflective material, notably of at least one metal or metallic material. The substrate may be made of one or more organic and/or mineral materials.

More particularly, it may be chosen from glasses, ceramics, graphite, metal oxides, aluminas, silicas, silicates, notably aluminosilicates and borosilicates, and synthetic mica, and mixtures thereof, this list not being limiting.

The reflective material may include a layer of metal or of a metallic material.

Reflective particles are notably described in JP-A-09188830, JP-A-10158450, JP-A-10158541, JP-A-07258460 and JP-A-05017710.

Again as an example of reflective particles including a mineral substrate coated with a layer of metal, mention may also be made of particles including a silver-coated borosilicate substrate.

Particles with a silver-coated glass substrate, in the form of platelets, are sold under the name Microglass Metashine REFSX 2025 PS by the company Toyal. Particles with a glass substrate coated with a nickel/chromium/molybdenum alloy are sold under the names Crystal Star GF 550 and GF 2525 by this same company.

Use may also be made of particles comprising a metal substrate, such as silver, aluminium, iron, chromium, nickel, molybdenum, gold, copper, zinc, tin, magnesium, steel, bronze or titanium, said substrate being coated with at least one layer of at least one metal oxide, such as titanium oxide, aluminium oxide, iron oxide, cerium oxide, chromium oxide, silicon oxides and mixtures thereof.

Examples that may be mentioned include aluminium powder, bronze powder or copper powder coated with $SiO_2$ sold under the name Visionaire by the company Eckart.

Mention may also be made of interference pigments which are not attached to a substrate, such as liquid crystals (Helicones HC from Wacker) or interference holographic glitter flakes (Geometric Pigments or Spectra f/x from Spectratek). Special effect pigments also comprise fluorescent pigments, whether these are substances that are fluorescent in daylight or that produce an ultraviolet fluorescence, phosphorescent pigments, photochromic pigments, thermochromic pigments and quantum dots, sold, for example, by the company Quantum Dots Corporation.

The variety of pigments that may be used in the present invention makes it possible to obtain a wide range of colours, and also particular optical effects such as metallic effects or interference effects.

The size of the pigment used in the composition according to the present invention is generally between 10 nm and 200 $\mu$m, preferably between 20 nm and 80 $\mu$m and more preferentially between 30 nm and 50 $\mu$m.

The pigments may be dispersed in the composition by means of a dispersant.

The dispersant serves to protect the dispersed particles against agglomeration or flocculation thereof. This dispersant may be a surfactant, an oligomer, a polymer or a mixture of several thereof, bearing one or more functionalities with strong affinity for the surface of the particles to be dispersed. In particular, they may become physically or chemically attached to the surface of the pigments. These dispersants also contain at least one functional group that is compatible with or soluble in the continuous medium. In particular, esters of 12-hydroxystearic acid in particular and of C8 to C20 fatty acid and of polyols such as glycerol or diglycerol are used, such as poly(12-hydroxystearic acid) stearate with a molecular weight of approximately 750 g/mol, such as the product sold under the name Solsperse 21 000 by the company Avecia, polyglyceryl-2 dipolyhydroxystearate (CTFA name) sold under the reference Dehymyls PGPH by the company Henkel, or else polyhydroxystearic acid such as the product sold under the reference Arlacel P100 by the company Uniqema, and mixtures thereof.

As other dispersants that may be used in the compositions of the invention, mention may be made of quaternary ammonium derivatives of polycondensed fatty acids, for instance Solsperse 17 000 sold by the company Avecia, and polydimethylsiloxane/oxypropylene mixtures such as those sold by the company Dow Corning under the references DC2-5185 and DC2-5225 C.

The pigments used in the composition may be surface-treated with an organic agent.

Thus, the pigments surface-treated beforehand that are useful in the context of the invention are pigments which have been completely or partially subjected to a surface treatment of chemical, electronic, electrochemical, mechanochemical or mechanical nature with an organic agent, such as those described notably in Cosmetics and Toiletries, February 1990, Vol. 105, pages 53-64, before being dispersed in the composition in accordance with the invention. These organic agents may be chosen, for example, from waxes, for example carnauba wax and beeswax; fatty acids, fatty alcohols and derivatives thereof, such as stearic acid, hydroxystearic acid, stearyl alcohol, hydroxystearyl alcohol and lauric acid and derivatives thereof; anionic surfactants; lecithins; sodium, potassium, magnesium, iron, titanium, zinc or

aluminium salts of fatty acids, for example aluminium stearate or laurate; metal alkoxides; polyethylene; (meth)acrylic polymers, for example polymethyl methacrylates; polymers and copolymers containing acrylate units; alkanolamines; silicone compounds, for example silicones, notably polydimethylsiloxanes; organofluorine compounds, for example perfluoroalkyl ethers; fluorosilicone compounds.

The surface-treated pigments that are useful in the composition may also have been treated with a mixture of these compounds and/or may have undergone several surface treatments.

The surface-treated pigments that are useful in the context of the present invention may be prepared according to surface-treatment techniques that are well known to those skilled in the art, or may be commercially available as is.

Preferably, the surface-treated pigments are coated with an organic layer.

The organic agent with which the pigments are treated may be deposited on the pigments by evaporation of solvent, chemical reaction between the molecules of the surface agent or creation of a covalent bond between the surface agent and the pigments.

The surface treatment may thus be performed, for example, by chemical reaction of a surface agent with the surface of the pigments and creation of a covalent bond between the surface agent and the pigments or the fillers. This method is notably described in patent US 4 578 266.

An organic agent covalently bonded to the pigments will preferably be used.

The agent for the surface treatment may represent from 0.1% to 50% by weight relative to the total weight of the surface-treated pigment, preferably from 0.5% to 30% by weight and even more preferentially from 1% to 20% by weight relative to the total weight of the surface-treated pigment.

Preferably, the surface treatments of the pigments are chosen from the following treatments:

- a PEG-silicone treatment, for instance the AQ surface treatment sold by LCW;
- a methicone treatment, for instance the SI surface treatment sold by LCW;
- a dimethicone treatment, for instance the Covasil 3.05 surface treatment sold by LCW;
- a dimethicone/trimethyl siloxysilicate treatment, for instance the Covasil 4.05 surface treatment sold by LCW;
- a magnesium myristate treatment, for instance the MM surface treatment sold by LCW;
- an aluminium dimyristate treatment, for instance the MI surface treatment sold by Miyoshi;
- a perfluoropolymethyl isopropyl ether treatment, for instance the FHC surface treatment sold by LCW;
- an isostearyl sebacate treatment, for instance the HS surface treatment sold by Miyoshi;
- a perfluoroalkyl phosphate treatment, for instance the PF surface treatment sold by Daito;
- an acrylate/dimethicone copolymer and perfluoroalkyl phosphate treatment, for instance the FSA surface treatment sold by Daito;
- a polymethylhydrogenosiloxane/perfluoroalkyl phosphate treatment, for instance the FS01 surface treatment sold by Daito;
- an acrylate/dimethicone copolymer treatment, for instance the ASC surface treatment sold by Daito;
- an isopropyl titanium triisostearate treatment, for instance the ITT surface treatment sold by Daito;
- an acrylate copolymer treatment, for instance the APD surface treatment sold by Daito;
- a perfluoroalkyl phosphate/isopropyl titanium triisostearate treatment, for instance the PF + ITT surface treatment sold by Daito.

According to a particular embodiment of the invention, the dispersant is present with organic or mineral pigments in submicron-sized particulate form in composition B.

The term "submicron-sized" or "submicronic" refers to pigments having a particle size that has been micronized by a micronization method and having a mean particle size of less than a micrometre ($\mu$m), in particular between 0.1 and 0.9 $\mu$m, and preferably between 0.2 and 0.6 $\mu$m.

According to one embodiment, the dispersant and the pigment(s) are present in an amount (dispersant:pigment), according to a weight ratio, of between 1:4 and 4:1, particularly between 1.5:3.5 and 3.5:1 or better still between 1.75:3 and 3:1.

The dispersant(s) may therefore have a silicone backbone, such as silicone polyether and dispersants of amino silicone type. Among the suitable dispersants that may be mentioned are:

- aminosilicones, i.e. silicones comprising one or more amino groups such as those sold under the names and references: BYK LPX 21879 by BYK, GP-4, GP-6, GP-344, GP-851, GP-965, GP-967 and GP-988-1, sold by Genesee Polymers,
- silicone acrylates such as Tego® RC 902, Tego® RC 922, Tego® RC 1041, and Tego® RC 1043, sold by Evonik,
- polydimethylsiloxane (PDMS) silicones bearing carboxyl groups such as X-22162 and X-22370 by Shin-Etsu, epoxy silicones such as GP-29, GP-32, GP-502, GP-504, GP-514, GP-607, GP-682, and GP-695 by Genesee Polymers, or Tego® RC 1401, Tego® RC 1403, Tego® RC 1412 by Evonik.

According to a particular embodiment, the dispersant(s) are of amino silicone type and are cationic.
Preferably, the pigment(s) are chosen from mineral, mixed mineral-organic or organic pigments.
In one variant of the invention, the pigment(s) are organic pigments, preferentially organic pigments surface-treated with an organic agent chosen from silicone compounds. In another variant of the invention, the pigment(s) are mineral pigments.
The pigment(s) may be present in a total content ranging from 0.05% to 20% by weight, preferably from 0.1% to 15% by weight and better still from 0.5% to 10% by weight, relative to the total weight of the hair dye composition.

*Direct dye*

The hair dye composition used in the context of the process according to the invention may comprise one or more direct dyes.
The term "direct dye" means natural and/or synthetic dyes, other than oxidation dyes. These are dyes which will spread superficially over the fibre.
They may be ionic or nonionic, preferably cationic or nonionic.
Examples of suitable direct dyes that may be mentioned include azo direct dyes; (poly)methine dyes such as cyanines, hemicyanines and styryls; carbonyl dyes; azine dyes; nitro(hetero)aryl dyes; tri(hetero)arylmethane dyes; porphyrin dyes; phthalocyanine dyes and natural direct dyes, alone or in the form of mixtures.
The direct dyes are preferably cationic direct dyes. Mention may be made of the hydrazono cationic dyes of formulae (III) and (IV) and the azo cationic dyes (V) and (VI) below:

$Het^{+}$-N(Ra)-N=C(Rb)-Ar, Q- (III),

$Het^{+}$-C(Ra)=N-N(Rb)-Ar, Q (IV),

Het+-N=N-Ar, Q- (V),

$Ar^{+}$-N=N-Ar", Q- (VI),

in which formulae (III) to (VI):

- $Het^{+}$ represents a cationic heteroaryl radical, preferentially bearing an endocyclic cationic charge, such as imidazolium, indolium or pyridinium, which is optionally substituted, preferentially with at least one ($C_1$-$C_8$)alkyl group such as methyl;
- $Ar^{+}$ represents an aryl radical, such as phenyl or naphthyl, bearing an exocyclic cationic charge, preferentially ammonium, particularly tri($C_1$-$C_8$)alkylammonium, such as trimethylammonium;
- Ar represents an aryl group, notably phenyl, which is optionally substituted, preferentially with one or more electron-donating groups such as i) optionally substituted ($C_1$-$C_8$)alkyl, ii) optionally substituted ($C_1$-$C_8$)alkoxy, iii) (di)($C_1$-$C_8$)(alkyl)amino optionally substituted on the alkyl group(s) with a hydroxyl group, iv) aryl($C_1$-$C_8$)alkylamino, v) optionally substituted N-($C_1$-$C_8$)alkyl-N-aryl($C_1$-$C_8$)alkylamino or alternatively Ar represents a julolidine group;
- Ar" represents an optionally substituted (hetero)aryl group, such as phenyl or pyrazolyl, which are optionally substituted, preferentially with one or more ($C_1$-$C_8$)alkyl, hydroxyl, (di)($C_1$-$C_8$)(alkyl)amino, ($C_1$-$C_8$)alkoxy or phenyl groups;
- Ra and Rb, which may be identical or different, represent a hydrogen atom or a ($C_1$-$C_8$)alkyl group, which is optionally substituted, preferentially with a hydroxyl group;
  or else the substituent Ra with a substituent of Het+ and/or Rb with a substituent of Ar form, together with the atoms that bear them, a (hetero)cycloalkyl; in particular, Ra and Rb represent a hydrogen atom or a ($C_1$-$C_4$)alkyl group optionally substituted with a hydroxyl group;
- Q- represents an organic or mineral anionic counterion, such as a halide or an alkyl sulfate.

In particular, mention may be made of the azo and hydrazono direct dyes bearing an endocyclic cationic charge of formulae (III) to (VI) as defined previously, more particularly, the cationic direct dyes bearing an endocyclic cationic charge described in patent applications WO 95/15144, WO 95/01772 and EP 714 954, preferentially the following direct dyes:

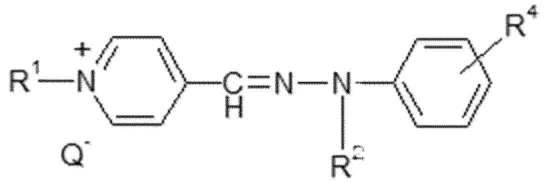

(VII),

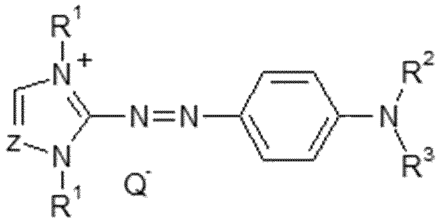

(VIII),

in which formulae (VII) and (VIII):

- $R^1$ represents a $(C_1-C_4)$alkyl group such as methyl;
- $R^2$ and $R^3$, which may be identical or different, represent a hydrogen atom or a $(C_1-C_4)$alkyl group, such as methyl; and
- $R^4$ represents a hydrogen atom or an electron-donating group such as optionally substituted $(C_1-C_8)$alkyl, optionally substituted $(C_1-C_8)$alkoxy, or (di)$(C_1-C_8)$(alkyl)amino optionally substituted on the alkyl group(s) with a hydroxyl group; in particular, $R^4$ is a hydrogen atom;
- Z represents a CH group or a nitrogen atom, preferentially CH;
- Q- is an anionic counterion as defined previously, in particular a halide, such as chloride, or an alkyl sulfate, such as methyl sulfate or mesyl.

In particular, the dyes of formulae (V) and (VI) are chosen from Basic Red 51, Basic Yellow 87 and Basic Orange 31 or derivatives thereof with Q' being an anionic counterion as defined previously, particularly a halide such as chloride, or an alkyl sulfate such as methyl sulfate or mesyl.

The direct dyes may be chosen from anionic direct dyes. The anionic direct dyes of the invention are dyes commonly referred to as "acid" direct dyes owing to their affinity for alkaline substances. The term "anionic direct dye" means any direct dye including in its structure at least one $CO_2R$ or $SO_3R$ substituent with R denoting a hydrogen atom or a cation originating from a metal or an amine, or an ammonium ion. The anionic dyes may be chosen from direct nitro acid dyes, azo acid dyes, azine acid dyes, triarylmethane acid dyes, indoamine acid dyes, anthraquinone acid dyes, indigoid dyes and natural acid dyes.

As acid dyes that are useful for the invention, mention may be made of the dyes of formulae (IX), (IX'), (X), (X'), (XI), (XI'), (XII), (XII'), (XIII), (XIV), (XV) and (XVI) below:

a) the diaryl anionic azo dyes of formula (IX) or (IX'):

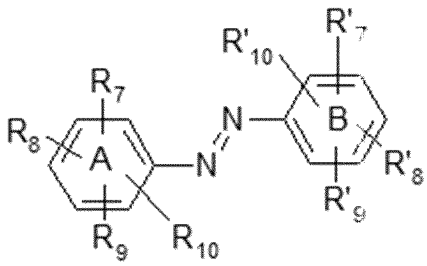

(IX),

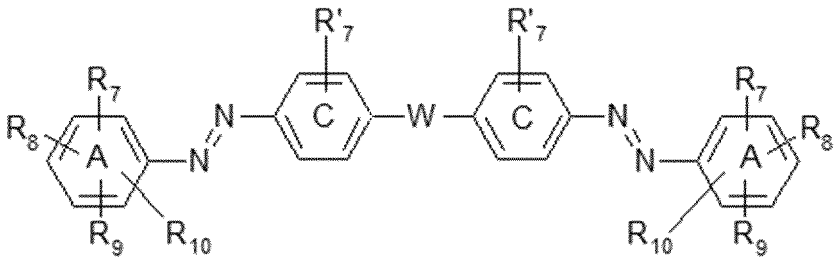

(IX'),

in which formulae (IX) and (IX'):

- $R_7$, $R_8$, $R_9$, $R_{10}$, $R'_7$, $R'_8$, $R'_9$ and $R'_{10}$, which may be identical or different, represent a hydrogen atom or a group chosen from:
- alkyl;
- alkoxy, alkylthio;
- hydroxyl, mercapto;
- nitro, nitroso;
- R°-C(X)-X'-, R°-X'-C(X)-, R°-X'-C(X)-X''- with R° representing a hydrogen atom or an alkyl or aryl group; X, X' and X'', which may be identical or different, representing an oxygen or sulfur atom, or NR with R representing a hydrogen atom or an alkyl group;
- $(O)_2S(O^-)$-, $M^+$ with $M^+$ representing a hydrogen atom or a cationic counterion;
- $(O)CO^-$-, $M^+$ with $M^+$ as defined previously;
- R"-$S(O)_2$-, with R" representing a hydrogen atom or an alkyl group, an aryl, (di)(alkyl)amino or aryl(alkyl)amino group; preferentially a phenylamino or phenyl group;
- R'''-$S(O)_2$-X'- with R''' representing an optionally substituted alkyl or aryl group, X' as defined previously;
- (di)(alkyl)amino;
- aryl(alkyl)amino optionally substituted with one or more groups chosen from i) nitro; ii) nitroso; iii) $(O)_2S(O^-)$-, $M^+$ and iv) alkoxy with $M^+$ as defined previously;
- optionally substituted heteroaryl; preferentially a benzothiazolyl group;
- cycloalkyl, notably cyclohexyl;
- Ar-N=N- with Ar representing an optionally substituted aryl group; preferentially a phenyl optionally substituted with one or more alkyl, $(O)_2S(O^-)$-, $M^+$ or phenylamino groups;
- or alternatively two contiguous groups $R_7$ with $R_8$ or $R_8$ with $R_9$ or $R_9$ with $R_{10}$ together form a fused benzo group A'; and $R'_7$ with $R'_8$ or $R'_8$ with $R'_9$ or $R'_9$ with $R'_{10}$ together form a fused benzo group B'; with A' and B' optionally substituted with one or more groups chosen from i) nitro; ii) nitroso; iii) $(O)_2S(O^-)$-, $M^+$; iv) hydroxyl; v) mercapto; vi) (di)(alkyl)amino; vii) R°-C(X)-X'-; viii) R°-X'-C(X)-; ix) R°-X'-C(X)-X''-; x) Ar-N=N- and xi) optionally substituted aryl(alkyl)amino; with $M^+$, R°, X, X', X" and Ar as defined previously;
- W represents a sigma bond $\sigma$, an oxygen or sulfur atom, or a divalent radical i) -NR- with R as defined previously, or ii) methylene -C(Ra)(Rb)- with Ra and Rb, which may be identical or different, representing a hydrogen atom or an aryl group, or alternatively Ra and Rb form, together with the carbon atom that bears them, a spiro cycloalkyl; preferentially, W represents a sulfur atom or Ra and Rb together form a cyclohexyl;
  it being understood that formulae (IX) and (IX') comprise at least one sulfonate radical $(O)_2S(O^-)$-, $M^+$ or one carboxylate radical $(O)CO^-$-, $M^+$ on one of the rings A, A', B, B' or C; preferentially sodium sulfonate.

As examples of dyes of formula (IX), mention may be made of: Acid Red 1, Acid Red 4, Acid Red 13, Acid Red 14, Acid Red 18, Acid Red 27, Acid Red 28, Acid Red 32, Acid Red 33, Acid Red 35, Acid Red 37, Acid Red 40, Acid Red 41, Acid Red 42, Acid Red 44, Pigment Red 57, Acid Red 68, Acid Red 73, Acid Red 135, Acid Red 138, Acid Red 184, Food Red 1, Food Red 13, Acid Orange 6, Acid Orange 7, Acid Orange 10, Acid Orange 19, Acid Orange 20, Acid Orange 24, Yellow 6, Acid Yellow 9, Acid Yellow 36, Acid Yellow 199, Food Yellow 3, Acid Violet 7, Acid Violet 14, Acid Blue 113, Acid Blue 117, Acid Black 1, Acid Brown 4, Acid Brown 20, Acid Black 26, Acid Black 52, Food Black 1, Food Black 2, Food Yellow 3 or Sunset Yellow;
and, as examples of dyes of formula (IX'), mention may be made of: Acid Red 111, Acid Red 134, Acid Yellow 38;

b) the pyrazolone anionic azo dyes of formulae (X) and (X'):

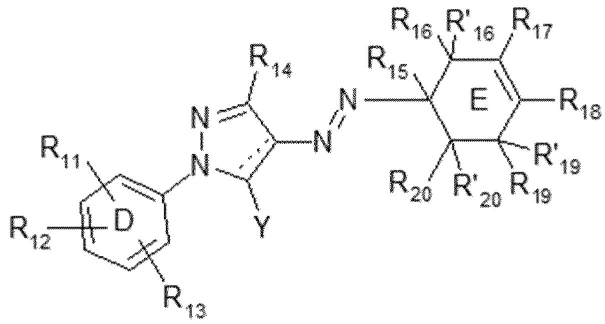

(X),

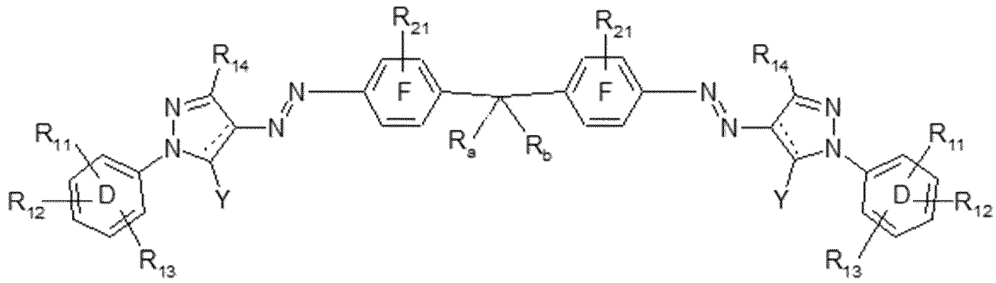

(X'),

in which formulae (X) and (X'):

- $R_{11}$, $R_{12}$ and $R_{13}$, which may be identical or different, represent a hydrogen or halogen atom, an alkyl group or $-(O)_2S(O^-)$, $M^+$ with $M^+$ as defined previously;
- $R_{14}$ represents a hydrogen atom, an alkyl group or a group $-C(O)O^-$, $M^+$ with $M^+$ as defined previously;
- $R_{15}$ represents a hydrogen atom;
- $R_{16}$ represents an oxo group, in which case $R'_{16}$ is absent, or alternatively $R_{15}$ with $R_{16}$ together form a double bond;
- $R_{17}$ and $R_{18}$, which may be identical or different, represent a hydrogen atom, or a group chosen from:
- $(O)_2S(O^-)-$, $M^+$ with $M^+$ as defined previously;
- $Ar-O-S(O)_2-$ with Ar representing an optionally substituted aryl group; preferentially a phenyl optionally substituted with one or more alkyl groups;
- $R_{19}$ and $R_{20}$ together form either a double bond, or a benzo group D', which is optionally substituted;
- $R'_{16}$, $R'_{19}$ and $R'_{20}$, which may be identical or different, represent a hydrogen atom or an alkyl or hydroxyl group;
- $R_{21}$ represents a hydrogen atom or an alkyl or alkoxy group;
- Ra and Rb, which may be identical or different, are as defined previously; preferentially, Ra represents a hydrogen atom and Rb represents an aryl group;
- Y represents either a hydroxyl group or an oxo group;
- represents a single bond when Y is an oxo group; and represents a double bond when Y represents a hydroxyl group;

  it being understood that formulae (X) and (X') comprise at least one sulfonate radical $(O)_2S(O^-)-$, $M^+$ or one carboxylate radical $-C(O)O^-$, $M^+$ on one of the rings D or E; preferentially sodium sulfonate.

As examples of dyes of formula (X), mention may be made of: Acid Red 195, Acid Yellow 23, Acid Yellow 27, Acid Yellow 76, and as examples of dyes of formula (X'), mention may be made of: Acid Yellow 17;

c) the anthraquinone dyes of formulae (XI) and (XI'):

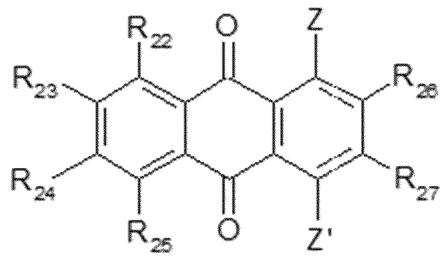

(XI),

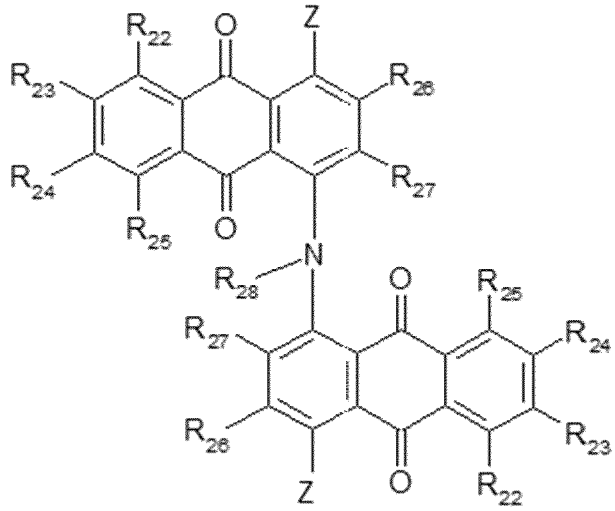

(XI'),

in which formulae (XI) and (XI'):

- $R_{22}$, $R_{23}$, $R_{24}$, $R_{25}$, $R_{26}$ and $R_{27}$, which may be identical or different, represent a hydrogen or halogen atom, or a group chosen from:
- alkyl;
- hydroxyl, mercapto;
- alkoxy, alkylthio;
- optionally substituted aryloxy or arylthio, preferentially substituted with one or more groups chosen from alkyl and $(O)_2S(O^-)$-, $M^+$ with $M^+$ as defined previously;
- aryl(alkyl)amino optionally substituted with one or more groups chosen from alkyl and $(O)_2S(O^-)$-, $M^+$ with $M^+$ as defined previously;
- (di)(alkyl)amino;
- (di)(hydroxyalkyl)amino;
- $(O)_2S(O^-)$-, $M^+$ with $M^+$ as defined previously;
- Z' represents a hydrogen atom or a group $NR_{28}R_{29}$ with $R_{28}$ and $R_{29}$, which may be identical or different, representing a hydrogen atom or a group chosen from:
- alkyl;
- polyhydroxyalkyl such as hydroxyethyl;
- aryl optionally substituted with one or more groups, particularly i) alkyl such as methyl, n-dodecyl, n-butyl; ii) $(O)_2S(O^-)$-, $M^+$ with $M^+$ as defined previously; iii) R°-C(X)-X'-, R°-X'-C(X)-, R°-X'-C(X)-X''- with R°, X, X' and X'' as defined previously, preferentially R° represents an alkyl group;
- cycloalkyl, notably cyclohexyl;
- Z represents a group chosen from hydroxyl and $NR'_{28}R'_{29}$ with $R'_{28}$ and $R'_{29}$, which may be identical or different, representing the same atoms or groups as $R_{28}$ and $R_{29}$ as defined previously;

  it being understood that formulae (XI) and (XI') comprise at least one sulfonate radical $(O)_2S(O^-)$-, $M^+$ or one carboxylate radical $C(O)O^-$, $M^+$; preferentially sodium sulfonate.

As examples of dyes of formula (XI), mention may be made of: Acid Blue 25, Acid Blue 43, Acid Blue 62, Acid Blue 78,

Acid Blue 129, Acid Blue 138, Acid Blue 140, Acid Blue 251, Acid Green 25, Acid Green 41, Acid Violet 42, Acid Violet 43, Mordant Red 3; EXT Violet No. 2; and, as an example of a dye of formula (XI'), mention may be made of: Acid Black 48;

d) the nitro dyes of formulae (XII) and (XII'):

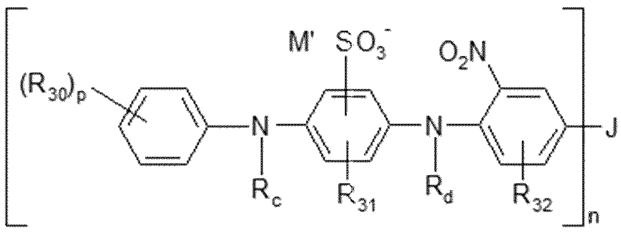

(XII),

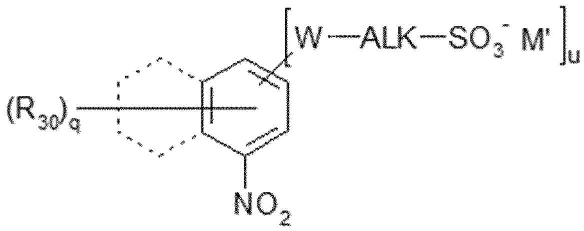

(XII'),

in which formulae (XII) and (XII'):

- $R_{30}$, $R_{31}$ and $R_{32}$, which may be identical or different, represent a hydrogen or halogen atom, or a group chosen from:
- alkyl;
- alkoxy optionally substituted with one or more hydroxyl groups, alkylthio optionally substituted with one or more hydroxyl groups;
- hydroxyl, mercapto;
- nitro, nitroso;
- polyhaloalkyl;
- R°-C(X)-X'-, R°-X'-C(X)-, R°-X'-C(X)-X''- with R°, X, X' and X'' as defined previously;
- $(O)_2S(O^-)$-, $M^+$ with $M^+$ as defined previously;
- $(O)CO^-$-, $M^+$ with $M^+$ as defined previously;
- (di)(alkyl)amino;
- (di)(hydroxyalkyl)amino;
- heterocycloalkyl such as piperidino, piperazino or morpholino; in particular, $R_{30}$, $R_{31}$ and $R_{32}$ represent a hydrogen atom;
- Rc and Rd, which may be identical or different, represent a hydrogen atom or an alkyl group;
- W is as defined previously; W particularly represents an -NH- group;
- ALK represents a linear or branched divalent $C_1$-$C_6$ alkylene group; in particular, ALK represents a $-CH_2-CH_2-$ group;
- n is 1 or 2;
- p represents an integer inclusively between 1 and 5;
- q represents an integer inclusively between 1 and 4;
- u is 0 or 1;
- when n is 1, J represents a nitro or nitroso group; particularly nitro;
- when n is 2, J represents an oxygen or sulfur atom, or a divalent radical $-S(O)_m-$ with m representing an integer 1 or 2; preferentially, J represents an - $SO_2$- radical;
- M' represents a hydrogen atom or a cationic counterion;

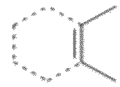

,

which may be present or absent, represents a benzo group optionally substituted with one or more groups $R_{30}$ as defined previously;

it being understood that formulae (XII) and (XII') comprise at least one sulfonate radical $(O)_2S(O^-)$-, $M^+$ or one carboxylate radical -$C(O)O^-$, $M^+$; preferentially sodium sulfonate.

As examples of dyes of formula (XII), mention may be made of: Acid Brown 13 and Acid Orange 3; as examples of dyes of formula (XII'), mention may be made of: Acid Yellow 1, the sodium salt of 2,4-dinitro-1-naphthol-7-sulfonic acid, 2-piperidino-5-nitrobenzenesulfonic acid, 2-(4'-N,N-(2"-hydroxyethyl)amino-2'-nitro)anilineethanesulfonic acid, 4-β-hydroxyethylamino-3-nitrobenzenesulfonic acid; EXT D&C Yellow 7;

e) the triarylmethane dyes of formula (XIII):

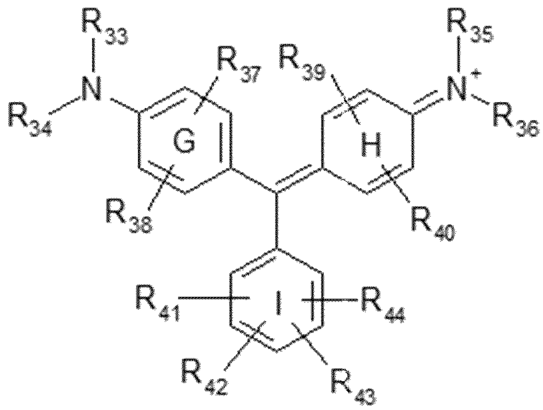

(XIII),

in which formula (XIII):

- $R_{33}$, $R_{34}$, $R_{35}$ and $R_{36}$, which may be identical or different, represent a hydrogen atom or a group chosen from alkyl, optionally substituted aryl and optionally substituted arylalkyl; particularly an alkyl and benzyl group optionally substituted with a group $(O)_mS(O^-)$-, $M^+$ with $M^+$ and m as defined previously;
- $R_{37}$, $R_{38}$, $R_{39}$, $R_{40}$, $R_{41}$, $R_{42}$, $R_{43}$ and $R_{44}$, which may be identical or different, represent a hydrogen atom or a group chosen from:
- alkyl;
- alkoxy, alkylthio;
- (di)(alkyl)amino;
- hydroxyl, mercapto;
- nitro, nitroso;
- R°-C(X)-X'-, R°-X'-C(X)-, R°-X'-C(X)-X''- with R° representing a hydrogen atom or an alkyl or aryl group; X, X' and X'', which may be identical or different, representing an oxygen or sulfur atom, or NR with R representing a hydrogen atom or an alkyl group;
- $(O)_2S(O^-)$-, $M^+$ with $M^+$ representing a hydrogen atom or a cationic counterion;
- $(O)CO^-$-, $M^+$ with $M^+$ as defined previously;
- or alternatively two contiguous groups $R_{41}$ with $R_{42}$ or $R_{42}$ with $R_{43}$ or $R_{43}$ with $R_{44}$ together form a fused benzo group: I'; with I' optionally substituted with one or more groups chosen from i) nitro; ii) nitroso; iii) $(O)_2S(O^-)$-, $M^+$; iv) hydroxyl; v) mercapto; vi) (di)(alkyl)amino; vii) R°-C(X)-X'-; viii) R°-X'-C(X)- and ix) R°-X'-C(X)-X"-; with $M^+$, R°, X, X' and X" as defined previously;

in particular, $R_{37}$ to $R_{40}$ represent a hydrogen atom, and $R_{41}$ to $R_{44}$, which may be identical or different, represent a hydroxyl group or $(O)_2S(O^-)$-, $M^+$; and when $R_{43}$ with $R_{44}$ together form a benzo group, it is preferentially substituted with an $(O)_2S(O^-)$- group;

it being understood that at least one of the rings G, H, I or I' comprises at least one sulfonate radical $(O)_2S(O^-)$-

or one carboxylate radical -C(O)O$^-$; preferentially sulfonate.

As examples of dyes of formula (XIII), mention may be made of: Acid Blue 1; Acid Blue 3; Acid Blue 7, Acid Blue 9; Acid Violet 49; Acid Green 3; Acid Green 5 and Acid Green 50.

f) the xanthene-based dyes of formula (XIV):

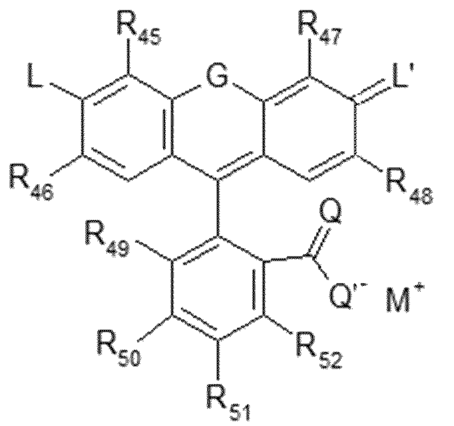

(XIV),

in which formula (XIV):

- $R_{45}$, $R_{46}$, $R_{47}$ and $R_{48}$, which may be identical or different, represent a hydrogen or halogen atom;
- $R_{49}$, $R_{50}$, $R_{51}$ and $R_{52}$, which may be identical or different, represent a hydrogen or halogen atom, or a group chosen from:
- alkyl;
- alkoxy, alkylthio;
- hydroxyl, mercapto;
- nitro, nitroso;
- $(O)_2S(O^-)$-, $M^+$ with $M^+$ representing a hydrogen atom or a cationic counterion;
- (O)CO$^-$-, $M^+$ with $M^+$ as defined previously;
  particularly, $R_{49}$, $R_{50}$, $R_{51}$ and $R_{52}$ represent a hydrogen or halogen atom;
- G represents an oxygen or sulfur atom or a group NRe with Re as defined previously; particularly G represents an oxygen atom;
- L represents an alkoxide O$^-$, $M^+$; a thioalkoxide S$^-$, $M^+$ or a group NRf, with Rf representing a hydrogen atom or an alkyl group, and $M^+$ as defined above; $M^+$ is particularly sodium or potassium;
- L' represents an oxygen or sulfur atom or an ammonium group: $N^+$RfRg, with Rf and Rg, which may be identical or different, representing a hydrogen atom or an optionally substituted alkyl or aryl group; L' particularly represents an oxygen atom or a phenylamino group optionally substituted with one or more alkyl or $(O)_mS(O^-)$-, $M^+$ groups with m and $M^+$ as defined previously;
- Q and Q', which may be identical or different, represent an oxygen or sulfur atom; particularly, Q and Q' represent an oxygen atom;
- $M^+$ is as defined previously.

As examples of dyes of formula (XIV), mention may be made of: Acid Yellow 73; Acid Red 51; Acid Red 52; Acid Red 87; Acid Red 92; Acid Red 95; Acid Violet 9;

g) the indole-based dyes of formula (XV):

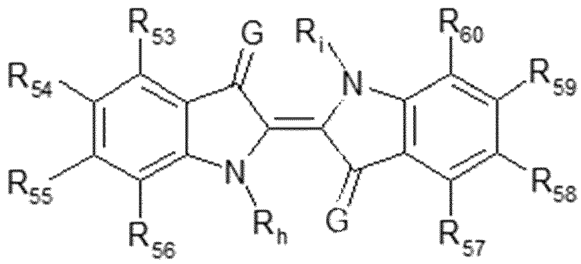

(XV),

in which formula (XV):

- $R_{53}$, $R_{54}$, $R_{55}$, $R_{56}$, $R_{57}$, $R_{58}$, $R_{59}$ and $R_{60}$, which may be identical or different, represent a hydrogen atom or a group chosen from:
- alkyl;
- alkoxy, alkylthio;
- hydroxyl, mercapto;
- nitro, nitroso;
- R°-C(X)-X'-, R°-X'-C(X)-, R°-X'-C(X)-X''- with R° representing a hydrogen atom or an alkyl or aryl group; X, X' and X'', which may be identical or different, representing an oxygen or sulfur atom, or NR with R representing a hydrogen atom or an alkyl group;
- $(O)_2S(O^-)$-, $M^+$ with $M^+$ representing a hydrogen atom or a cationic counterion;
- $(O)CO^-$-, $M^+$ with $M^+$ as defined previously;
- G represents an oxygen or sulfur atom or a group NRe with Re as defined previously; particularly, G represents an oxygen atom;
- Ri and Rh, which may be identical or different, represent a hydrogen atom or an alkyl group;
  it being understood that formula (XIII) comprises at least one sulfonate radical $(O)_2S(O^-)$-, $M^+$ or one carboxylate radical -C(O)O⁻, $M^+$; preferentially sodium sulfonate.

As examples of dyes of formula (XV), mention may be made of: Acid Blue 74;

h) the quinoline-based dyes of formula (XVI):

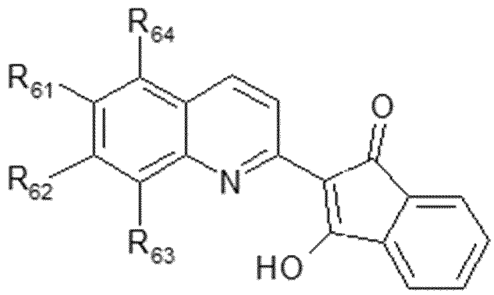

(XVI),

in which formula (XVI):

- $R_{61}$ represents a hydrogen or halogen atom or an alkyl group;
- $R_{62}$, $R_{63}$ and $R_{64}$, which may be identical or different, represent a hydrogen atom or a group $(O)_2S(O^-)$-, $M^+$ with $M^+$ representing a hydrogen atom or a cationic counterion;

  or alternatively $R_{61}$ with $R_{62}$, or $R_{61}$ with $R_{64}$, together form a benzo group optionally substituted with one or more groups $(O)_2S(O^-)$-, $M^+$ with $M^+$ representing a hydrogen atom or a cationic counterion;
  it being understood that formula (XVI) comprises at least one sulfonate radical $(O)_2S(O^-)$-, $M^+$, preferentially sodium sulfonate.

As examples of dyes of formula (XVI), mention may be made of: Acid Yellow 2, Acid Yellow 3 and Acid Yellow 5.

Among the natural direct dyes that may be used according to the invention, mention may be made of lawsone, juglone, alizarin, purpurin, carminic acid, kermesic acid, purpurogallin, protocatechaldehyde, indigo, isatin, curcumin, spinulosin, apigenidin and orceins. Use may also be made of extracts or decoctions containing these natural dyes and notably henna-based poultices or extracts.

The direct dye(s) may be present in a total content ranging from 0.001% to 10% by weight relative to the total weight of the composition, preferably from 0.005% to 5% by weight relative to the total weight of the hair dye composition.

The colouring agent(s) may be present in a total content ranging from 0.001% to 20% by weight and preferably from 0.005% to 15% by weight relative to the total weight of the hair dye composition.

Preferably, one or more pigments will be used.

The pH of the hair dye composition is generally greater than or equal to 5. Preferably, the hair dye composition has a pH ranging from 6.0 to 12.0, preferentially from 6 to 11, preferentially from 6.5 to 8.

The hair dye composition may comprise a cosmetically acceptable medium. The cosmetically acceptable medium may comprise a solvent chosen from water, organic solvents, and a mixture thereof.

Organic solvents

The hair dye composition used in the context of the process according to the invention may comprise one or more organic solvents.

Examples of organic solvents that may be mentioned include lower $C_1$-$C_4$ alkanols, such as ethanol and isopropanol; polyols and polyol ethers, for instance 2-butoxyethanol, propylene glycol, propylene glycol monomethyl ether and diethylene glycol monoethyl ether and monomethyl ether, and also aromatic alcohols, for instance benzyl alcohol or phenoxyethanol, and mixtures thereof.

Preferably, the hair dye composition comprises one or more organic solvents chosen from $C_1$-$C_4$ lower alkanols, more preferentially ethanol.

The organic solvents may be present in a total amount inclusively between 0.01% and 60% by weight approximately relative to the total weight of the hair dye composition, preferably between 0.05% and 50% by weight and more preferentially inclusively between 0.1% and 45% by weight relative to the total weight of the hair dye composition.

The hair dye composition used in the context of the process according to the invention is preferably aqueous. The water content may range from 20% to 99% by weight, preferably from 50% to 98% by weight and more preferentially from 60% to 95% by weight relative to the total weight of the hair dye composition.

Additives

The hair dye composition used in the context of the process according to the invention may contain any adjuvant or additive usually used.

Among the additives that may be contained in the hair dye composition, mention may be made of reducing agents, softeners, antifoams, moisturizers, UV-screening agents, peptizers, solubilizers, fragrances, thickeners, silicones, fatty substances, anionic, cationic, nonionic or amphoteric surfactants, proteins, vitamins, preserving agents, oils, waxes and mixtures thereof.

The hair treatment composition, notably the hair dye composition, may notably be in the form of a suspension, a dispersion, a gel, an emulsion, notably an oil-in-water (O/W) or water-in-oil (W/O) emulsion, or a multiple emulsion (W/O/W or polyol/O/W or O/W/O), in the form of a cream, a mousse, a stick, a dispersion of vesicles, notably of ionic or nonionic lipids, or a two-phase or multi-phase lotion.

A person skilled in the art may select the appropriate presentation form, and also the method for preparing it, on the basis of his general knowledge, taking into account firstly the nature of the constituents used, notably their solubility in the support, and secondly the intended application of the composition.

**Makeup-removing composition**

As previously indicated, the makeup removal process, notably for removing colour from hair keratin fibres, in particular from the hair, comprises the application of at least one composition for removing makeup from said hair keratin fibres, which have been treated beforehand with at least one composition, in particular a hair dye composition, as defined above, said makeup-removing composition, in particular for removing colour, comprising at least one surfactant, preferably an anionic or nonionic surfactant, and at least one acidifying agent, said composition having a pH of less than or equal to 5.5.

**Surfactant**

As indicated above, the colour-removing composition used in the context of the process according to the invention comprises at least one surfactant.
Preferably, the surfactant is an anionic or nonionic surfactant.
The term "anionic surfactant" means a surfactant including, as ionic or ionizable groups, only anionic groups.
In the present description, a species is termed as being "anionic" when it bears at least one permanent negative charge or when it can be ionized to a negatively charged species, under the conditions of use of the composition of the invention (for example the medium or the pH) and not comprising any cationic charge.
The anionic surfactants may be chosen from sulfate, sulfonate and/or carboxylic (or carboxylate) surfactants. Needless to say, a mixture of these surfactants may be used.
It is understood in the present description that:

- the carboxylate anionic surfactants comprise at least one carboxylic or carboxylate function (-COOH or $-COO^-$) and may optionally also comprise one or more sulfate and/or sulfonate functions;
- the sulfonate anionic surfactants comprise at least one sulfonate function ($-SO_3H$ or $-SO_3^-$) and may optionally also comprise one or more sulfate functions, but do not comprise any carboxylate functions; and
- the sulfate anionic surfactants comprise at least one sulfate function but do not comprise any carboxylate or sulfonate functions.

The carboxylate anionic surfactants that may be used thus include at least one carboxylic or carboxylate function (-COOH or $-COO^-$).
They may be chosen from the following compounds: fatty acids, acylglycinates, acyllactylates, acylsarcosinates, acylglutamates; alkyl-D-galactosideuronic acids, alkyl ether carboxylic acids, alkyl($C_6$-$C_{30}$ aryl) ether carboxylic acids, alkylamido ether carboxylic acids; and also the salts of these compounds; and mixtures thereof;

the alkyl and/or acyl groups of these compounds including from 6 to 30 carbon atoms, notably from 12 to 28, even better still from 14 to 24 or even from 16 to 22 carbon atoms; the aryl group preferably denoting a phenyl or benzyl group;
these compounds possibly being polyoxyalkylenated, notably polyoxyethylenated, and then preferably including from 1 to 50 ethylene oxide units and better still from 2 to 10 ethylene oxide units.

Use may also be made of $C_6$-$C_{24}$ alkyl monoesters of polyglycoside-polycarboxylic acids such as $C_6$-$C_{24}$ alkyl polyglycoside-citrates, $C_6$-$C_{24}$ alkyl polyglycoside-tartrates and $C_6$-$C_{24}$ alkyl polyglycoside-sulfosuccinates, and salts thereof.
Preferentially, the carboxylate anionic surfactants are chosen, alone or as a mixture, from:

- fatty acids;
- acyl glutamates, notably $C_6$-$C_{24}$ or even $C_{12}$-$C_{20}$ acyl glutamates, such as stearoyl glutamates, and in particular disodium stearoyl glutamate;
- acyl sarcosinates, notably $C_6$-$C_{24}$ or even $C_{12}$-$C_{20}$ acyl sarcosinates, such as palmitoyl sarcosinates, and in particular sodium palmitoyl sarcosinate;
- acyl lactylates, notably $C_{12}$-$C_{28}$ or even $C_{14}$-$C_{24}$ acyl lactylates, such as behenoyl lactylates, and in particular sodium behenoyl lactylate;
- $C_6$-$C_{24}$ and notably $C_{12}$-$C_{20}$ acyl glycinates;
- ($C_6$-$C_{24}$)alkyl ether carboxylates, and notably ($C_{12}$-$C_{20}$)alkyl ether carboxylates;
- polyoxyalkylenated ($C_6$-$C_{24}$)alkyl(amido) ether carboxylic acids, in particular those including from 2 to 50 ethylene oxide groups;

in particular in the form of alkali metal or alkaline-earth metal, ammonium or amino alcohol salts.

Among the above carboxylic surfactants, mention may be made most particularly of surfactants of fatty acid type, notably of $C_6$-$C_{30}$. These surfactants are preferably chosen from the compounds of formula (a) below: R-C(O)-OX (a)

with
- X denoting a hydrogen atom, an ammonium ion, an ion derived from an alkali metal or an alkaline-earth metal or an ion derived from an organic amine, preferably a hydrogen atom, and

- R denoting a linear or branched, saturated or unsaturated alkyl group of 5 to 29 carbon atoms.

Preferably, R denotes a linear or branched, saturated or unsaturated alkyl group of 7 to 23 carbon atoms, preferably of 11 to 21 carbon atoms.
Among the fatty acids, mention may be made of lauric acid, palmitic acid, myristic acid, stearic acid, oleic acid and behenic acid.
The fatty acids are advantageously chosen from palmitic acid, myristic acid, stearic acid, and mixtures thereof.
Among the above carboxylic surfactants, mention may be made most particularly of surfactants of sarcosinate type, notably chosen from ($C_6$-$C_{30}$)acyl sarcosinates of formula (I) below:

$$R\text{-}C(O)\text{-}N(CH_3)\text{-}CH_2\text{-}C(O)\text{-}OX \qquad (I)$$

with

- X denoting a hydrogen atom, an ammonium ion, an ion derived from an alkali metal or an alkaline-earth metal or an ion derived from an organic amine, preferably a hydrogen atom, and
- R denoting a linear or branched alkyl group of 5 to 29 carbon atoms.

Preferably, R denotes a linear or branched alkyl group of 8 to 24 carbon atoms, preferably of 12 to 20 carbon atoms.
Among the ($C_6$-$C_{30}$)acyl sarcosinates of formula (I) that may be used in the present composition, mention may be made of palmitoyl sarcosinates, stearoyl sarcosinates, myristoyl sarcosinates, lauroyl sarcosinates and cocoyl sarcosinates, in acid form or in salified form.
The anionic surfactant(s) of sarcosinate type are advantageously chosen from sodium lauroyl sarcosinate, stearoylsarcosine, myristoylsarcosine, and mixtures thereof, preferably from stearoylsarcosine, myristoylsarcosine, and mixtures thereof.
Among the above carboxylic surfactants, mention may also be made of polyoxyalkylenated alkyl(amido) ether carboxylic acids and salts thereof, in particular those including from 2 to 50 alkylene oxide and in particular ethylene oxide groups, such as the compounds sold by the company Kao under the Akypo names.
The polyoxyalkylenated alkyl(amido) ether carboxylic acids that may be used are preferably chosen from those of formula (II):

$$R1\text{-}(OC_2H_4)_n\text{-}OCH_2COOA \qquad (II)$$

in which:

- R1 represents a linear or branched $C_6$-$C_{24}$ alkyl or alkenyl radical, a ($C_8$-$C_9$)alkylphenyl radical, a radical $R_2$CONH-$CH_2$-$CH_2$- with R2 denoting a linear or branched $C_9$-$C_{21}$ alkyl or alkenyl radical;
  preferably, R1 is a $C_8$-$C_{20}$ and preferably $C_8$-$C_{18}$ alkyl radical, and aryl preferably denotes phenyl,
- n is an integer or decimal number (mean value) ranging from 2 to 24 and preferably from 2 to 10,
- A denotes H, ammonium, Na, K, Li, Mg or a monoethanolamine or triethanolamine residue.

Use may also be made of mixtures of compounds of formula (II), in particular mixtures of compounds bearing different groups R1.
The polyoxyalkylenated alkyl(amido) ether carboxylic acids that are particularly preferred are those of formula (II) in which:

- R1 denotes a $C_{12}$-$C_{14}$ alkyl, cocoyl, oleyl, nonylphenyl or octylphenyl radical,
- A denotes a hydrogen or sodium atom, and
- n ranges from 2 to 20, preferably from 2 to 10.

Even more preferentially, use is made of the compounds of formula (II) in which R1 denotes a $C_{12}$ alkyl radical, A denotes a hydrogen or sodium atom and n ranges from 2 to 10.
The sulfonate anionic surfactants that may be used include at least one sulfonate function ($-SO_3H$ or $-SO_3^-$).
They may be chosen from the following compounds: alkyl sulfonates, alkylamide sulfonates, alkylaryl sulfonates, $\alpha$-olefin sulfonates, paraffin sulfonates, alkyl sulfosuccinates, alkyl ether sulfosuccinates, alkylamide sulfosuccinates, alkyl sulfoacetates, N-acyl taurates, acyl isethionates; alkyl sulfolaurates; and also the salts of these compounds;

the alkyl groups of these compounds including from 6 to 30 carbon atoms, notably from 12 to 28, even better still from 14 to 24 or even from 16 to 22 carbon atoms; the aryl group preferably denoting a phenyl or benzyl group; these compounds possibly being polyoxyalkylenated, notably polyoxyethylenated, and then preferably including from 1 to 50 ethylene oxide units and better still from 2 to 10 ethylene oxide units.

Preferentially, the sulfonate anionic surfactants are chosen, alone or as a mixture, from:

- $C_6$-$C_{24}$ and notably $C_{12}$-$C_{20}$ alkyl sulfosuccinates, notably lauryl sulfosuccinates;
- $C_6$-$C_{24}$ and notably $C_{12}$-$C_{20}$ alkyl ether sulfosuccinates;
- $C_6$-$C_{24}$ and notably $C_{12}$-$C_{20}$ N-acyl taurates;
- ($C_6$-$C_{24}$)acyl isethionates, preferably ($C_{12}$-$C_{18}$)acyl isethionates;

in particular in the form of alkali metal or alkaline-earth metal, ammonium or amino alcohol salts.

Preferably, the anionic surfactant(s) of sulfonate type are chosen from N-acyltaurates, and notably N-acyl N-methyltaurates, acylisethionates, and also salts thereof and mixtures thereof.

More preferentially, the anionic surfactant(s) of sulfonate type are chosen from acylisethionates, and also salts thereof and mixtures thereof.

The sulfate anionic surfactants that may be used include at least one sulfate function ($-OSO_3H$ or $-OSO_3^-$).

They may be chosen from the following compounds: alkyl sulfates, alkyl ether sulfates, alkylamido ether sulfates, alkylaryl polyether sulfates, monoglyceride sulfates; and the salts of these compounds;

the alkyl groups of these compounds including from 6 to 30 carbon atoms, notably from 12 to 28, even better still from 14 to 24 or even from 16 to 22 carbon atoms; the aryl group preferably denoting a phenyl or benzyl group; these compounds possibly being (poly)oxyalkylenated, notably (poly)oxyethylenated, and then preferably including from 1 to 50 ethylene oxide units and better still from 1 to 10 ethylene oxide units.

Preferentially, the sulfate anionic surfactants are chosen, alone or as a mixture, from:

- alkyl sulfates, notably $C_6$-$C_{24}$ or even $C_{12}$-$C_{20}$ alkyl sulfates;
- alkyl ether sulfates, notably $C_6$-$C_{24}$ or even $C_{12}$-$C_{20}$ alkyl ether sulfates, preferably comprising from 1 to 20 ethylene oxide units;

in particular in the form of alkali metal or alkaline-earth metal, ammonium or amino alcohol salts.

When the anionic surfactant is in salt form, said salt may be chosen from alkali metal salts, such as the sodium or potassium salt, ammonium salts, amine salts and in particular amino alcohol salts, and alkaline-earth metal salts, such as the magnesium salt.

Examples of amino alcohol salts that may be mentioned include monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxymethyl)aminomethane salts.

Alkali metal or alkaline-earth metal salts and in particular sodium or magnesium salts are preferably used.

Preferentially, the anionic surfactant(s) are chosen from:

- $C_6$-$C_{30}$ and notably $C_8$-$C_{24}$ fatty acids;
- $C_6$-$C_{24}$ and notably $C_{12}$-$C_{20}$ alkyl sulfates;
- $C_6$-$C_{24}$ and notably $C_{12}$-$C_{20}$ alkyl ether sulfates; preferably comprising from 1 to 20 ethylene oxide units;
- $C_6$-$C_{24}$ and notably $C_{12}$-$C_{20}$ alkyl sulfosuccinates; notably lauryl sulfosuccinates;
- $C_6$-$C_{24}$ and notably $C_{12}$-$C_{20}$ alkyl ether sulfosuccinates;
- $C_6$-$C_{24}$ and notably $C_{12}$-$C_{20}$ N-acyltaurates;
- ($C_6$-$C_{24}$)acylisethionates, preferably ($C_{12}$-$C_{18}$)acylisethionates;
- $C_6$-$C_{24}$ and notably $C_{12}$-$C_{20}$ acylsarcosinates; notably palmitoylsarcosinates, stearoylsarcosinates and myristoylsarcosinates;
- ($C_6$-$C_{24}$)alkyl ether carboxylates, preferably ($C_{12}$-$C_{20}$)alkyl ether carboxylates;
- polyoxyalkylenated ($C_6$-$C_{24}$)alkyl(amido) ether carboxylic acids and salts thereof, in particular those including from 2 to 50 alkylene oxide and in particular ethylene oxide groups;
- $C_6$-$C_{24}$ and notably $C_{12}$-$C_{20}$ acylglutamates;
- $C_6$-$C_{24}$ and notably $C_{12}$-$C_{20}$ acyl glycinates;
- and also salts thereof, in particular the alkali metal or alkaline-earth metal or zinc, ammonium or amino alcohol salts thereof;

- and mixtures thereof.

Advantageously, the anionic surfactant(s) are chosen from sulfate anionic surfactants, sulfonate anionic surfactants and mixtures thereof.

Nonionic surfactant

The nonionic surfactants that may be used may be chosen from alcohols, α-diols and (C1-20)alkylphenols, these compounds being polyethoxylated and/or polypropoxylated and/or polyglycerolated, the number of ethylene oxide and/or propylene oxide groups possibly ranging from 1 to 100, and the number of glycerol groups possibly ranging from 2 to 30; and/or these compounds comprising at least one fatty chain including from 8 to 30 carbon atoms and notably from 16 to 30 carbon atoms.

Mention may also be made of condensates of ethylene oxide and of propylene oxide with fatty alcohols; polyethoxylated fatty amides preferably containing from 2 to 30 ethylene oxide units, polyglycerolated fatty amides including on average from 1 to 5, and in particular from 1.5 to 4, glycerol groups; C8-C30 fatty acid alkanolamides, such as C8-C30 fatty acid monoalkanolamides, in particular C8-C30 fatty acid monoethanolamides or monoisopropanolamides; ethoxylated fatty acid esters of sorbitan preferably containing from 2 to 40 ethylene oxide units, fatty acid esters of sucrose, polyoxyalkylenated and preferably polyoxyethylenated fatty acid esters containing from 2 to 150 mol of ethylene oxide, including oxyethylenated plant oils, N-(C6-24 alkyl)glucamine derivatives, amine oxides such as (C10-14 alkyl)amine oxides or N-(C 10-14 acyl)aminopropylmorpholine oxides.

Mention may also be made of nonionic surfactants of alkylpolyglycoside type, notably represented by the following general formula: $R_1O-(R_2O)_t-(G)_v$
in which:

- $R_1$ represents a linear or branched alkyl or alkenyl radical including 6 to 24 carbon atoms and notably 8 to 18 carbon atoms, or an alkylphenyl radical of which the linear or branched alkyl radical includes 6 to 24 carbon atoms and notably 8 to 18 carbon atoms;
- $R_2$ represents an alkylene radical including 2 to 4 carbon atoms;
- G represents a sugar unit including 5 to 6 carbon atoms;
- t denotes a value ranging from 0 to 10 and preferably from 0 to 4;
- v denotes a value ranging from 1 to 15 and preferably from 1 to 4.

Preferably, the alkylpolyglycoside surfactants are compounds of the formula described above in which:

- $R_1$ denotes a linear or branched, saturated or unsaturated alkyl radical including from 8 to 18 carbon atoms,
- $R_2$ represents an alkylene radical including 2 to 4 carbon atoms,
- t denotes a value ranging from 0 to 3 and preferably equal to 0,
- G denotes glucose, fructose or galactose, preferably glucose,
- it being possible for the degree of polymerization, i.e. the value of v, to range from 1 to 15 and preferably from 1 to 4; the mean degree of polymerization more particularly being between 1 and 2.

The glucoside bonds between the sugar units are generally of 1-6 or 1-4 type and preferably of 1-4 type. Preferably, the alkylpolyglycoside surfactant is an alkylpolyglucoside surfactant. C8/C16 alkyl polyglycosides 1,4, and notably decyl glucosides and caprylyl/capryl glucosides, are most particularly preferred.

Among the commercial products, mention may be made of the products sold by the company Cognis under the names Plantaren® (600 CS/U, 1200 and 2000) or Plantacare® (818, 1200 and 2000); the products sold by the company SEPPIC under the names Oramix CG 110 and Oramix® NS 10; the products sold by the company BASF under the name Lutensol GD 70, or the products sold by the company Chem Y under the name AG10 LK.

Preferably, use is made of C8/C16 alkyl polyglycosides of 1-4 type, notably as an aqueous 53% solution, such as those sold by Cognis under the reference Plantacare® 818 UP.

Preferentially, the nonionic surfactants are chosen from (C6-24 alkyl)polyglycosides, and more particularly (C8-18 alkyl)polyglycosides, ethoxylated C8-C30 fatty acid esters of sorbitan, polyethoxylated C8-C30 fatty alcohols, polyoxyethylenated C8-C30 fatty acid esters, these compounds preferably containing from 2 to 150 mol of ethylene oxide, and C8-C30 fatty acid alkanolamides such as C8-C30 fatty acid monoalkanolamides, in particular C8-C30 fatty acid monoethanolamides or monoisopropanolamides, and mixtures thereof.

Preferably, the surfactant(s), preferably the anionic or nonionic surfactants, are present in a total content ranging from 0.1% to 30% by weight, preferably ranging from 1% to 25% by weight, better still from 3% to 20% by weight, and better still from 10% to 18% by weight, relative to the total weight of the makeup-removing composition.

**Acidifying agent**

Preferably, the acidifying agent(s) may be chosen from organic acidifying agents and inorganic acidifying agents, preferably organic acidifying agents.
Preferably, the organic acidifying agent(s) are chosen from organic acids, the pKa of which at 20°C is less than 4.5, more preferentially ranging from 2.5 to 4. It should be noted that it is the pKa corresponding to the function which has the lowest acidity.
The mineral acidifying agents are chosen, for example, from hydrochloric acid, orthophosphoric acid and sulfonic acids, preferably orthophosphoric acid.
The organic acidifying agents are chosen, for example, from carboxylic acids.
The term "carboxylic acid" means simple carboxylic acids, polycarboxylic acids, notably dicarboxylic acids, amino dicarboxylic acids, tricarboxylic acids, (poly)hydroxy(poly)carboxylic acids, notably $\alpha$-hydroxy or dihydroxy carboxylic acids, which may of course be used alone or as a mixture.
As carboxylic acids that can be used in the compositions according to the invention, mention may more particularly be made of citric acid, maleic acid, succinic acid, aspartic acid, glutamic acid, lactic acid, malic acid and tartaric acid.
According to a particularly preferred embodiment of the process of the present invention, the acid used is citric acid.
The carboxylic acids used in the invention may be present in the composition, partially or totally, in the form of a salt thereof. The salts that can be used may result from the combination of the acids of the invention with an organic or mineral base, preferably a mineral base.
Among the organic bases that may be mentioned are amines and in particular alkanolamines. Among the mineral bases that may be mentioned are aqueous ammonia and alkali metal or alkaline-earth metal hydroxides. Preferably, the mineral bases are alkali metal hydroxides and even more preferentially sodium hydroxide, resulting in sodium salts.
Preferably, the acidifying agent(s) are present in a total content ranging from 0.05% to 20% by weight, preferably ranging from 0.1% to 10% by weight and better still from 0.3% to 5% by weight, relative to the total weight of the makeup-removing composition.

**Polyol**

The colour-removing composition used in the context of the process according to the invention may also comprise at least one polyol.
For the purposes of the present invention, the term "polyol" means an organic compound consisting of a hydrocarbon-based chain optionally interrupted with one or more oxygen atoms and bearing at least two free hydroxyl groups (-OH) borne by different carbon atoms, this compound possibly being cyclic or acyclic, linear or branched, and saturated or unsaturated.
More particularly, the polyol(s) comprise from 2 to 30 hydroxyl groups, more preferentially from 2 to 10 hydroxyl groups, even more preferentially from 2 to 3 hydroxyl groups.
Preferably, the colour-removing composition comprises one or more polyols chosen from diglycerol, glycerol, propylene glycol, propane-1,3-diol, 1,3-butylene glycol, pentane-1,2-diol, octane-1,2-diol, dipropylene glycol, hexylene glycol, polyethylene glycols, sorbitol, sugars, such as glucose, and mixtures thereof, preferably from propylene glycol and glycerol.
Advantageously, the polyol(s) are present in a total content ranging from 1% to 30% by weight, more preferentially from 3% to 20% by weight, and even more preferentially from 5% to 15% by weight, relative to the total weight of the makeup-removing composition.

**Alkyl or alkylene carbonate**

The makeup-removing composition used in the context of the process according to the invention may also comprise at least one alkyl or alkylene carbonate.
The term "alkyl carbonate" means an alkyl or dialkyl carbonate.
Preferably, the alkyl or alkylene carbonate is a $C_1$-$C_{30}$, preferably $C_1$-$C_6$ alkyl or $C_1$-$C_{30}$, preferably $C_1$-$C_6$ alkylene carbonate; more preferentially, the alkyl or alkylene carbonate is chosen from alkylene carbonates, better still from propylene carbonate, butylene carbonate, pentylene carbonate and mixtures thereof.
Particularly preferably, the alkyl or alkylene carbonate is chosen from propylene carbonate.
Advantageously, when it is (they are) present, the content of alkyl or alkylene carbonate ranges from 1% to 60% by weight, preferably from 3% to 20% by weight, even more preferentially from 5% to 15% by weight, relative to the total weight of the makeup-removing composition.

**Cosmetically acceptable medium**

The makeup-removing composition may comprise a cosmetically acceptable medium. The cosmetically acceptable medium may comprise a solvent chosen from water, organic solvents, and a mixture thereof.

Organic solvents

The makeup-removing composition used in the context of the process according to the invention may comprise one or more organic solvents.

Examples of organic solvents that may be mentioned include lower $C_1$-$C_4$ alkanols, such as ethanol and isopropanol; polyols and polyol ethers, for instance 2-butoxyethanol, propylene glycol, propylene glycol monomethyl ether and diethylene glycol monoethyl ether and monomethyl ether, and also aromatic alcohols, for instance benzyl alcohol or phenoxyethanol, and mixtures thereof.

The organic solvents may be present in a total amount inclusively between 0.01% and 60% by weight approximately relative to the total weight of the makeup-removing composition, preferably between 0.05% and 50% by weight and more preferentially inclusively between 0.1% and 45% by weight relative to the total weight of the makeup-removing composition.

The makeup-removing composition used in the context of the process according to the invention is preferably aqueous. The water content may range from 20% to 99% by weight, preferably from 50% to 98% by weight and more preferentially from 60% to 95% by weight relative to the total weight of the colour-removing composition.

The makeup-removing composition used in the process of the invention has a pH of less than 5, preferably ranging from 1 to 5, more preferably from 1 to 4, better still from 1.5 to 3.5.

The composition may comprise pH adjusters other than the acids of the invention. The pH adjusters may be basifying agents.

Among the basifying agents, examples that may be mentioned include aqueous ammonia, alkali metal carbonates, alkanolamines, such as monoethanolamine, diethanolamine and triethanolamine and derivatives thereof, sodium hydroxide, potassium hydroxide and the compounds of the following formula:

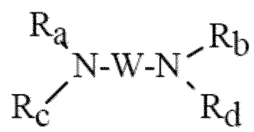

in which W is a propylene residue optionally substituted with a hydroxyl group or a $C_1$-$C_4$ alkyl radical; Ra, Rb, Rc and Rd, which may be identical or different, represent a hydrogen atom or a $C_1$-$C_4$ alkyl or $C_1$-$C_4$ hydroxyalkyl radical.

Preferably, the pH adjusters may be chosen from aqueous ammonia, monoethanolamine, diethanolamine, triethanolamine, 1,3-propanediamine and an alkaline hydroxide, such as 2-amino-2-methyl-1-propanol.

Moreover, the additives as mentioned above may be included in the makeup-removing composition used in the context of the process according to the invention.

**Protocol**

The hair treatment composition, notably the dye composition, may be used on wet or dry keratin fibres, and also on any type of fair or dark, natural or dyed, permanent-waved, bleached or relaxed keratin fibres.

According to a particular embodiment of the invention, the keratin fibres are washed before application of the hair treatment composition, notably the dye composition, comprising a photodimerizable polymer and optionally a colouring agent.

Preferably, a washing, rinsing, dewatering or drying step is performed after the application of the dye composition to the keratin fibres.

More preferentially, a drying step is performed after the application of the treatment composition, notably the hair dye composition, to the keratin fibres.

The application to the keratin fibres may be performed via any standard means, in particular using a comb, a fine brush, a coarse brush, a sponge or with the fingers.

The application of the treatment composition, notably the hair dye composition, is generally performed at room temperature (between 15 and 25°C).

After application of the hair dye composition to the keratin fibres, it is possible to wait for between 1 minute and 6 hours, in particular between 1 minute and 2 hours, more particularly between 1 minute and 1 hour, more preferentially between 1 minute and 30 minutes, before, for example, a washing, rinsing, dewatering or drying step, preferably a drying

step.

The process of the invention comprises, after the step of applying the dye composition, optionally a step of irradiating the composition on the keratin fibres to crosslink the polymer.

This irradiation may consist in illuminating the composition applied to the keratin fibres with ambient light or with an artificial light source.

The ambient or artificial light may emit radiation in the visible and/or UV range. Preferably, it emits at least a proportion of radiation in the UV range, for example a UV proportion of at least 2% of the total illuminating energy of the ambient light.

According to a particular embodiment, the exposure comprises, or even consists of, illumination with ambient light of the surface of said coat, in particular for a time of at least 1 minute.

The time of exposure to the ambient light may range more particularly from 10 seconds to 30 minutes and notably from 1 to 15 minutes.

According to another particular embodiment, the exposure comprises, or even consists of, illumination with a source of artificial light of the surface of said coat.

The time of exposure to said artificial light may range from 1 second to 20 minutes, in particular from 1 second to 10 minutes, better still from 1 second to 1 minute.

The crosslinking may take place with natural or artificial light, for example using lighting with a lamp, a flash, a laser or LEDs, for example in the form of an LED array.

The artificial light source may emit radiation in the visible range and/or radiation in the UV range.

The light emitted may or may not be monochromatic. The wavelength of the emitted light is preferably centred on 365 nm, in particular between 100 nm and 500 nm and better still between 200 nm and 450 nm.

Advantageously, the crosslinking is initiated by simple illumination without the need for a photoinitiator.

Preferably, it will be a source of artificial light emitting energy of between 0.5 and 5 W/cm$^2$, the exposure times being adapted accordingly.

The crosslinking may take place with a reduced light intensity: the lighting system may produce, for example, light intensity of between 500 mJ/cm$^2$ and 40 J/cm$^2$, preferably from 1 to 30 J/cm$^2$, better still from 5 to 25 J/cm$^2$.

The twofold characteristic of the absence of a photoinitiator and the relatively low light intensity is particularly advantageous since it makes it possible to limit the harmful effects of aggressive initiators or of prolonged exposure to intense light, in particular in the UV wavelengths.

A person skilled in the art will be capable of adapting the illumination characteristics, notably in terms of exposure time and of radiation wavelengths, with regard to the nature of the photocrosslinkable polymer(s) (A) used.

After applying the hair treatment composition, the hair can be left to dry naturally or preferably may be dried, for example at a temperature of greater than or equal to 30°C.

The process according to the invention may thus comprise a step of applying heat to the keratin fibres using a heating tool.

The heat application step of the process of the invention can be implemented by any suitable device such as a hair dryer, a heating helmet, a heating brush or a straightening or curling iron, a device emitting infrared radiation, a Climazon hood, etc.

Preferably, the heat application step of the process of the invention is performed using a hairdryer.

The process according to the invention may include a step of applying a mechanical action to the locks such as combing, brushing, or running the fingers through.

This mechanical action on the locks may be exerted before, during and/or after the step of applying heat to the hair keratin fibres.

When the step of applying heat to the hair keratin fibres is performed using a hood or a hairdryer, the temperature is preferably between 30°C and 110°C, preferentially between 50°C and 90°C.

When the step of applying heat to the hair keratin fibres is performed using a straightening iron, the temperature is preferably between 110°C and 220°C, preferably between 140°C and 200°C.

In a particular variant, the process of the invention involves a step (b1) of applying heat using a hood, a hairdryer or a Climazon hood, preferably a hairdryer, and a step (b2) of applying heat using a straightening or curling iron, preferably a straightening iron.

Step (b1) may be performed before step (b2).

During step (b 1), also referred to as the drying step, the hair keratin fibres may be dried, for example at a temperature of greater than or equal to 30°C. According to a particular embodiment, this temperature is greater than 40°C. According to a particular embodiment, this temperature is greater than 45°C and less than 110°C.

Preferably, if the hair keratin fibres are dried, they are dried, in addition to a supply of heat, with a flow of air. This flow of air during drying makes it possible to improve the strand separation of the coating.

During the drying, a mechanical action may be exerted on the locks, such as combing, brushing or running the fingers through.

During step (b2), the passage of the straightening or curling iron, preferably the straightening iron, may be performed at a temperature ranging from 110°C to 220°C, preferably between 140°C and 200°C.

After the heating step, a shaping step may be performed, for example with a straightening iron; the temperature for the shaping step is between 110 and 220°C, preferably between 140 and 200°C.

Preferably, the step of applying the hair dye composition to the keratin fibres is repeated several times.

According to a preferred embodiment, the hair keratin fibre treatment process is a treatment process for removing colour from hair keratin fibres, such as the hair, which have been dyed beforehand with the hair dye composition as defined above, comprising the application of the hair dye composition to the keratin fibres, comprising at least one photodimerizable polymer, at least one colouring agent chosen from pigments, direct dyes and mixtures thereof.

Next, as indicated previously, the process according to the invention comprises the application of at least one makeup-removing composition for removing colour from keratin fibres, as defined above, said fibres having been dyed beforehand using at least one hair dye composition as defined above.

The makeup-removing composition is preferably left in contact with the hair to be treated for a leave-on time which may range from 30 seconds to 30 minutes, preferably ranging from 1 minute to 25 minutes, more particularly from 10 to 20 minutes.

The process according to the invention preferably comprises a step of applying heat to the keratin fibres using a heating tool during the application of the makeup-removing composition.

The application of heat may be performed using any suitable device such as a hair dryer, a heated helmet, a heated brush, a device emitting infrared radiation, a Climazon hood, etc.

Preferably, the keratin fibres are heated to a temperature ranging from 30°C to 90°C, preferably from 40 to 80°C.

In conjunction with this heat input, an occlusive covering will preferentially be used on the treated hair to prevent evaporation of the composition.

For example, aluminium foil wraps can be used on the locks of hair or, optionally, a cap can be used on the whole head.

Following the application of the colour-removing composition and its leave-on time, the process of the invention will preferentially comprise a mechanical action, which may be performed with a tool such as a brush and notably a double-sided brush clamp, an exfoliating glove, or without any tool other than the hands. A combination of finger massage and the action of a tool such as a brush may be used.

The process may also include steps after the application of the makeup-removing composition to remove the colour, such as rinsing or drying steps or applications of additional cosmetic compositions and notably hair shaping or conditioning compositions.

Preferably, the makeup-removing composition for removing colour is then rinsed out after an optional leave-on time, optionally followed by shampoo washing.

The step of applying the makeup-removing composition notably for removing colour may be repeated several times, optionally with intermediate rinsing.

The time between the step of applying the hair keratin fibre dye composition and the step of applying the makeup-removing composition notably for removing colour may range from a few minutes to several days, for example several tens of days. Preferably, the time between the step of applying the hair keratin fibre dye composition and the step of applying the makeup-removing composition notably for removing colour ranges from 1 hour to 30 days, more preferentially from 1 day to 15 days.

Preferably, the invention is a treatment process notably for removing colour from keratin fibres, such as the hair, which have been dyed beforehand, notably comprising:

i) the application to said hair keratin fibres of the hair treatment composition as described above, and then
ii) optionally a leave-on time of said hair dye composition on the fibres of from 1 minute to 30 minutes; and then
iii) optionally a step of irradiation with wavelengths ranging from 280 to 450 nm;
vi) optionally a step of washing, rinsing, dewatering or drying said hair keratin fibres;
v) application of the makeup-removing composition as defined above to the moistened keratin fibres;
vi) optionally occlusive covering of the locks with a material such as aluminium foil wraps;
vii) application of heat at a temperature of more than 30°C preferably from 35 to 90°C for 1 to 30 minutes;
viii) after removal of the covering, mechanical action on the locks, preferably with a tool in particular with a double-sided brush clamp;
ix) rinsing the keratin fibres.

Use

The present invention also relates to the use of the makeup-removing composition for removing colour, according to the invention or as used in the context of the process according to the invention, from hair keratin fibres which have been

dyed beforehand with a dye composition comprising at least one photodimerizable polymer and at least one colouring agent chosen from pigments, direct dyes and mixtures thereof.

The present invention will now be described more specifically by means of examples, which do not in any way limit the scope of the invention. However, the examples make it possible to support specific features, variants and preferred embodiments of the invention.

**Examples**

**Example 1:**

Composition C1 was prepared with the compounds in Table 1 below: the amounts are expressed as g of unmodified starting material/100 g.

[Tables 1]

| C1 | |
|---|---|
| Propyl-PVA-SbQ (Polyquaternium-114 PPVA-SBQ from Murami) as an aqueous solution containing 15% active material | 40.3% (6% AM) |
| Mica and iron oxide (Pearlescent Pigment Prestige Soft Bronze from Sudarshan Chemical) | 6.5% |
| Glycerol | 5% |
| Propylene carbonate | 5.5% |
| Mixture of dimethicone and amomethicone as an aqueous emulsion (Belsil DADM 5050$^E$ from Wacker) | 15% (7.5% AM) |
| Water qs | 100% |

Dyeing protocol:

Composition C1 was applied to locks of dry sensitized hair (AS20) containing 90% white hair strands, at a rate of 0.5 g of composition per gram of lock, for 15 minutes. The locks of hair are then dried using a hair dryer with a round or flat brush or a comb, and then combed.

The treated hair is then irradiated with UVA at 365 nm with an energy of 8 J/cm$^2$.

The locks of hair thus dyed are subsequently stored at 60°C for 48 hours, and then undergo a colour removal treatment.

Makeup-removing compositions for removing colour

A colour-removing composition D1 according to the invention described in the table below was prepared. The amounts are expressed as percentages of active material (AM).

[Table 2]

| Composition | D1 |
|---|---|
| Sodium lauryl ether sulfate as an aqueous solution containing 70% AM | 14% AM |
| Citric acid | 0.8 |
| Sodium chloride | 1.75 |
| Water qs | 100 |

The pH of the composition is 3.

Protocol for makeup removal from the locks of hair

The locks of hair dyed with composition C1 were treated with composition D1 above.

Composition D1 was applied to a lock of wet dyed hair at a rate of 2 g of composition per g of lock.

The locks were placed in aluminium foil wraps and left to stand for 15 minutes while supplying heat using a

Climazon hood set at 50-55°C so as to be at least 45°C inside the wrap.

After removing the wraps, the locks were brushed with a double-sided brush clamp and then massaged with the fingers.

The locks were then rinsed and washed with shampoo, and then dried.

The colour-removing (or decolourizing) power is then measured by colorimetry.

The colour of the locks was evaluated in the CIE L* a* b* system, using a Minolta Spectrophotometer CM3600A colorimeter (illuminant D65, angle 10°, specular component included).

In this L*a*b* system, L* represents the intensity of the colour, a* indicates the green/red colour axis and b* the blue/yellow colour axis.

The colour removal is evaluated by the colour difference ΔE between coloured and uncoloured locks, on the one hand, and between coloured locks and makeup-removed locks, on the other hand. The closer the ΔE values are, the more colour has been removed from the locks.

The ΔE value is calculated according to the following equation:

[Math.1]

$$\Delta E = \sqrt{(L^* - L_o^*)^2 + (a^* - a_o^*)^2 + (b^* - b_o^*)^2}$$

In this equation, L*, a* and b* represent the values measured after dyeing the hair, and $L_0$*, $a_0$* and $b_0$* represent the values measured before dyeing the hair or after makeup removal.

The results are described in the table below:

[Table 3]

| | ΔE |
|---|---|
| Non-dyed lock | 12.7 |
| Dyed lock | - |
| After makeup removal | 12.6 |

The ΔE values are very close, which shows that the locks which have been freed of the makeup have regained the colour of the original non-dyed lock.

It was found that the makeup-removing composition according to the invention has excellent colour-removing power.

Thus, the process according to the invention makes it possible to improve the removal of colour from a lock of hair which has been dyed beforehand using a hair dye composition comprising a photodimerizable polymer and a colouring agent.

Example 2

The makeup-removing compositions according to the invention were prepared.

[Table 4]

| **Composition** | **D2** | **D3** |
|---|---|---|
| Sodium lauryl ether sulfate as an aqueous solution containing 70% AM | 21% AM | 5.6% AM |
| Citric acid | 0.8 | 5 |
| Sodium chloride | 2 | |
| Phenoxyethanol | | 0.7 |
| Water qs | 100% | 100% |

pH 3

Composition D1 can be replaced with compositions D2 or D3 to obtain the same type of results.

## Claims

1. Process for removing makeup from keratin fibres to remove colour of keratin fibres which have been treated beforehand with a composition comprising:

   - at least one photocrosslinkable polymer including at least one photodimerizable group; and
   - at least one colouring agent chosen from pigments, direct dyes and mixtures thereof,

   this process comprising the application to the keratin fibres of a makeup-removing composition for removing colour, comprising:

   - at least one surfactant, preferably an anionic or nonionic surfactant, preferably an anionic surfactant; and
   - at least one acidifying agent;

   said makeup-removing composition having a pH of less than or equal to 5.5, preferably from 1 to 5,
   the photodimerizable group(s) are chosen from monovalent radicals of formulae (I) and (II) below:

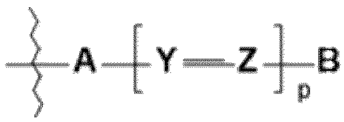

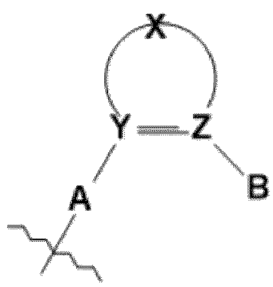

   and also the geometrical isomers thereof,
   in which formulae **(I)** and **(II):**

   - **Y** and **Z** denote, independently of each other, a nitrogen atom or a group C(R) with R representing a hydrogen atom or a $(C_1-C_4)$alkyl group such as methyl;
   - **A** represents a bond or a divalent group chosen from $(C_1-C_8)$alkylene, arylene, heteroarylene, cycloalkylene, heterocycloalkylene, (thio)carbonyl and $(C_2-C_8)$alkenylene radicals and combinations thereof;
   - **B** represents a monovalent group chosen from $(C_1-C_8)$alkyl radicals, aryl radicals, optionally cationic heteroaryl radicals, cycloalkyl radicals, optionally cationic heterocycloalkyl radicals, (thio)carbonyl radicals and $(C_2-C_8)$ alkenyl radicals and combinations thereof;
   - **X** represents a divalent group chosen from $(C_2-C_8)$alkylene, arylene, heteroarylene, cycloalkylene, heterocycloalkylene, (thio)carbonyl and $(C_2-C_8)$alkenylene radicals and combinations thereof;
   - **p** represents an integer between 1 and 5 inclusive, in particular between 1 and 3; preferably, p is equal to 1;

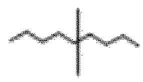

   represents the bond which connects the part of the monovalent radical to the rest of the molecule;

   and

   each of the groups mentioned may optionally be substituted with one or more halogen atoms or groups chosen from $(C_1-C_6)$alkyl, hydroxyl, amino, (di)$(C_1-C_6)$alkylamino, phenyl, carboxyl and $(C_1-C_6)$alkoxy groups, $(C_1-C_6)$alkoxy(thio)carbonyl, hydrogeno(thio)carbonyl, sulfonato $R-O-S(O)_2-$ or $R-S(O)_2-O-$, amide RR'N-C(O)- or R-C(O)-N(R')- or acyl R-C(O)-, ammonium $RR'R''N^+-$ with R, R' and R", which

may be identical or different, representing a hydrogen atom or a $(C_1-C_4)$alkyl group.

**2.** Process according to Claim 1, **characterized in that** the photodimerizable group(s) are chosen from the monovalent radicals of the following compounds:

- stilbene,
- styrylpyridinium (stilbazolium) having the formula below, and the geometrical isomers thereof:

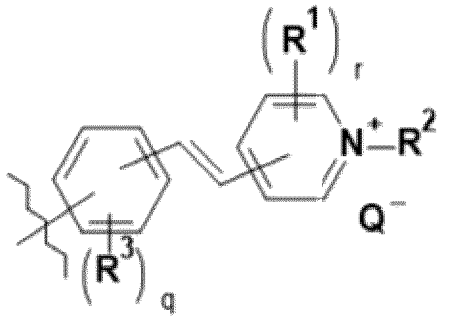

(A1)

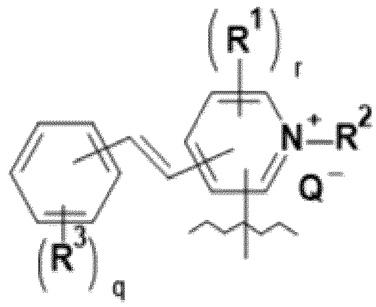

(A2)

where

- **$R^1$** and **$R^3$,** which may be identical or different, represent a halogen atom or a $(C_1-C_6)$alkyl group; or else two contiguous $R^1$ or $R^3$ groups form, together with the carbon atoms bearing them, a benzo group;
- **$R^2$** represents a hydrogen atom, a $(C_1-C_6)$alkyl group optionally substituted with one or more halogen atoms such as chlorine or hydroxyl; preferably, $R^2$ represents a $(C_1-C_6)$alkyl group such as methyl, ethyl or propyl;
- **q** and **r** represent an integer between 0 and 4 inclusive; and
- **$Q^-$** represents an anionic counter ion preferably chosen from halide ions such as chlorides, bromides and iodides, perchlorates, tetrafluoroborates, methyl sulfate, phosphates, sulfates, methanesulfonates, p-toluenesulfonate; and

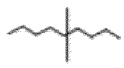

represents the bond which connects the part of the monovalent radical to the rest of the molecule, it being understood that the pendent group $A_2$ can be connected to the rest of the molecule *via* $R^2$;
preferably, the

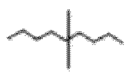

bond is located on the phenyl para to the styryl group on $A_1$ or connected to the rest of the molecule *via* $R^2$ on $A_2$; preferentially, the styryl group of $A_1$ and $A_2$ is located in the position para to the pyridinium group;

- styrylazolium of the formula below, and the geometrical isomers thereof:

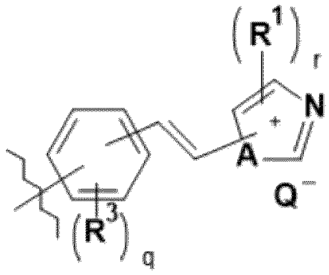 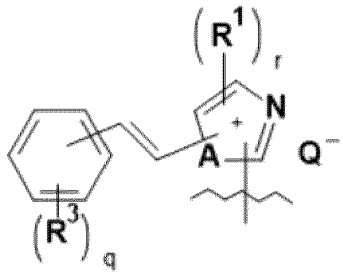

where:

- A represents a sulfur atom, an oxygen atom, or a group $NR^2$ or $C(R^2)_2$; and
-

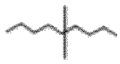

$Q^-$, r, q, $R^1$, $R^2$ and $R^3$ being as defined previously,
preferably, the

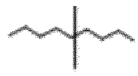

bond is located on the phenyl in the position para to the stryryl group,

- styrylpyrazine,
- chalcone,
- (thio)cinnamate and (thio)cinnamamide,
- maleimide,
- (thio)coumarin,
- thymine,
- uracil,
- butadiene,
- anthracene,
- pyridone,
- pyrrolizinone,
- acridizinium salts,
- furanone,
- phenylbenzoxazole, and
- derivatives thereof.

3. Process according to any one of the preceding claims, **characterized in that** the photocrosslinkable polymer has a natural or synthetic backbone chosen from polysaccharides, poly(vinyl) polymers and polydiorganosiloxanes, preferably from polyvinyl alcohols and poly(vinyl acetate)s, which are preferably partially or totally hydrolysed.

4. Process according to any one of the preceding claims, **characterized in that** the photocrosslinkable polymer includes at least one hydrophobic pendent group.

5. Process according to the preceding claim, **characterized in that** the hydrophobic group(s) are chosen from:

- a saturated or unsaturated $(C_1-C_{30})$alkyl group, optionally substituted and/or interrupted with one or more heteroatoms,
- an alkenyl group,
- an aryl group such as phenyl, pyridyl, furyl, indoyl, benzofuryl, thiophenyl, imizadoyl, oxazoyl, thiazoyl, pyrazinyl, pyrimidinyl;
- a fluoro group such as fluorocarbon group such as $-CF_3$, $-CHF_2$, $-OCF_3$, $SCF_3$ or $CF_3C(O)-$,

- a silicone group such as $-SiR_aR_bR_c$ such as $-Si(CH_3)_3$, polydimethylsiloxane PDMS, $-Si(OR)_3$, PDMS $\alpha,\omega$-diaminopropyl, PDMS $\alpha,\omega$-dihydroxyalkyl, PDMS $\alpha,\omega$-dicarboxyalkyl, with $R_a$, $R_b$ and $R_c$, which may be identical or different, representing a $(C_1-C_8)$alkyl group which is optionally interrupted and/or terminated with one or more non-contiguous heteroatoms such as O or S; and R representing a $(C_1-C_6)$alkyl group;

preferably from a $(C_2-C_{22})$alkyl group, more preferentially a $(C_3-C_{16})$alkyl group.

6. Process according to any one of the preceding claims, **characterized in that** the photocrosslinkable polymer has a natural or synthetic backbone chosen from polysaccharides, poly(vinyl) polymers and polydiorganosiloxanes, preferably from polyvinyl alcohols and poly(vinyl acetate)s, which are preferably partially or totally hydrolysed.

7. Process according to any one of the preceding claims, **characterized in that** the photocrosslinkable polymer(s) are chosen from polymers comprising at least the units below:

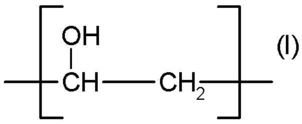
(I)

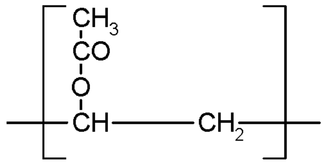
(II)

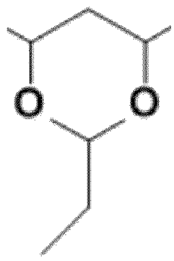

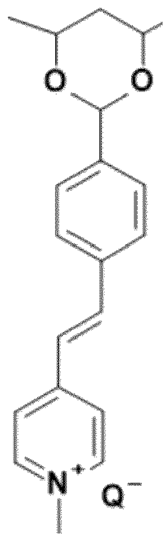

with $Q^-$ representing an anionic counterion preferably chosen from halide ions such as chlorides, bromides, iodides, perchlorates, tetrafluoroborates, methylsulfate, phosphates, sulfates, methanesulfonates, p-toluenesulfonate, preferably mesylate $CH_3OSO_3^-$;

8. Process according to any one of the preceding claims, **characterized in that** the total amount of the photocrosslinkable polymer(s) ranges from 0.01% to 40% by weight, preferably from 0.1% to 30% by weight, better still from 0.5% to 25% by weight and even better still from 1% to 10% by weight, relative to the total weight of composition (C).

9. Process according to any one of the preceding claims, **characterized in that** the content of colouring agent(s) ranges from 0.001% to 20% by weight and preferably from 0.005% to 15% by weight relative to the total weight of the hair dye composition; preferably, the colouring agent(s) are chosen from pigments.

10. Process according to any one of the preceding claims, **characterized in that** the makeup-removing composition for removing colour comprises at least one anionic surfactant chosen from anionic sulfate surfactants, carboxylate anionic surfactants, sulfonate anionic surfactants and mixtures thereof, preferably from alkyl sulfates, alkyl ether sulfates, alkyl amido ether sulfates, alkylaryl polyether sulfates, monoglyceride sulfates; and also the salts of these compounds;

the alkyl groups of these compounds including from 6 to 30 carbon atoms, notably from 12 to 28, even better still

from 14 to 24 or even from 16 to 22 carbon atoms; the aryl group preferably denoting a phenyl or benzyl group; these compounds possibly being (poly)oxyalkylenated, notably (poly)oxyethylenated, and then preferably including from 1 to 50 ethylene oxide units and better still from 1 to 10 ethylene oxide units, and mixtures thereof.

**11.** Process according to any one of the preceding claims, **characterized in that** the makeup-removing composition for removing colour comprises at least one nonionic surfactant chosen from:

- alcohols, α-diols and (C1-20)alkylphenols, these compounds being polyethoxylated and/or polypropoxylated and/or polyglycerolated, the number of ethylene oxide and/or propylene oxide groups possibly ranging from 1 to 100, and the number of glycerol groups possibly ranging from 2 to 30; and/or these compounds comprising at least one fatty chain including from 8 to 30 carbon atoms and notably from 16 to 30 carbon atoms;
- condensates of ethylene oxide and of propylene oxide with fatty alcohols; polyethoxylated fatty amides preferably containing from 2 to 30 ethylene oxide units, polyglycerolated fatty amides including on average from 1 to 5, and in particular from 1.5 to 4, glycerol groups; C8-C30 fatty acid alkanolamides, such as C8-C30 fatty acid monoalkanolamides, in particular C8-C30 fatty acid monoethanolamides or monoisopropanolamides; ethoxylated fatty acid esters of sorbitan preferably containing from 2 to 40 ethylene oxide units, fatty acid esters of sucrose, polyoxyalkylenated and preferably polyoxyethylenated fatty acid esters containing from 2 to 150 mol of ethylene oxide, including oxyethylenated plant oils, N-(C6-24 alkyl)glucamine derivatives, amine oxides such as (C10-14 alkyl)amine oxides or N-(C10-14 acyl)aminopropylmorpholine oxides;
- nonionic surfactants of alkylpolyglycoside type, notably represented by the following general formula: R1O-(R2O)t-(G)v in which:
- R1 represents a linear or branched alkyl or alkenyl radical including 6 to 24 carbon atoms and notably 8 to 18 carbon atoms, or an alkylphenyl radical of which the linear or branched alkyl radical includes 6 to 24 carbon atoms and notably 8 to 18 carbon atoms;
- R2 represents an alkylene radical including 2 to 4 carbon atoms;
- G represents a sugar unit including 5 to 6 carbon atoms;
- t denotes a value ranging from 0 to 10 and preferably from 0 to 4;
- v denotes a value ranging from 1 to 15 and preferably from 1 to 4.

**12.** Process according to any one of the preceding claims, **characterized in that** the surfactant(s), preferably the anionic surfactants, are present in a total content ranging from 0.1% to 30% by weight, preferably ranging from 1% to 25% by weight, better still from 3% to 20% by weight, and better still from 10% to 18% by weight, relative to the total weight of the makeup-removing composition notably for removing colour.

**13.** Process according to any one of the preceding claims, **characterised in that** the acidifying agent(s) are chosen from organic acids, mineral acids and mixtures thereof, preferably from organic carboxylic acids.

**14.** Process according to any one of the preceding claims, **characterized in that** the acidifying agent(s) are present in a total content ranging from 0.05% to 20% by weight, more preferentially from 0.1% to 10% by weight, and even more preferentially from 0.3% to 5% by weight, relative to the total weight of the makeup-removing composition notably for removing colour.

**15.** Process according to any one of the preceding claims, **characterized in that** it also comprises a step of applying heat to the keratin fibres during or after the application of the makeup removing composition notably for removing colour.

**16.** Process according to any one of the preceding claims, **characterized in that** it also comprises a step of applying a mechanical action, notably friction, to the keratin fibres after the application of the makeup removing composition for removing colour.

**17.** Process according to any one of the preceding claims, **characterized in that** it comprises

i) the application to said hair keratin fibres of the hair treatment composition according to one of Claims 1 to 9; and then
ii) optionally a leave-on time of said hair dye composition on the fibres of from 1 minute to 30 minutes; and then
iii) optionally a step of irradiation with wavelengths ranging from 280 to 450 nm;
vi) optionally a step of washing, rinsing, dewatering or drying said hair keratin fibres;
v) application of the makeup-removing composition according to one of Claims 1 and 11 to 15 to the moistened keratin fibres;

vi) optionally occlusive covering of the locks with a material such as aluminium foil wraps;
vii) application of heat at a temperature of more than 30°C preferably from 35 to 90°C for 1 to 30 minutes;
viii) after removal of the covering, mechanical action on the locks preferably with a tool;
ix) rinsing the keratin fibres.

**18.** Use of the makeup-removing composition for removing colour, as defined in any one of Claims 1 to 14, from keratin fibres which have been treated beforehand with a hair dye composition comprising at least one photocrosslinkable polymer such as disclosed in any claims 1 to 8 and at least one colouring agent chosen from pigments, direct dyes and mixtures thereof.

**Patentansprüche**

**1.** Verfahren zum Entfernen von Make-up von Keratinfasern zum Entfernen von Farbe von Keratinfasern, die zuvor mit einer Zusammensetzung behandelt worden sind, die umfasst:

- wenigstens ein photovernetzbares Polymer, das wenigstens eine photodimerisierbare Gruppe enthält; und
- wenigstens ein Farbmittel ausgewählt aus Pigmenten, Direktfarbstoffen und Gemischen davon,

wobei das Verfahren Aufbringen einer Make-up-Entfernungszusammensetzung zum Entfernen von Farbe auf die Keratinfasern umfasst, die umfasst:

- wenigstens ein Tensid, vorzugsweise ein anionisches oder nichtionisches Tensid, vorzugsweise ein anionisches Tensid; und
- wenigstens ein Säuerungsmittel;

wobei die Make-up-Entfernungszusammensetzung einen pH-Wert von weniger als oder gleich 5,5, vorzugsweise von 1 bis 5, aufweist,
die photodimerisierbaren Gruppe(n) ausgewählt sind aus einwertigen Resten der nachstehenden Formeln (I) und (II):

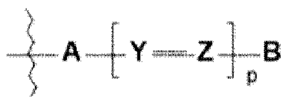

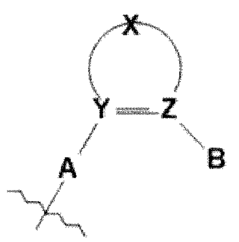

und den geometrischen Isomeren davon,
wobei in den Formeln **(I)** und **(II):**

- **Y** und **Z** unabhängig voneinander ein Stickstoffatom oder eine Gruppe C(R) bedeuten, wobei R ein Wasserstoffatom oder eine $(C_1-C_4)$Alkylgruppe, wie z.B. Methyl, darstellt;
- **A** eine Bindung oder eine zweiwertige Gruppe ausgewählt aus $(C_1-C_8)$Alkylen-, Arylen-, Heteroarylen-, Cycloalkylen-, Heterocycloalkylen-, (Thio)carbonyl- und $(C_2-C_8)$Alkenylenresten und Kombinationen davon darstellt;
- **B** eine einwertige Gruppe ausgewählt aus $(C_1-C_8)$Alkylresten, Arylresten, gegebenenfalls kationischen Heteroarylresten, Cycloalkylresten, gegebenenfalls kationischen Heterocycloalkylresten, (Thio)carbonylresten und $(C_2-C_8)$Alkenylresten und Kombinationen davon darstellt;
- **X** eine zweiwertige Gruppe ausgewählt aus $(C_2-C_8)$Alkylen-, Arylen-, Heteroarylen-, Cycloalkylen-, Heterocycloalkylen-, (Thio)carbonyl- und $(C_2-C_8)$Alkenylenresten und Kombinationen davon darstellt;
- **P** eine ganze Zahl zwischen einschließlich 1 und 5, insbesondere zwischen 1 und 3, darstellt; wobei p vorzugsweise gleich 1 ist;

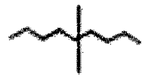

die Bindung darstellt, die den Teil des einwertigen Rests mit dem Rest des Moleküls verbindet;

und

jede der genannten Gruppen gegebenenfalls substituiert sein kann mit einem oder mehreren Halogenatomen oder Gruppen ausgewählt aus $(C_1-C_6)$Alkyl-, Hydroxy-, Amino-, (Di)$(C_1-C_6)$alkylamino-, Phenyl-, Carboxyl- und $(C_1-C_6)$ Alkoxygruppen,
$(C_1-C_6)$ Alkoxy (thio) carbonyl, Hydrogeno (thio) carbonyl, Sulfonato R-O-$S(O)_2$- oder R-$S(O)_2$-O-, Amid-RR'N-C(O)- oder R-C(O)-N(R')- oder Acyl-R-C(O)-, Ammonium-RR'R"$N^+$-, wobei R, R' und R", die gleich oder verschieden sein können, ein Wasserstoffatom oder eine $(C_1-C_4)$Alkylgruppe darstellen.

**2.** Verfahren nach Anspruch 1, **dadurch gekennzeichnet, dass** die photodimerisierbaren)Gruppe(n) ausgewählt sind aus den einwertigen Resten der folgenden Verbindungen:

- Stilben,
- Styrylpyridinium (Stilbazolium) mit den folgenden Formeln und den geometrischen Isomeren davon:

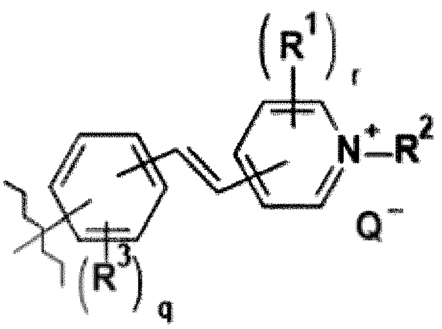

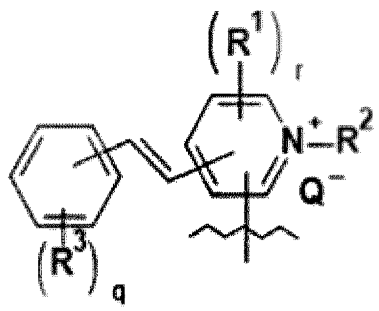

$(A_1)$ $(A_2)$

wobei
- **$R^1$** und **$R^3$,** die gleich oder verschieden sein können, ein Halogenatom oder eine $(C_1-C_6)$-Alkylgruppe darstellen; oder zwei benachbarte $R^1$- oder $R^3$-Gruppen zusammen mit den Kohlenstoffatomen, die sie tragen, eine Benzogruppe bilden;
- **$R^2$** ein Wasserstoffatom, eine $(C_1-C_6)$Alkylgruppe, gegebenenfalls substituiert mit einem oder mehreren Halogenatomen, wie z.B. Chlor, oder Hydroxy, darstellt; wobei $R^2$ vorzugsweise eine $(C_1-C_6)$Alkylgruppe, wie z.B. Methyl, Ethyl oder Propyl, darstellt;
- **q** und **r** eine ganze Zahl zwischen einschließlich 0 und 4 darstellen; und
- **$Q^-$** ein anionisches Gegenion darstellt, vorzugsweise ausgewählt aus Halogenidionen, wie z.B. Chloriden, Bromiden und Iodiden, Perchloraten, Tetrafluorboraten, Methylsulfat, Phosphaten, Sulfaten, Methansulfonaten, p-Toluolsulfonat; und

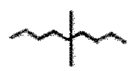

die Bindung darstellt, die den Teil des einwertigen Rests mit dem Rest des Moleküls verbindet, wobei zu beachten ist, dass die Seitengruppe $A_2$ über $R^2$ mit dem Rest des Moleküls verbunden sein kann;
wobei die

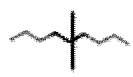

-Bindung vorzugsweise an dem Phenyl para bezogen auf die Styrylgruppe an $A_1$ angeordnet ist oder über $R^2$ an $A_2$ mit dem Rest des Moleküls verbunden ist; wobei die Styrylgruppe von $A_1$ und $A_2$ vorzugsweise an der Position para bezogen auf die Pyridiniumgruppe angeordnet ist;
- Styrylazolium der nachstehenden Formel und den geometrischen Isomeren davon:

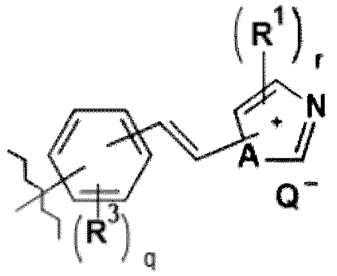

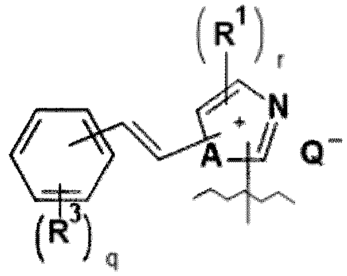

wobei:

- A ein Schwefelatom, ein Sauerstoffatom oder eine Gruppe $NR^2$ oder $C(R^2)_2$ darstellt; und

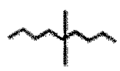

$Q^-$, r, q, $R^1$, $R^2$ und $R^3$ wie vorstehend definiert sind,
die

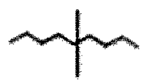

-Bindung vorzugsweise an dem Phenyl in der Position para bezogen auf die Stryrylgruppe angeordnet ist,
- Styrylpyrazin,
- Chalcon,
- (Thio)cinnamat und (Thio)cinnamamid,
- Maleimid,
- (Thio)cumarin,
- Thymin,
- Uracil,
- Butadien,
- Anthracen,
- Pyridon,
- Pyrrolizinon,
- Acridiziniumsalzen,
- Furanon,
- Phenylbenzoxazol, und
- Derivaten davon.

3. Verfahren nach einem der vorstehenden Ansprüche, **dadurch gekennzeichnet, dass** das photovernetzbare Polymer ein natürliches oder synthetisches Gerüst ausgewählt aus Polysacchariden, Poly(vinyl)polymeren und Polydiorganosiloxanen, vorzugsweise aus Polyvinylalkoholen und Poly(vinylacetat)en, die vorzugsweise teilweise oder vollständig hydrolysiert sind, aufweist.

4. Verfahren nach einem der vorstehenden Ansprüche, **dadurch gekennzeichnet, dass** das photovernetzbare Polymer wenigstens eine hydrophobe Seitengruppe enthält.

5. Zusammensetzung nach dem vorstehenden Anspruch, **dadurch gekennzeichnet, dass** die hydrophoben Gruppe(n) ausgewählt sind aus:

- einer gesättigten oder ungesättigten ($C_1$-$C_{30}$)Alkylgruppe, gegebenenfalls substituiert mit und/oder unterbrochen von einem oder mehreren Heteroatomen,
- einer Alkenylgruppe,
- einer Arylgruppe, wie z.B. Phenyl, Pyridyl, Furyl, Indoyl, Benzofuryl, Thiophenyl, Imizadoyl, Oxazoyl, Thiazoyl, Pyrazinyl, Pyrimidinyl;
- einer Fluorgruppe, wie z.B. Fluorkohlenstoffgruppe, wie z.B. $-CF_3$, $-CHF_2$, $-OCF_3$, $SCF_3$ oder $CF_3C(O)$-,
- einer Silicongruppe, wie z.B. $-SiR_aR_bR_c$, wie z.B. $-Si(CH_3)_3$, Polydimethylsiloxan PDMS, $-Si(OR)_3$, PDMS $\alpha,\omega$-Diaminopropyl, PDMS $\alpha,\omega$-Dihydroxyalkyl, PDMS $\alpha,\omega$-Dicarboxyalkyl, wobei $R_a$, $R_b$ und $R_c$, die gleich oder verschieden sein können, eine ($C_1$-$C_8$)-Alkylgruppe darstellen, die gegebenenfalls mit einem oder mehreren nicht zusammenhängenden Heteroatomen, wie z.B. O oder S, unterbrochen und/oder terminiert ist; und R eine ($C_1$-$C_6$)-Alkylgruppe darstellt;

vorzugsweise aus einer ($C_2$-$C_{22}$)Alkylgruppe, bevorzugter einer ($C_3$-$C_{16}$)Alkylgruppe.

6. Verfahren nach einem der vorstehenden Ansprüche, **dadurch gekennzeichnet, dass** das photovernetzbare Polymer ein natürliches oder synthetisches Gerüst ausgewählt aus Polysacchariden, Poly(vinyl)polymeren und Polydiorganosiloxanen, vorzugsweise aus Polyvinylalkoholen und Poly(vinylacetat)en, die vorzugsweise teilweise oder vollständig hydrolysiert sind, aufweist.

7. Verfahren nach einem der vorstehenden Ansprüche, **dadurch gekennzeichnet, dass** die photovernetzbaren Polymer(e) ausgewählt sind aus Polymeren, die wenigstens die nachstehenden Einheiten umfassen:

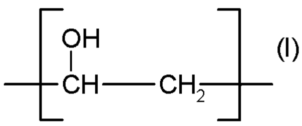 (I) 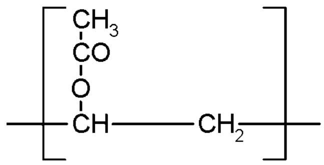 (II)

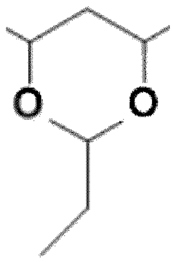 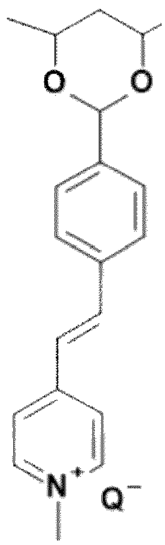

wobei $Q^-$ ein anionisches Gegenion vorzugsweise ausgewählt aus Halogenidionen, wie z.B. Chloriden, Bromiden, Iodiden, Perchloraten, Tetrafluorboraten, Methylsulfat, Phosphaten, Sulfaten, Methansulfonaten, p-Toluolsulfonat, vorzugsweise Mesylat $CH_3OSO_3^-$, darstellt.

8. Verfahren nach einem der vorstehenden Ansprüche, **dadurch gekennzeichnet, dass** die Gesamtmenge der photoverrnetzbaren Polymer(e) in dem Bereich von 0,01 Gew.-% bis 40 Gew.-%, vorzugsweise von 0,1 Gew.-% bis 30 Gew.-%, noch besser von 0,5 Gew.-% bis 25 Gew.-% und sogar noch besser von 1 Gew.-% bis 10 Gew.-%, bezogen auf das Gesamtgewicht von Zusammensetzung (C), liegt.

9. Verfahren nach einem der vorstehenden Ansprüche, **dadurch gekennzeichnet, dass** der Gehalt an Farbmittel(n) in dem Bereich von 0,001 Gew.-% bis 20 Gew.-% und vorzugsweise von 0,005 Gew.-% bis 15 Gew.-%, bezogen auf das

Gesamtgewicht vder Haarfärbezusammensetzung, liegt; wobei die Farbmittel vorzugsweise aus Pigmenten ausgewählt sind.

**10.** Verfahren nach einem der vorstehenden Ansprüche, **dadurch gekennzeichnet, dass** die Make-up-Entfernungszusammensetzung zum Entfernen von Farbe wenigstens ein anionisches Tensid ausgewählt aus anionischen Sulfattensiden, anionischen Carboxylattensiden, anionischen Sulfonattensiden und Gemischen davon, vorzugsweise aus Alkylsulfaten, Alkylethersulfaten, Alkylamidoethersulfaten, Alkylarylpolyethersulfaten, Monoglyceridsulfaten; sowie den Salze dieser Verbindungen umfasst;

wobei die Alkylgruppen dieser Verbindungen von 6 bis 30 Kohlenstoffatome, insbesondere von 12 bis 28, noch besser von 14 bis 24 oder sogar von 16 bis 22 Kohlenstoffatome enthalten; wobei die Arylgruppe vorzugsweise eine Phenyl- oder Benzylgruppe bedeutet;
wobei diese Verbindungen gegebenenfalls (poly)oxyalkyliert sind, insbesondere (poly)oxyethyleniert, und dann vorzugsweise von 1 bis 50 Ethylenoxideinheiten und noch besser 1 bis 10 Ethylenoxideinheiten und Gemische davon enthalten.

**11.** Verfahren nach einem der vorstehenden Ansprüche, **dadurch gekennzeichnet, dass** die Make-up-Entfernungszusammensetzung zum Entfernen von Farbe wenigstens ein nichtionisches Tensid umfasst, das ausgewählt ist aus

- Alkoholen, $\alpha$-Diolen und (C1-20)-Alkylphenolen, wobei diese Verbindungen polyethoxyliert und/oder polypropoxyliert und/oder polyglyceroliert sind, wobei die Zahl der Ethylenoxid- und/oder Propylenoxidgruppen möglicherweise in dem Bereich von 1 bis 100 und die Zahl der Glycerolgruppen möglicherweise in dem Bereich von 2 bis 30 liegt; und/oder diese Verbindungen wenigstens eine Fettkette mit von 8 bis 30 Kohlenstoffatomen und insbesondere von 16 bis 30 Kohlenstoffatomen umfassen;
- Kondensaten von Ethylenoxid und von Propylenoxid mit Fettalkoholen; polyethoxylierten Fettamiden mit vorzugsweise von 2 bis 30 Ethylenoxideinheiten, polyglycerolierten Fettamiden mit im Mittel von 1 bis 5 und insbesondere 1,5 bis 4 Glycerolgruppen; C8-C30-Fettsäurealkanolamiden, wie z.B. C8-C30-Fettsäuremonoalkanolamiden, insbesondere C8-C30-Fettsäuremonoethanolamiden oder Monoisopropanolamiden; ethoxylierten Fettsäureestern von Sorbitan mit vorzugsweise von 2 bis 40 Ethylenoxideinheiten, Fettsäureestern von Saccharose, polyoxyalkylierten und vorzugsweise polyoxyethylenierten Fettsäureestern mit von 2 bis 150 Mol Ethylenoxid, einschließlich oxethylierter Pflanzenöle, N-(C6-24-Alkyl)glucaminderivaten, Aminoxiden, wie z. B. (C10-14-Alkyl)aminoxiden oder N-(C10-14-Acyl)aminopropylmorpholinoxiden;
- nichtionischen Tensiden vom Alkylpolyglycosid-Typ, insbesondere dargestellt durch die folgende allgemeine Formel: R1O-(R2O)t-(G)v, wobei:
- R1 einen linearen oder verzweigten Alkyl- oder Alkenylrest mit 6 bis 24 Kohlenstoffatomen und insbesondere 8 bis 18 Kohlenstoffatomenn, oder einen Alkylphenylrest, dessen linearer oder verzweigter Alkylrest 6 bis 24 Kohlenstoffatome und insbesondere 8 bis 18 Kohlenstoffatome umfasst, darstellt;
- R2 einen Alkylenrest mit 2 bis 4 Kohlenstoffatomen darstellt;
- G eine Zuckereinheit mit 5 bis 6 Kohlenstoffatomen darstellt;
- t einen Wert in dem Bereich von 0 bis 10 und vorzugsweise von 0 bis 4 bedeutet;
- v einen Wert in dem Bereich von 1 bis 15 und vorzugsweise von 1 bis 4 bedeutet.

**12.** Verfahren einem der vorstehenden Ansprüche, **dadurch gekennzeichnet, dass** die Tensid(e), vorzugssweise die anionischen Tensid(e), in einem Gesamtgehalt in dem Bereich von 0,1 Gew.-% bis 30 Gew.-%, vorzugsweise in dem Bereich von 1 Gew.-% bis 25 Gew.-%, noch besser von 3 Gew.-% bis 20 Gew.-% und noch besser von 10 Gew.-% bis 18 Gew.-%, bezogen auf das Gesamtgewicht der Make-up-Entfernungszusammensetzung, insbesondere zum Entfernen von Farbe, vorhanden sind.

**13.** Verfahren nach einem der vorstehenden Ansprüche, **dadurch gekennzeichnet, dass** die Säuerungsmittel ausgewählt sind aus organischen Säuren, Mineralsäuren und Gemischen davon, vorzugsweise aus organischen Carbonsäuren.

**14.** Verfahren nach einem der vorstehenden Ansprüche, **dadurch gekennzeichnet, dass** die Säuerungsmittel in einem Gesamtgehalt in dem Bereich von 0,05 Gew.-% bis 20 Gew.-%, bevorzugter von 0,1 Gew.-% bis 10 Gew.-% und noch bevorzugter on 0,3 Gew.-% bis 5 Gew.-%, bezogen auf das Gesamtgewicht Make-up-Entfernungszusammensetzung, insbesondere zum Entfernen von Farbe, vorhanden sind.

**15.** Verfahren nach einem der vorstehenden Ansprüche, **dadurch gekennzeichnet, dass** es ferner einen Schritt der

Anwendung von Wärme auf die Keratinfasern während des oder nach dem Aufbringen der Make-up-Entfernungszusammensetzung, insbesondere zum Entfernen von Farbe, umfasst.

**16.** Verfahren nach einem der vorstehenden Ansprüche, **dadurch gekennzeichnet, dass** es ferner einen Schritt der Anwendung einer mechanischen Wirkung, insbesondere Reibung, auf die Keratinfasern nach dem Aufbringen der Make-up-Entfernungszusammensetzung zum Entfernen von Farbe umfasst.

**17.** Verfahren nach einem der vorstehenden Ansprüche, **dadurch gekennzeichnet, dass** es umfasst:

i) Aufbringen der Haarbehandlungszusammensetzung nach einem der Ansprüche 1 bis 9 auf die Haarkeratinfasern; und anschließend
ii) gegebenenfalls eine Verweilzeit der Haarfärbezusammensetzung auf den Fasern von 1 Minute bis 30 Minuten; und anschließend
iii) gegebenenfalls einen Schritt der Bestrahlung mit Wellenlängen in dem Bereich von 280 bis 450 nm;
vi) gegebenenfalls einen Schritt des Waschens, Spülens, Entwässerns oder Trocknens der Haarkeratinfasern;
v) Aufbringen der Make-up-Entfernungszusammensetzung nach einem der Ansprüche 1 und 11 bis 15 auf die befeuchteten Keratinfasern;
vi) gegebenenfalls verschließendes Abdecken der Locken mit einem Material, wie z.B. Aluminiumfolienumhüllungen;
vii) Anwenden von Wärme mit einer Temperatur von mehr als 30 °C, vorzugsweise von 35 bis 90 °C, für 1 bis 30 Minuten;
viii) nach Entfernen der Abdeckung mechanische Einwirkung auf die Locken, vorzugsweise mit einem Werkzeug;
ix) Spülen der Keratinfasern.

**18.** Verwendung der Make-up-Entfernungszusammensetzung zum Entfernen von Farbe nach einem der Ansprüche 1 bis 14 von Keratinfasern, die zuvor mit einer Haarfärbezusammensetzung behandelt worden sind, die wenigstens ein photovernetzbares Polymer, wie z.B. in einem der Ansprüche 1 bis 8 offenbart, und wenigstens ein Farbmittel ausgewählt aus Pigmenten, Direktfarbstoffen und Gemischen davon umfasst.

## Revendications

**1.** Procédé de démaquillage de fibres kératiniques pour éliminer la couleur de fibres kératiniques qui ont été préalablement traitées par une composition comprenant :

- au moins un polymère photoréticulable comportant au moins un groupe photodimérisable ; et
- au moins un agent colorant choisi parmi les pigments, les colorants directs et leurs mélanges,

ce procédé comprenant l'application sur les fibres kératiniques d'une composition démaquillante pour l'élimination de couleur comprenant :

- au moins un tensioactif, de préférence un tensioactif anionique ou non ionique, de préférence un tensioactif anionique ; et
- au moins un agent acidifiant ;

ladite composition démaquillante ayant un pH inférieur ou égal à 5,5, de préférence de 1 à 5,
le ou les groupes photodimérisables sont choisis parmi des radicaux monovalents de formules (I) et (II) suivantes :

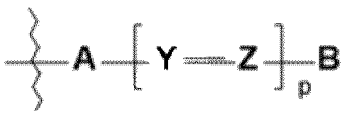

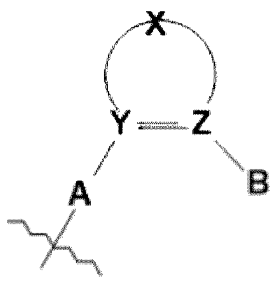

et également leurs isomères géométriques,
dans lesquelles formules **(I)** et **(II)** :

- **Y** et **Z** désignent, indépendamment l'un de l'autre, un atome d'azote ou un groupe C(R), R représentant un atome d'hydrogène ou un groupe ($C_1$-$C_4$)alkyle tel que méthyle ;
- **A** représente une liaison ou un groupe divalent choisi parmi des radicaux ($C_1$-$C_8$) alkylène, arylène, hétéroarylène, cycloalkylène, hétérocycloalkylène, (thio)carbonyle, ($C_2$-$C_8$)alcénylène et leurs combinaisons ;
- **B** représente un groupe monovalent choisi parmi les radicaux ($C_1$-$C_8$)alkyle, les radicaux aryle, les radicaux hétéroaryle éventuellement cationiques, les radicaux cycloalkyle, les radicaux hétérocycloalkyle éventuellement cationiques, les radicaux (thio)carbonyle et les radicaux ($C_2$-$C_8$)alcényle et leurs combinaisons ;
- **X** représente un groupe divalent choisi parmi des radicaux ($C_2$-$C_8$)alkylène, arylène, hétéroarylène, cycloalkylène, hétérocycloalkylène, (thio)carbonyle et ($C_2$-$C_8$)alcénylène et leurs combinaisons ;
- **p** représente un entier compris inclusivement entre 1 et 5, en particulier entre 1 et 3 ; de préférence p est égal à 1 ;

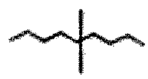

représente la liaison qui relie la partie du radical monovalent au reste de la molécule ;

et

chacun des groupes mentionnés peut éventuellement être substitué par un ou plusieurs atomes d'halogène ou groupes choisis parmi des groupes ($C_1$-$C_6$)alkyle, hydroxy, amino, (di) ($C_1$-$C_6$) alkylamino, phényle, carboxyle et ($C_1$-$C_6$)alcoxy,
($C_1$-$C_6$)alcoxy (thio) carbonyle, hydrogéno(thio)carbonyle, sulfonato R-O-S(O)$_2$- ou R-S(O)$_2$-O-, amide RR'N-C(O)-, R-C(O)-N(R')- ou acyle R-C(O)-, ammonium RR'R"N$^+$-, R, R' et R'', qui peuvent être identiques ou différents, représentant un atome d'hydrogène ou un groupe ($C_1$-$C_4$)alkyle.

**2.** Procédé selon la revendication 1, **caractérisé en ce que** le ou les groupe(s) photodimérisable(s) sont choisis parmi les radicaux monovalents des composés suivants :

- stilbène,
- styrylpyridinium (stilbazolium) de formule suivante et leurs isomères géométriques :

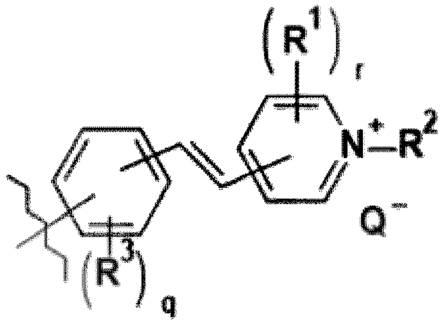

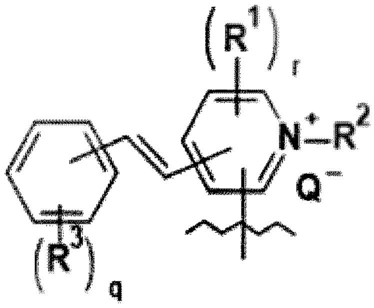

(A1) (A2)

où

- **R¹** et **R³,** qui peuvent être identiques ou différents, représentent un atome d'halogène ou un groupe ($C_1$-$C_6$) alkyle ; ou alors deux groupes $R^1$ ou $R^3$ contigus forment, conjointement avec les atomes de carbone les portant, un groupe benzo ;
- **R²** représente un atome d'hydrogène, un groupe ($C_1$-$C_6$)alkyle éventuellement substitué par un ou plusieurs atomes d'halogène tels que le chlore ou hydroxyle ; de préférence $R^2$ représente un groupe ($C_1$-$C_6$)alkyle tel que méthyle, éthyle ou propyle ;
- **q** et **r** représentent un entier compris inclusivement entre 0 et 4 ; et
- **Q⁻** représente un contre-ion anionique de préférence choisi parmi les ions halogénure tels que chlorures, bromures et iodures, perchlorates, tétrafluoroborates, méthylsulfate, phosphates, sulfates, méthanesulfonates, p-toluènesulfonate ; et
-

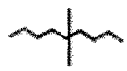

représente la liaison qui relie la partie du radical monovalent au reste de la molécule, étant entendu que le groupe pendant $A_2$ peut être relié au reste de la molécule *via* $R^2$ ; de préférence la liaison

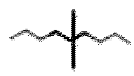

est située sur le phényle en para par rapport au groupe styryle sur $A_1$ ou reliée au reste de la molécule *via* $R^2$ sur $A_2$; préférentiellement le groupe styryle de $A_1$ et $A_2$ est situé en position para par rapport au groupe pyridinium ;
- styrylazolium de la formule suivante et leurs isomères géométriques :

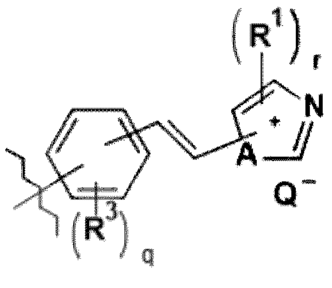

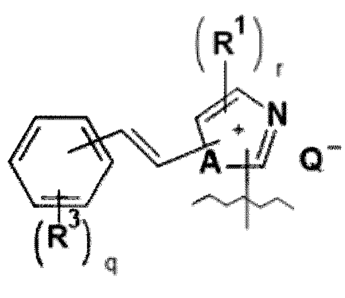

où :

- A représente un atome de soufre, un atome d'oxygène, ou un groupe $NR^2$ ou $C(R^2)_2$ ; et

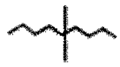

$Q^-$, R, q, $R^1$, $R^2$ et $R^3$ étant tels que définis précédemment,
de préférence la liaison

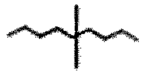

est située sur le phényle en position para par rapport au groupe stryryle,
- styrylpyrazine,
- chalcone,
- (thio)cinnamate et (thio)cinnamamide,
- maléimide,
- (thio)coumarine,
- thymine,
- uracile,
- butadiène,
- anthracène,
- pyridone,
- pyrrolizinone,
- sels d'acridizinium,
- furanone,
- phénylbenzoxazole, et
- leurs dérivés.

3. Procédé selon l'une quelconque des revendications précédentes, **caractérisé en ce que** le polymère photoréticulable a un squelette naturel ou synthétique choisi parmi les polysaccharides, les polymères poly(vinyliques) et les polydiorganosiloxanes, de préférence parmi les poly(alcools vinyliques) et les poly(acétate de vinyle), de préférence partiellement ou totalement hydrolysés.

4. Procédé selon l'une quelconque des revendications précédentes, **caractérisé en ce que** le polymère photoréticulable comprend au moins un groupe pendant hydrophobe.

5. Procédé selon l'une quelconque des revendications précédentes, **caractérisé en ce que** le ou les groupes hydrophobes sont choisis parmi :

- un groupe ($C_1$-$C_{30}$)alkyle, saturé ou insaturé, éventuellement substitué et/ou interrompu par un ou plusieurs hétéroatomes,
- un groupe alcényle,
- un groupe aryle tel que phényle, pyridinyle, furyle, indoyle, benzofuryle, thiophényle, imizadoyle, oxazoyle, thiazoyle, pyrazinyle, pyrimidinyle ;
- un groupe fluoré tel qu'un groupe fluorocarbure tel que $-CF_3$, $-CHF_2$, $-OCF_3$, $SCF_3$ ou $CF_3(O)$-,
- un groupe siliconé du type $-SiR_aR_bR_c$, tel que -si $(CH_3)_3$ , polydiméthylsiloxane PDMS, $-Si(OR)_3$, PDMS $\alpha,\Omega$-diaminopropyle, PDMS $\alpha,\Omega$-dihydroxyalkyle, PDMS $\alpha,\Omega$-dicarboxyalkyle, $R_a$, $R_b$ et $R_c$ qui peuvent être identiques ou différents, représentant un groupe ($C_1$-$C_8$)alkyle éventuellement interrompu et/ou terminé par un ou plusieurs hétéroatomes non contigus tels que O ou S ; et R représentant un groupe ($C_1$-$C_6$)alkyle ;

de préférence parmi un groupe ($C_2$-$C_{22}$)alkyle, plus préférentiellement parmi un groupe ($C_3$-$C_{16}$)alkyle.

6. Procédé selon l'une quelconque des revendications précédentes, **caractérisé en ce que** le polymère photoréticulable a un squelette naturel ou synthétique choisi parmi les polysaccharides, les polymères poly(vinyliques) et les polydiorganosiloxanes, de préférence parmi les poly(alcools vinyliques) et les poly(acétate de vinyle), de préférence partiellement ou totalement hydrolysés.

7. Procédé selon l'une quelconque des revendications précédentes, **caractérisé en ce que** le ou les polymères photoréticulables sont choisis parmi des polymères comprenant au moins les motifs ci-dessous :

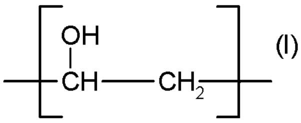 (I) 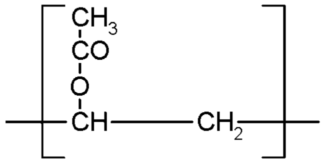 (II)

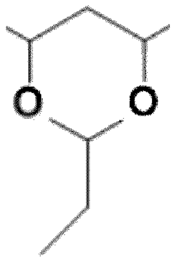

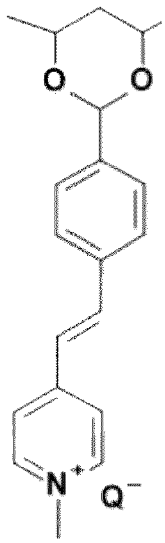

$Q^-$ représentant un contre-ion anionique de préférence choisi parmi les ions halogénures tels que chlorures, bromures, iodures, perchlorates, tetrafluoroborates, méthylsulfate, phosphates, sulfates, méthanesulfonates, p-toluènesulfonate, de préférence mésylate $CH_3OSO_3^-$ ;

8. Procédé selon l'une quelconque des revendications précédentes, **caractérisé en ce que** la quantité totale du ou des polymères photoréticulables se situe dans la plage de 0,01 % à 40 % en poids, de préférence de 0,1 % à 30 % en poids, encore mieux de 0,5 % à 25 % en poids et même mieux encore de 1 % à 10 % en poids par rapport au poids total de la composition (C).

9. Procédé selon l'une quelconque des revendications précédentes, **caractérisé en ce que** la teneur de l'agent ou des agents colorants se situe dans la plage de 0,001 % à 20 % en poids et de préférence 0,005 % à 15 % en poids par rapport au poids total de la composition de coloration capillaire ; de préférence, l'agent ou les agents colorants sont choisis parmi des pigments.

10. Procédé selon l'une quelconque des revendications précédentes, **caractérisé en ce que** la composition démaquillante pour l'élimination de couleur comprend au moins un tensioactif anionique choisi parmi des tensioactifs anioniques sulfatés, des tensioactifs anioniques de carboxylate, des tensioactifs anioniques de sulfonate et leurs mélanges, de préférence parmi des sulfates d'alkyle, sulfates d'éther d'alkyle, sulfates d'éther d'alkylamido, sulfates de polyéther d'alkylaryle, sulfates de monoglycéride ; et également des sels de ces composés ;

    les groupes alkyle de ces composés comprenant de 6 à 30 atomes de carbone, notamment de 12 à 28, encore mieux de 14 à 24, voire de 16 à 22, atomes de carbone; le groupe aryle désignant de préférence un groupe phényle ou benzyle ;
    ces composés pouvant être (poly)oxyalkylénés, notamment (poly)oxyéthylénés et comprenant alors de préférence de 1 à 50 motifs oxyde d'éthylène, et encore mieux de 1 à 10 motifs oxyde d'éthylène, et leurs mélanges.

11. Procédé selon l'une quelconque des revendications précédentes, **caractérisé en ce que** la composition démaquillante pour l'élimination de couleur comprend au moins un tensioactif non ionique choisi parmi :

    - les alcools, $\alpha$-diols et alkyl(C1-20)phénols, ces composés étant polyéthoxylés et/ou polypropoxylés et/ou polyglycérolés, le nombre de groupes oxyde d'éthylène et/ou oxyde de propylène pouvant aller de 1 à 100, et le nombre de groupes glycérol pouvant aller de 2 à 30 ; et/ou ces composés comprenant au moins une chaîne grasse comportant de 8 à 30 atomes de carbone, notamment de 16 à 30 atomes de carbone ;

- les condensats d'oxyde d'éthylène et d'oxyde de propylène avec des alcools gras ; les amides gras polyéthoxylés ayant de préférence de 2 à 30 motifs d'oxyde d'éthylène, les amides gras polyglycérolés comprenant en moyenne de 1 à 5, et en particulier de 1,5 à 4, groupes glycérol ; les alcanolamides d'acides gras en C8-C30, tels que les monoalcanolamides d'acides gras en C8-C30, en particulier les monoéthanolamides ou les monoisopropanolamides d'acides gras en C8-C30; les esters d'acides gras de sorbitane éthoxylés contenant de préférence de 2 à 40 motifs d'oxyde d'éthylène, les esters d'acides gras de saccharose, les esters d'acides gras polyoxyalkylénés et de préférence polyoxyéthylénés ayant de 2 à 150 moles d'oxyde d'éthylène, y compris les huiles végétales oxyéthylénées, les dérivés de N-alkyl(C6-24)glucamine, les oxydes d'amines tels que les oxydes de(C10-14 alkyle) amine, les oxydes de N-(C10-14 acyl)aminopropylmorpholine ;
- les tensioactifs non ioniques de type alkylpolyglycoside, notamment représentés par la formule générale suivante : R1O-(R2O)t-(G)v dans laquelle:
- R1 représente un radical alkyle ou alcényle linéaire ou ramifié comprenant 6 à 24 atomes de carbone et notamment 8 à 18 atomes de carbone, ou un radical alkylphényle dont le radical alkyle linéaire ou ramifié comprend 6 à 24 atomes de carbone et notamment 8 à 18 atomes de carbone ;
- R2 représente un radical alkylène comprenant 2 à 4 atomes de carbone ;
- G représente un motif de sucre comprenant 5 à 6 atomes de carbone ;
- t désigne une valeur allant de 0 à 10 et de préférence de 0 à 4 ;
- v désigne une valeur allant de 1 à 15 et de préférence de 1 à 4,

**12.** Procédé selon l'une quelconque des revendications précédentes, **caractérisé en ce que** le ou les tensioactifs, de préférence les tensioactifs anioniques, sont présents en une teneur totale allant de 0,1 % à 30 % en poids, de préférence allant de 1 % à 25 % en poids, encore mieux de 3 % à 20 % en poids et mieux encore de 10 % à 18 % en poids par rapport au poids total de la composition démaquillante notamment pour l'élimination de couleur.

**13.** Procédé selon l'une quelconque des revendications précédentes, **caractérisé en ce que** l'agent ou les agents acidifiants sont choisis parmi les acides organiques, les acides minéraux et leurs mélanges, de préférence parmi les acides carboxyliques organiques.

**14.** Procédé selon l'une quelconque des revendications précédentes, **caractérisé en ce que** l'agent ou les agents acidifiants sont présents en une teneur totale allant de 0,05 % à 20 % en poids, plus préférentiellement de 0,1 % à 10 % en poids et encore plus préférentiellement de 0,3 % à 5 % en poids, par rapport au poids total de la composition démaquillante notamment pour l'élimination de couleur.

**15.** Procédé selon l'une quelconque des revendications précédentes, **caractérisé en ce qu'**il comprend également une étape d'application de chaleur sur les fibres kératiniques pendant ou après l'application de la composition démaquillante, notamment pour l'élimination de couleur.

**16.** Procédé selon l'une quelconque des revendications précédentes, **caractérisé en ce qu'**il comprend en outre une étape d'application d'une action mécanique, notamment de friction, sur les fibres kératiniques après l'application de la composition démaquillante pour l'élimination de couleur.

**17.** Procédé selon l'une quelconque des revendications précédentes, **caractérisé en ce qu'**il comprend

i) l'application sur lesdites fibres kératiniques capillaires de la composition de traitement des cheveux selon l'une des revendications 1 à 9 ; puis
ii) éventuellement un temps de pose de ladite composition colorante capillaire sur les fibres de 1 minute à 30 minutes ; et ensuite
iii) éventuellement une étape d'irradiation avec des longueurs d'onde allant de 280 à 450 nm ;
vi) éventuellement une étape de lavage, de rinçage, de déshydratation ou de séchage desdites fibres kératiniques capillaires ;
v) l'application de la composition démaquillante selon l'une des revendications 1 et 11 à 15 sur les fibres kératiniques humidifiées ;
vi) éventuellement un recouvrement occlusif des mèches par un matériau tel que des enveloppements de feuille d'aluminium ;
vii) l'application de chaleur à une température de plus de 30 °C, de préférence de 35 à 90 °C, pendant 1 à 30 minutes ;
viii) après l'élimination du recouvrement, une action mécanique sur les mèches de préférence avec un outil ;
ix) le rinçage des fibres kératiniques.

**18.** Utilisation de la composition démaquillante pour l'élimination de couleur, telle que définie dans l'une quelconque des revendications 1 à 14, des fibres kératiniques préalablement traitées par une composition de coloration capillaire comprenant au moins un polymère photoréticulable tel que divulgué dans l'une quelconque des revendications 1 à 8 et au moins un agent colorant choisi parmi les pigments, les colorants directs et leurs mélanges.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- FR 3090364
- WO 2021104709 A1
- WO 2017108767 A1
- US 2811510 A
- EP 0313220 A
- EP 0313221 A
- EP 092901 A
- GB 2030575 A
- GB 2076826 A
- US 20070112094 A
- WO 9629312 A
- US 5061603 A
- EP 313220 A
- FR 2679771
- EP 1184426 A
- JP 09188830 A
- JP 10158450 A
- JP 10158541 A
- JP 07258460 A
- JP 05017710 A
- US 4578266 A
- WO 9515144 A
- WO 9501772 A
- EP 714954 A

### Non-patent literature cited in the description

- **J. MARCH**. Advanced Organic Chemistry. Wiley Interscience, 1992, 855
- *Chemical Review*, 1983, vol. 83 (5), 507
- *Polym, Paint Colour Journal*, 1988, vol. 178, 209
- **ELLIS MORWOOD**. Current Trends in Polymer Photochemistry. 1995
- **T. UHLICH et al.** *Reactive & Functional Polymers*, 1995, vol. 28, 55-40
- **ICHIMURA K. et al.** Preparation and characteristics of photocrosslinkable poly(vinyl alcohol). *Journal of Polymer Science, Polymer Chemistry*, 1982, vol. 20, 1419-1432
- Cosmetics and Toiletries. February 1990, vol. 105, 53-64